(19) **Europäisches Patentamt / European Patent Office / Office européen des brevets**

(11) **EP 3 463 479 B1**

(12) # FASCICULE DE BREVET EUROPEEN

(45) Date de publication et mention
de la délivrance du brevet:
**15.04.2026 Bulletin 2026/16**

(21) Numéro de dépôt: **17743405.7**

(22) Date de dépôt: **02.06.2017**

(51) Classification Internationale des Brevets (IPC):
*A61K 31/05* (2006.01)    *A61K 31/43* (2006.01)
*A61K 31/424* (2006.01)   *A61P 1/02* (2006.01)
*A61P 11/00* (2006.01)    *A61P 13/00* (2006.01)
*A61P 13/10* (2006.01)    *A61P 13/12* (2006.01)
*A61P 15/00* (2006.01)    *A61P 19/02* (2006.01)
*A61P 19/08* (2006.01)    *A61P 27/16* (2006.01)
*A61P 31/04* (2006.01)

(52) Classification Coopérative des Brevets (CPC):
(C-Sets disponibles)
A61K 31/43; A61K 9/0095; A61K 9/145;
A61K 9/146; A61K 31/05; A61K 31/424;
A61P 1/02; A61P 11/00; A61P 13/00; A61P 13/10;
A61P 13/12; A61P 15/00; A61P 19/02; A61P 19/08;
A61P 27/16;                                    (Cont.)

(86) Numéro de dépôt international:
**PCT/MA2017/000014**

(87) Numéro de publication internationale:
**WO 2017/209588 (07.12.2017 Gazette 2017/49)**

(54) **FORMULATION PHARMACEUTIQUE DE CINÉOL ET D'AMOXICILLINE**

PHARMAZEUTISCHE FORMULIERUNG MIT CINEOL UND AMOXICILLIN

PHARMACEUTICAL FORMULATION COMPRISING CINEOL AND AMOXICILLIN

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats de validation désignés:
**MA**

(30) Priorité: **02.06.2016 FR 1655017**
**02.06.2016 FR 1655018**
**02.06.2016 MA 39084**
**02.06.2016 MA 39085**

(43) Date de publication de la demande:
**10.04.2019 Bulletin 2019/15**

(73) Titulaire: **Advanced Scientific Developements
Bouskoura (MA)**

(72) Inventeurs:
• **REMMAL, Adnane
30080 Fes (MA)**

• **AKHMOUCH, Ahmed Amine
27182 Bouskoura (MA)**

(74) Mandataire: **Cabinet Becker et Associés
25, rue Louis le Grand
75002 Paris (FR)**

(56) Documents cités:
**WO-A1-2016/041958**

• **ADINA CATINCA GRADINARU ET AL:
"INTERACTIONS BETWEEN CARDAMOM
ESSENTIAL OIL AND CONVENTIONAL
ANTIBIOTICS AGAINST STAPHYLOCOCCUS
AUREUS CLINICAL ISOLATES", FARMACIA, 1
January 2014 (2014-01-01), pages 1214,
XP055335750, Retrieved from the Internet
<URL:http://www.revistafarmacia.ro/201406/
art-17-Gradinaru_Aprotosoaie_1214-1222.pdf>**

**(Cont. page suivante)**

• HANAN H ABD EL-KALEK AND EMAN A MOHAMED: "SYNERGISTIC EFFECT OF CERTAIN MEDICINAL PLANTS ANDAMOXICILLIN AGAINST SOME CLINICAL ISOLATES OFMETHICILLIN-RESISTANT Staphylococcus Aureus (MRSA)", vol. 3, no. 3, 1 January 2012 (2012-01-01), pages 387 - 398, XP002735981, Retrieved from the Internet <URL:http://bipublication.com/files/IJPA-V3I32012-3.pdf> [retrieved on 20150212]

• KNEZEVIC PETAR ET AL: "Antimicrobial activity ofEucalyptus camaldulensisessential oils and their interactions with conventional antimicrobial agents against multi-drug resistantAcinetobacter baumannii", JOURNAL OF ETHNOPHARMACOLOGY, ELSEVIER IRELAND LTD, IE, vol. 178, 6 December 2015 (2015-12-06), pages 125 - 136, XP029392961, ISSN: 0378-8741, DOI: 10.1016/J.JEP.2015.12.008

• IOANA MARINAS ET AL: "Rosmarinus officinalis essential oil as antibiotic potentiator against Staphylococcus aureus", BIOINTERFACE RESEARCH IN APPLIED CHEMISTRY, 15 February 2012 (2012-02-15), pages 271 - 276, XP055086305, Retrieved from the Internet <URL:http://biointerfaceresearch.com/wp-content/uploads/downloads/2012/02/35.BRIAC.Grumezescu.pdf>

• BRIAN DAVID CRAFT ET AL: "Antioxidant Properties of Extracts Obtained from Raw, Dry-roasted, and Oil-roasted US Peanuts of Commercial Importance", PLANT FOODS FOR HUMAN NUTRITION, KLUWER ACADEMIC PUBLISHERS, DO, vol. 65, no. 3, 3 March 2010 (2010-03-03), pages 311 - 318, XP019829058, ISSN: 1573-9104

(52) Classification Coopérative des Brevets (CPC): (Cont.)**A61P 31/04; A61P 43/00**

C-Sets
**A61K 31/05, A61K 2300/00;
A61K 31/424, A61K 2300/00;
A61K 31/43, A61K 2300/00**

## Description

La présente invention relève du domaine de la médecine, en particulier des infections bactériennes. Elle concerne de nouveaux traitements et de nouvelles formulations pharmaceutiques particulièrement adaptés à un usage médical ou vétérinaire dans la lutte contre les infections bactériennes, notamment des infections résistantes aux antibiotiques.

## Arrière-plan technologique de l'invention

Les antibiotiques sont des substances naturelles ou synthétiques qui ont une activité bactéricide ou bactériostatique. Leur introduction généralisée après la Seconde Guerre mondiale a été l'un des progrès thérapeutiques les plus importants du XXe siècle. Les traitements antibiotiques ont fait progresser l'espérance de vie de plus de dix ans, soit plus qu'aucun autre traitement médical. Cependant, l'usage généralisé, voire abusif de certains antibiotiques, y compris en traitement préventif, curatif ou en complément alimentaire dans l'alimentation animale, dans les piscicultures, en médecine vétérinaire et humaine, ou encore comme pesticides pour le traitement des végétaux, a introduit une pression de sélection qui a conduit au développement de populations de micro-organismes résistants aux antibiotiques et à une baisse générale de l'efficacité thérapeutique. En milieu hospitalier, cela conduit à une augmentation du risque nosocomial, notamment faute de traitement adapté contre certains germes multi-résistants.

Dans son dernier rapport (avril 2014) sur la résistance des bactéries aux antibiotiques, l'OMS souligne « qu'à moins que les nombreux acteurs concernés agissent d'urgence, de manière coordonnée, le monde s'achemine vers une ère post-antibiotiques, où des infections courantes et des blessures mineures qui ont été soignées depuis des décennies pourraient à nouveau tuer ». La résistance à certains traitements dits de derniers recours est déjà une réalité. Ainsi, la résistance aux traitements des infections potentiellement mortelles causées par une bactérie intestinale courante, *Klebsiella pneumoniae,* s'est propagée à toutes les régions du monde. Il en va de même pour la résistance à un des médicaments antibactériens les plus largement utilisés dans le traitement des infections des voies urinaires causées par *E. coli* - les fluoroquinolones. Récemment, l'échec du traitement de dernier recours contre la gonorrhée - les céphalosporines de troisième génération - a été confirmé en Afrique du Sud, en Australie, en Autriche, au Canada, en France, au Japon, en Norvège, au Royaume-Uni, en Slovénie et en Suède.

Pour faire face à ce péril, l'OMS en appelle notamment à la mise au point de nouveaux produits diagnostics, de nouveaux antibiotiques et d'autres outils indispensables pour que les professionnels de santé puissent conserver leur avance sur la progression des résistances.

L'amoxicilline est un antibiotique β-lactamine bactéricide de la famille des aminopénicillines, indiqué dans le traitement des infections bactériennes à germes sensibles. L'amoxicilline est l'antibiotique le plus couramment utilisé, notamment chez les enfants, car elle présente une bonne absorption par voie orale, un spectre d'action antimicrobien large et un coût faible. L'amoxicilline est utilisée dans le traitement de diverses maladies infectieuses, notamment celles des poumons, des bronches, du nez, de la gorge ou des oreilles, du sang, de l'appareil digestif ou urinaire, des voies génitales, des gencives et des dents.

L'amoxicilline est souvent utilisée en combinaison avec une autre molécule, l'acide clavulanique, un inhibiteur de la β-lactamase. La β-lactamase est une enzyme produite par des bactéries résistantes aux antibiotiques β-lactamine. L'acide clavulanique, en inhibant les β-lactamases, empêche l'inactivation de l'amoxicilline par les β-lactamases, et lui permet ainsi de conserver son activité sur les germes résistants producteurs de β-lactamases.

Cependant, on assiste ces dernières années à l'apparition de germes bactériens particulièrement résistants, notamment des bactéries à β-lactamases à spectre étendu (BLSE), qui ne sont plus, ou partiellement, sensibles aux inhibiteurs classiques des β-lactamases tel que l'acide clavulanique. WO2016/041958 divulgue une composition comprenant un mélange cinéol, amoxicilline/ acide clavulanique ayant des propriétés synergiques sur différentes souches bactériennes résistantes, mais sans huile d'arachide.

Par conséquent, il est indispensable de trouver des solutions permettant de restaurer l'efficacité de l'amoxicilline à l'encontre des bactéries résistantes, en particulier des bactéries productrices de β-lactamases à spectre étendu (BLSE).

## Résumé de l'invention

Les inventeurs ont découvert que le cinéol permet d'accroître l'efficacité de l'amoxicilline, en particulier vis-à-vis des bactéries résistantes. Ils ont en effet démontré que la combinaison d'amoxicilline et de cinéol permettait d'obtenir un effet synergique renforçant considérablement l'activité antibactérienne de l'amoxicilline. Ils ont ainsi mis au point une nouvelle combinaison de molécules comprenant de l'amoxicilline, du cinéol, et éventuellement de l'acide clavulanique permettant de lutter efficacement contre les germes bactériens résistants, en particulier les germes résistants à la combinaison d'amoxicilline et d'acide clavulanique.

Les inventeurs ont également mis au point une formulation pharmaceutique permettant d'augmenter considérablement l'activité antibactérienne de l'amoxicilline, seule ou en association avec l'acide clavulanique, en particulier vis-à-vis de germes bactériens résistants à la combinaison d'amoxicilline et d'acide clavulanique.

L'objet de la présente invention est défini par les revendications.

Ainsi, la présente invention concerne, dans un premier aspect, une formulation pharmaceutique sous forme de poudre comprenant, ou consistant essentiellement en, du cinéol, de l'amoxicilline, un inhibiteur de β-lactamases et une huile pharmaceutiquement acceptable, ladite huile pharmaceutiquement acceptable étant l'huile d'arachide.

De préférence, l'inhibiteur de β-lactamase est l'acide clavulanique.

La formulation selon l'invention peut comprendre entre environ 5 mg et environ 100 mg de cinéol par gramme de poudre, de préférence entre environ 10 mg et environ 50 mg de cinéol par gramme de poudre, de manière encore préférée entre environ 20 mg et environ 40 mg de cinéol par gramme de poudre, et de manière tout particulièrement préférée environ 33 mg de cinéol par gramme de poudre.

La formulation selon l'invention peut également comprendre entre environ 20 mg et environ 500 mg d'amoxicilline par gramme de poudre, de préférence entre environ 50 mg et environ 300 mg d'amoxicilline par gramme de poudre, de manière encore préférée entre environ 150 mg et environ 200 mg d'amoxicilline par gramme de poudre, et de manière tout particulièrement préférée environ 167 mg d'amoxicilline par gramme de poudre.

La formulation selon l'invention peut encore comprendre entre environ 2 mg et environ 50 mg d'huile par gramme de poudre, de préférence entre environ 10 mg et environ 25 mg d'huile par gramme de poudre, de manière encore préférée entre environ 15 mg et environ 20 mg d'huile par gramme de poudre, et de manière tout particulièrement préférée environ 17 mg d'huile par gramme de poudre.

La formulation selon l'invention peut enfin comprendre entre environ 1 mg et environ 100 mg d'inhibiteur de β-lactamase, de préférence d'acide clavulanique, par gramme de poudre, de préférence entre environ 5 mg et environ 50 mg d'inhibiteur de β-lactamase, de préférence d'acide clavulanique, par gramme de poudre, de manière encore préférée entre environ 15 mg et environ 25 mg d'inhibiteur de β-lactamase, de préférence d'acide clavulanique, par gramme de poudre, et de manière tout particulièrement préférée environ 21 mg d'inhibiteur de β-lactamase, de préférence d'acide clavulanique, par gramme de poudre.

La formulation selon l'invention peut comprendre en particulier entre environ 5 mg et environ 100 mg, de préférence entre environ 10 mg et environ 50 mg, de cinéol par gramme de poudre ; et/ou entre environ 20 mg et environ 500 mg, de préférence entre environ 50 mg et environ 300 mg, d'amoxicilline par gramme de poudre ; et/ou entre environ 2 mg et environ 50 mg, de préférence entre environ 10 mg et environ 25 mg, d'huile par gramme de poudre ; et/ou optionnellement entre environ 1 mg et environ 100 mg, de préférence entre environ 5 mg et environ 50 mg, d'inhibiteur de β-lactamase, de préférence d'acide clavulanique, par gramme de poudre.

De préférence, la formulation selon l'invention comprend entre environ 20 mg et environ 40 mg, de préférence environ 33 mg, de cinéol par gramme de poudre ; entre environ 150 mg et environ 200 mg, de préférence environ 167 mg, d'amoxicilline par gramme de poudre ; entre environ 15 mg et environ 20 mg, de préférence environ 17 mg, d'huile par gramme de poudre ; et/ou optionnellement entre environ 15 mg et environ 25 mg, de préférence environ 21 mg d'inhibiteur de β-lactamase, de préférence d'acide clavulanique, par gramme de poudre.

La formulation selon l'invention peut présenter un ratio massique amoxicilline/cinéol compris entre 2 et 8, de préférence entre 3 et 7, de manière plus particulièrement préférée entre 4 et 6, et de manière tout particulièrement préférée un ratio massique amoxicilline/cinéol d'environ 5.

La formulation selon l'invention peut présenter un ratio massique amoxicilline/huile compris entre 5 et 15, de préférence entre 7 et 13, de manière plus particulièrement préférée entre 8 et 12, et de manière tout particulièrement préférée un ratio massique amoxicilline/huile d'environ 10.

La formulation selon l'invention peut présenter un ratio massique cinéol/huile compris entre 0,1 et 5, de préférence entre 0.5 et 4, de manière plus particulièrement préférée entre 1 et 3, et de manière tout particulièrement préférée un ratio massique cinéol/huile est d'environ 2.

La formulation selon l'invention peut présenter un ratio massique amoxicilline/inhibiteur de β-lactamases compris entre 5 et 11, de préférence entre 6 et 10, de manière plus particulièrement préférée entre 7 et 9, et de manière tout particulièrement préférée, un ratio massique amoxicilline/inhibiteur de β-lactamases d'environ 8.

La formulation selon l'invention peut être destinée à une administration par voie orale, de préférence après suspension dans un solvant aqueux.

La formulation suivant l'invention peut être conditionnée dans un récipient unidose, de préférence un récipient unidose contenant entre environ 1 g et environ 150 g de poudre, de manière encore préférée entre environ 1 g et environ 50 g de poudre, de manière toujours préférée entre environ 1 g et environ 10 g de poudre, et de manière plus particulièrement préférée environ 3 g de poudre.

La formulation suivant l'invention peut en outre comprendre au moins un excipient ou support pharmaceutiquement acceptable, de préférence sélectionné dans le groupe consistant en un édulcorant, un aromatisant, un antiagglomérant, un lubrifiant, un désintégrant, et un mélange de ceux-ci.

Dans un deuxième aspect, la présente invention concerne également la formulation tel que décrite ci-dessus pour une utilisation dans le traitement d'une pathologie infectieuse chez un sujet, de préférence un animal ou un humain.

De préférence, ladite pathologie infectieuse est une pathologie infectieuse d'origine bactérienne, de manière encore préférée une pathologie infectieuse causée par une bactérie résistante aux antibiotiques de la famille des β-lactamines.

De préférence, la formulation est administrée ou destinée à être administrée au sujet durant une période allant de 1 jour à 4 semaines et à raison de 3 à 30 grammes par jour, en une seule ou plusieurs prises.

La présente invention concerne encore, dans un troisième aspect, un procédé de fabrication de la formulation pharmaceutique selon l'invention comprenant :

- l'obtention d'une solution de mouillage par mélange de cinéol et d'une huile pharmaceutiquement acceptable ; et
- le mouillage d'une poudre comprenant de l'amoxicilline par la solution de mouillage afin d'obtenir une préparation poudreuse comprenant l'amoxicilline, le cinéol et l'huile.

Optionnellement, le procédé peut comprendre en outre le mélange de la préparation poudreuse comprenant l'amoxicilline, le cinéol et l'huile avec une poudre comprenant un inhibiteur de β-lactamase, de préférence de l'acide clavulanique ; et/ou

- l'ajout d'édulcorant, d'aromatisant et/ou de lubrifiant, et le mélange de ceux-ci afin d'obtenir une poudre homogène ; et/ou
- le tamisage de la poudre ainsi obtenue; et/ou
- le conditionnement de la poudre tamisée dans des récipients unidoses.

De préférence, la poudre comprenant de l'amoxicilline et/ou la poudre comprenant un inhibiteur de β-lactamase, de préférence de l'acide clavulanique, comprennent en outre un désintégrant et/ou un antiagglomérant.

De préférence, l'infection bactérienne est causée par une bactérie résistante aux antibiotiques, de préférence aux antibiotiques de la famille des β-lactamines.

Le sujet traité est un animal, de préférence un mammifère, et de manière tout particulièrement préférée un humain.

La présente invention concerne également une formulation selon l'invention pour une utilisation dans le traitement d'une infection bactérienne chez un sujet, de préférence une infection bactérienne causée par une bactérie résistante aux antibiotiques.

De préférence, l'infection bactérienne est sélectionnée dans le groupe constitué des cystites, en particulier les cystites aiguës récidivantes, des sinusites bactériennes, en particulier les sinusites maxillaires aiguës, des otites, en particulier les otites moyennes aiguës, des bronchites, en particulier les bronchites chroniques et/ou aiguës, des bronchopneumopathies, en particulier les bronchopneumopathies chroniques et/ou aiguës, des pyélonéphrites, des infections gynécologiques hautes, des parodontites, des infections stomatologiques sévères, en particulier les abcès, les phlegmons et les cellulites, des morsures animales, des infections des os et des articulations, en particulier l'ostéomyélite, de préférence ladite infection bactérienne est une cystite, en particulier une cystite causée par une bactérie résistante aux antibiotiques de la famille des β-lactamines.

**Brève description des dessins**

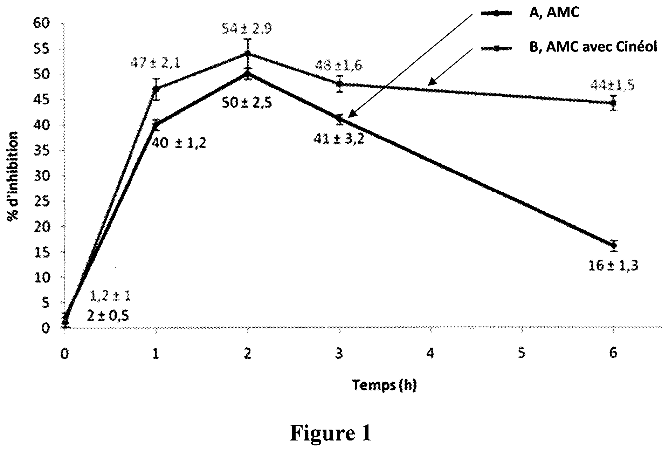

**Figure 1 :** Etude de l'activité antibactérienne de sérum de lapin traité par une combinaison d'amoxicilline, d'acide clavulanique et de cinéol. A (AMC sans cinéol) : après administration par voie orale d'une dose unique d'une composition comprenant de l'Amoxicilline (1.5 g) et de l'acide clavulanique (186.5 mg) chez 3 lapins, le pourcentage d'inhibition du sérum aux temps $T_0$, $T_{1h}$, $T_{2h}$, $T_{3h}$ et $T_{6h}$ a été calculé vis-à-vis d'une souche d'*Escherichia coli* BLSE multirésistante. B (AMC avec cinéol) : après administration par voie orale d'une dose unique d'une composition comprenant de l'Amoxicilline (1.5g), de l'acide clavulanique (186.5 mg) et du cinéol (300 mg) chez 3 lapins, le pourcentage d'inhibition du sérum aux temps $T_0$, $T_{1h}$, $T_{2h}$, $T_{3h}$ et $T_{6h}$ a été calculé vis-à-vis d'une souche d'*Escherichia coli* BLSE multirésistante.

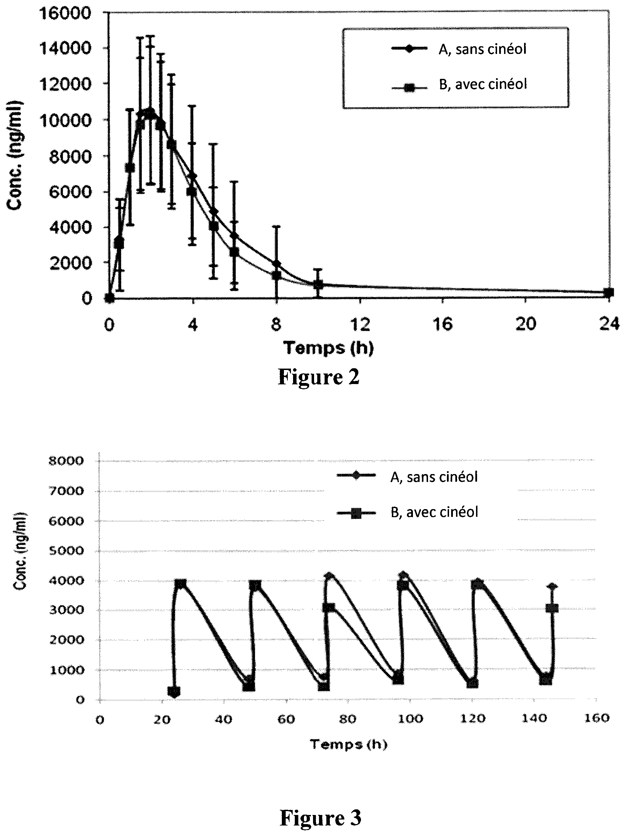

**Figure 2** : Suivi des concentrations sériques moyennes de l'amoxicilline pendant 24 h. A (sans cinéol) : après administration par voie orale d'une dose unique (12 g) d'une composition comprenant de l'Amoxicilline (2 g) et de l'acide clavulanique (250 mg), la concentration sérique en amoxicilline est suivie pendant 24 h chez 12 volontaires sains. B (avec cinéol) : après administration par voie orale d'une dose unique (12 g) d'une composition comprenant de l'Amoxicilline (2 g), de l'acide clavulanique (250 mg) et du cinéol (400 mg), la concentration sérique en amoxicilline est suivie pendant 24 h chez 12 volontaires sains.

**Figure 3 :** Suivi des concentrations plasmatiques moyennes de l'amoxicilline pendant 7 jours. A (sans cinéol) : après administration par voie orale d'une dose unique (12 g) d'une composition comprenant de l'amoxicilline (2 g) et de l'acide clavulanique (250 mg), les 12 volontaires reçoivent, 3 fois par jour pendant 7 jours, des doses d'entretien (3 g) de la même composition comprenant de l'amoxicilline (500 mg) et de l'acide clavulanique (62,5 mg). La concentration plasmatique en amoxicilline est suivie pendant 7 jours chez les 12 volontaires sains. B (avec cinéol) : après administration par voie orale d'une dose unique (12 g) d'une composition comprenant de l'amoxicilline (2 g), de l'acide clavulanique (250 mg) et du cinéol (400 mg), les 12 volontaires reçoivent, 3 fois par jour pendant 7 jours, des doses d'entretien (3 g) de la même composition comprenant de l'amoxicilline (500 mg), de l'acide clavulanique (62,5 mg) et du cinéol (100 mg). La concentration plasmatique en amoxicilline est suivie pendant 7 jours chez les 12 volontaires sains.

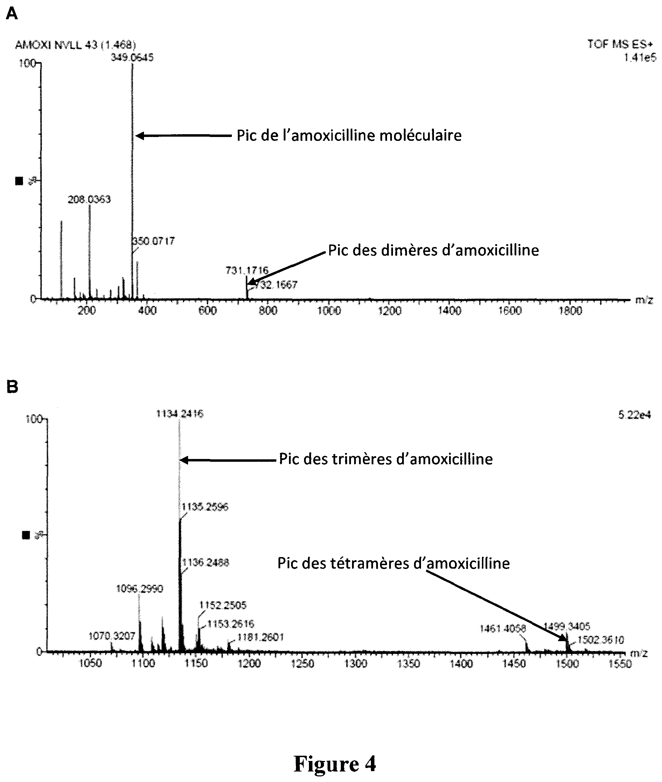

**Figure 4 :** Etude spectroscopique de la formation de complexes d'amoxicilline. A (AMX seule) : Analyse par spectroscopie de masse d'Amoxicilline seule en solution dans de l'eau. B (AMX avec Cinéol) : Analyse par spectroscopie de masse d'Amoxicilline avec du Cinéol en solution dans de l'eau.

## Description détaillée de l'invention

Les inventeurs ont démontré que la combinaison de l'amoxicilline avec le cinéol permettait d'obtenir un effet synergique accroissant considérablement l'efficacité de l'amoxicilline, en particulier vis-à-vis des bactéries résistantes. Ils ont notamment observé que le cinéol, à des concentrations sub-thérapeutiques, protège l'amoxicilline de l'effet inhibiteur des β-lactamases, une enzyme produite par des bactéries résistantes à l'amoxicilline.

Les inventeurs ont donc mis au point une nouvelle combinaison thérapeutique comprenant de l'amoxicilline, du cinéol et éventuellement de l'acide clavulanique permettant de lutter efficacement contre les germes bactériens résistants, en particulier les germes résistants à la combinaison d'amoxicilline et d'acide clavulanique.

Les inventeurs ont également mis au point une formulation pharmaceutique sous forme de poudre combinant du cinéol, de l'amoxicilline et de l'acide clavulanique. Ils ont observé que cette combinaison permettait d'augmenter considérablement l'activité antibactérienne de l'amoxicilline et ainsi de traiter efficacement des patients atteints d'infections à germes résistants.

Cette nouvelle formulation possède en outre d'excellentes propriétés de conservation. En particulier, l'utilisation d'huile dans la formulation permet de fixer très efficacement le cinéol, un agent hautement volatil, au support poudreux de cette formulation. Cela permet de proposer une formulation unique pour l'administration des composés actifs qui, après suspension dans un solvant aqueux, peut facilement être administrée par voie orale. Disposer d'un médicament unique, qui plus est administré par voie oral, augmente grandement l'observance des traitements et par là même leur efficacité.

Les inventeurs ont encore mis en évidence que l'amoxicilline, en présence de cinéol, forme un complexe d'au moins trois molécules d'amoxicilline. La formation de ce complexe rend les molécules d'amoxicilline moins accessibles aux β-lactamases et augmente ainsi leur efficacité thérapeutique.

Ainsi, selon un premier aspect, la présente invention concerne une formulation pharmaceutique sous forme de poudre comprenant, ou consistant essentiellement en, du cinéol, de l'amoxicilline, et une huile pharmaceutiquement acceptable.

Selon un second aspect, la présente demande divulgue une combinaison thérapeutique (non revendiquée) comprenant du cinéol et de l'amoxicilline pour une utilisation dans le traitement d'une infection bactérienne chez un sujet. La combinaison divulguée peut en outre comprendre un inhibiteur de β-lactamases, de préférence de l'acide clavulanique. En particulier, la combinaison divulguée peut être une préparation combinée pour l'utilisation simultanée, séparée, ou séquentielle des principes actifs de la combinaison, de préférence du cinéol, de l'amoxicilline et de l'acide clavulanique.

## Définitions

Dans le présent document, le terme « environ » se réfère à une gamme de valeurs de ± 10% de la valeur spécifiée. A titre d'exemple, « environ 50 » comprend les valeurs de ± 10 % de 50, c'est-à-dire les valeurs de la gamme 45 à 55. De préférence, le terme « environ » se réfère à une gamme de valeurs de ± 5% de la valeur spécifiée. Il est entendu que les valeurs précédées du terme « environ » doivent également être considérées comme spécifiquement décrites dans la présente demande.

Le terme « consiste essentiellement en », tel qu'utilisé ici, se réfère à une formulation selon l'invention ne comprenant pas d'autre principe actif que ceux mentionnés dans ladite formulation, en particulier pas d'autres antibiotiques ou inhibiteurs de β-lactamases.

Tel qu'utilisé ici, le terme « excipient ou support pharmaceutiquement acceptable » se réfère à toute substance autre qu'un principe actif présent dans une formulation pharmaceutique. Son addition est notamment destinée à conférer une consistance particulière, ou d'autres caractéristiques physiques ou gustatives particulières, au produit final, tout en évitant toute interaction, notamment chimique, avec le ou les principes actifs.

Tel qu'utilisé ici, le terme « principe actif » se réfère à une molécule présentant un effet thérapeutique. En particulier, le cinéol, l'amoxicilline et les inhibiteurs de β-lactamases sont des principes actifs.

Tel qu'utilisé ici, le terme « effet thérapeutique » se réfère à un effet induit par un principe actif, une composition selon l'invention, ou par une combinaison selon l'invention, capable de prévenir ou de retarder l'apparition d'une infection bactérienne, ou de guérir ou de réduire les effets d'une infection bactérienne.

Tel qu'utilisé ici, le terme « effet antibactérien » se réfère à un effet induit par un principe actif, une composition selon l'invention, ou par une combinaison selon l'invention, capable de réduire chez un sujet la quantité et/ou la concentration des bactéries responsables de l'infection bactérienne.

Tel qu'utilisé ici, le terme « effet synergique » se réfère à une composition selon l'invention ou à une combinaison selon l'invention ayant un effet thérapeutique et/ou un effet antibactérien supérieur à la somme des effets thérapeutiques et/ou à la somme des effets antibactériens de tous les principes actifs présents dans ladite composition ou dans ladite combinaison, lorsqu'ils sont pris individuellement. La présence d'un tel effet peut être évaluée en calculant l'indice de fraction des concentrations inhibitrices (fic-index) comme illustré dans l'exemple 1.

Tel qu'utilisé ici, le terme « traitement » se réfère à tout acte visant à améliorer le statut médical d'une personne atteinte d'une infection bactérienne. Le traitement peut viser aussi bien à une amélioration de l'état du patient, c'est-à-dire à une régression de l'infection ou de certains de ses symptômes, qu'à une éradication de l'infection ou de certains de ses symptômes. Le traitement peut également avoir pour effet d'empêcher ou de ralentir la progression d'une infection bactérienne. Le traitement peut encore avoir un effet prophylactique ou préventif, c'est-à-dire empêcher ou retarder l'apparition de l'infection bactérienne.

Tel qu'utilisés ici, les termes de « quantité » et « dose » sont équivalents et peuvent être employés l'un pour l'autre.

Tel qu'utilisé ici, le terme « quantité thérapeutiquement efficace » se réfère à une quantité de principe actif, de composition selon l'invention, ou de combinaison selon l'invention suffisante pour induire un effet thérapeutique. Alternativement, le terme « quantité thérapeutiquement efficace » peut se référer à une quantité de principe actif, de composition selon l'invention, ou de combinaison selon l'invention suffisante pour induire un effet antibactérien. Il est évident que la quantité à administrer peut être adaptée par l'homme du métier, en fonction du sujet à traiter, de la nature de l'infection bactérienne, etc. En particulier, les doses et régimes d'administration dépendent de la nature, du stade de développement et de la sévérité de l'infection bactérienne à traiter, ainsi que du poids, de l'âge et de l'état de santé général du sujet à traiter, ou encore du jugement du médecin prescripteur.

Tel qu'utilisé ici, le terme « quantité sub-thérapeutique » se réfère à une quantité de principe actif insuffisante pour induire seule un effet thérapeutique. Alternativement, le terme « quantité sub-thérapeutique » peut se référer à une quantité de principe actif insuffisante pour induire seule un effet antibactérien.

Tel qu'utilisé ici, les termes de « combinaison thérapeutique » et « préparation combinée » se réfèrent à une association de principes actifs, de préférence du cinéol, de l'amoxicilline et optionnellement de l'acide clavulanique, qui peuvent être formulés chacun séparément ou en une ou plusieurs formulations pour une administration simultanée, séparée, séquentielle, ou un mélange de ces modes d'administrations lorsque la combinaison comprend plus de deux principes actifs.

Tel qu'utilisés ici, le terme « simultanée » se réfère à une combinaison selon l'invention dans laquelle les principes actifs de la combinaison sont utilisés ou administrés simultanément, c'est-à-dire en même temps.

Tel qu'utilisé ici, le terme « séquentielle » se réfère à une combinaison selon l'invention dans laquelle les principes actifs de la combinaison sont utilisés ou administrés séquentiellement, c'est-à-dire l'un après l'autre. De préférence, lorsque l'administration est séquentielle, l'ensemble des principes actifs est administré dans un intervalle d'au plus environ 1 heure, de préférence d'au plus environ 10 minutes, de manière encore préférée d'au plus environ 1 minute.

Tel qu'utilisé ici, le terme « séparée » se réfère à une combinaison selon l'invention dans laquelle les principes actifs de la combinaison sont utilisés ou administrés à des moments distincts de la journée. De préférence, lorsque l'administration est séparée, les principes actifs sont administrés à des intervalles d'environ 1 heure à environ 15 heures, de préférence d'environ 1 heure à environ 8 heures, de manière encore préférée d'environ 1 heure à environ 5 heures.

### Le cinéol

Tel qu'utilisé ici, le terme « cinéol », « eucalyptol » ou « 1,8 cinéol » se réfère à un éther cyclique (N° CAS : 470-82-6) appartenant au groupe des monoterpènes, c'est-à-dire des terpénoïdes ayant dix atomes de carbones. Le cinéol est un composé naturel, organique et incolore pouvant être notamment extrait des huiles essentielles de certains eucalyptus (par exemple *Eucalyptus polybractea*), du romarin (par exemple *Rosmarinus officinalis*), de l'armoise (par exemple *Artemisia vulgaris*), mais également des huiles essentielles d'absinthe, de laurier, de sauge, du basilic, et des feuilles de camphrier (*Cinnamomum camphora*). Le cinéol peut être utilisé sous toute forme pharmaceutiquement acceptable. Tel qu'utilisé ici, le terme « pharmaceutiquement acceptable » se réfère à une molécule, à un composé ou à une composition adapté à une administration pharmaceutique. Il est de préférence utilisé sous une forme purifiée. Sous sa forme purifiée, le cinéol est un liquide.

**L'amoxicilline**

L'amoxicilline (N° CAS : 26787-78-0) est un antibiotique β-lactamine bactéricide de la famille des aminopénicillines et qui est indiqué à ce jour dans le traitement des infections bactériennes à germes sensibles. Les β-lactamines sont une large classe d'antibiotiques comprenant les dérivés de la pénicilline, les céphalosporines, les monobactames et les carbapénèmes. Les β-lactamines sont caractérisées par la présence d'un noyau β-lactame dans leur structure moléculaire qui leur confère leur pouvoir bactéricide.

Dans la formulation selon l'invention, l'amoxicilline peut être sous toute forme pharmaceutiquement acceptable. L'amoxicilline peut ainsi être sous la forme d'un sel pharmaceutiquement acceptable, en particulier un sel de sodium ou de potassium, sous une forme anhydre ou hydratée, de préférence sous la forme d'un trihydrate, ou un mélange de ces formes. Dans un mode de réalisation préféré, la formulation selon l'invention comprend de l'amoxicilline trihydratée.

**Huile pharmaceutiquement acceptable**

La formulation pharmaceutique selon l'invention comprend également une huile pharmaceutiquement acceptable. Tel qu'utilisé ici, le terme « huile » se réfère à une phase constituée de corps gras liquides à température ambiante et ne se mélangeant pas avec l'eau.

L'huile utilisée dans la formulation divulguée dans ce document peut être toute huile pharmaceutiquement acceptable, c'est-à-dire toute huile dont les données toxicologiques sont compatibles avec l'administration par voie orale chez un sujet. Dans un aspect non revendiqué, l'huile est de préférence sélectionnée dans le groupe constitué des huiles animales, minérales, végétales, synthétiques et des mélanges de celles-ci

L'huile utilisée dans la combinaison n'est pas une huile essentielle. Tel qu'utilisé ici, le terme « huile essentielle » se réfère à un liquide concentré et hydrophobe des composés aromatiques (odoriférants) volatils d'une plante. L'huile essentielle peut être obtenue par extraction mécanique, par expression à froid, aux solvants volatils, au $CO_2$ supercritique, par entraînement à la vapeur d'eau ou distillation à sec.

L'huile peut être utilisée dans la formulation selon l'invention pour ses propriétés adsorbantes. Tel qu'utilisé ici, le terme « adsorbant » se réfère à un excipient capable de fixer des molécules liquides, par exemple des molécules de cinéol, sur un support solide, par exemple poudreux, dans une formulation pharmaceutique.

Dans un aspect non revendiqué, la formulation divulguée comprend une huile minérale. Les huiles minérales sont obtenues par distillation de la houille, du pétrole ou de certains schistes bitumineux. Les huiles minérales comprennent notamment des hydrocarbures, des alcanes et des paraffines. En particulier, l'huile minérale peut être sélectionnée dans le groupe constitué des paraffines raffinées, des cires microcristallines, des ozokérites, des ceresines, du pétrolatum, et un mélange de ceux-ci.

Dans un autre aspect non revendiqué, la formulation divulguée comprend une huile synthétique. En particulier, l'huile synthétique peut être sélectionnée dans le groupe constitué des huiles de silicone, des cires synthétiques, des mono-, di- et tri-glycérides synthétiques, par exemple les triglycérides capryliques capriques, et un mélange de ceux-ci.

Dans encore un autre aspect non revendiqué, la formulation divulguée comprend une huile animale. Cette huile animale peut être par exemple sélectionnée dans le groupe constitué de l'huile de vison, de cachalot, de baleine, de phoque, d'émeu, de pieds de bœuf, des huiles de poissons, en particulier l'huile d'anchois, de sardine, de capelan, de hareng, de saumon, de sprat, de morue, de merlan bleu, de pilchard, de thon, de requin, et un mélange de celles-ci.

Dans un mode de réalisation préféré, la formulation selon l'invention comprend une huile végétale, ladite huile végétale étant l'huile d'arachide. D'autres exemples non revendiqués d'huiles végétales incluent, sans y être limité, des huiles de germe de blé, de maïs, de tournesol, de karité, de ricin, d'amandes douces, de macadamia, d'abricot, de soja, de coton, de luzerne, de graines de pavot, de graines de potimarron, de sésame, de graines de courge, d'avocat, de noisette, de pépins de raisin, de pépins de cassis, d'onagre, de millet, d'orge, de quinoa, d'olive, d'arachide, de seigle, de carthame, de bancoulier, de passiflore, de rosier muscat, de coco, d'argan, de colza, de coprah, de lin, de noix, de noix de cajou, de margousier, de pistache, de riz, de cameline, de sacha inchi, de bourrache, de chanvre, de pois, de jojoba, de neem, de cumin noir, de perilla, et un mélange de celles-ci.

**Inhibiteur des β-lactamases**

La formulation pharmaceutique selon l'invention comprend en outre un ou plusieurs inhibiteurs des β-lactamases. Ces inhibiteurs peuvent bloquer l'activité des β-lactamases de différentes manières, par exemple en agissant comme un substrat suicide se fixant de manière irréversible à ces enzymes comme c'est notamment le cas pour l'acide clavulanique et le sulbactam.

L'inhibiteur des β-lactamases peut être tout inhibiteur des β-lactamases pharmaceutiquement acceptable. Cet inhibiteur est, de préférence, sélectionné dans le groupe constitué de l'acide clavulanique, du sulbactam, du tazobactam, de l'aztréonam, de l'avibactam, des sels pharmaceutiquement acceptables de ceux-ci, et des mélanges de ceux-ci.

Dans un mode de réalisation préféré, la formulation selon l'invention comprend de l'acide clavulanique. L'acide clavulanique (N° CAS : 58001-44-8) est couramment utilisé en combinaison avec les antibiotiques β-lactamines, notamment l'amoxicilline.

Dans la formulation selon l'invention, l'acide clavulanique peut être sous toute forme pharmaceutiquement acceptable, de préférence sous la forme d'un sel pharmaceutiquement acceptable, notamment sous la forme d'un sel potassique d'acide clavulanique.

**Formulation pharmaceutique**

Selon un premier aspect, la présente invention concerne une formulation pharmaceutique sous forme de poudre comprenant, ou consistant essentiellement en, du cinéol, de l'amoxicilline, un inhibiteur de β-lactamases et une huile pharmaceutiquement acceptable, ladite huile pharmaceutiquement acceptable étant l'huile d'arachide.

Tel qu'utilisé ici le terme de « poudre » se réfère à un état fractionné de la matière, il s'agit d'un état solide présent sous forme de petites particules.

De préférence, les particules de poudre ont un diamètre inférieur ou égal à environ 5 mm, de manière encore préférée inférieur ou égal à environ 2,5 mm, et de manière tout particulièrement préférée inférieur ou égal à environ 1,25 mm.

La poudre de la formulation pharmaceutique selon l'invention est de préférence une poudre sèche. Tel qu'utilisé ici, le terme de « poudre sèche » se réfère à une poudre ayant un taux d'humidité inférieur ou égal à 20 %, de préférence inférieur ou égal à 15 %, de manière encore préférée inférieur ou égal à 10 %.

La formulation pharmaceutique selon l'invention répond de préférence aux critères d'exigence de la Pharmacopée Européenne (8ème édition), notamment en terme d'impuretés et/ou en terme de qualité microbiologique.

En particulier, le taux de bactéries présent dans la poudre de la formulation pharmaceutique selon l'invention est de préférence inférieur à 10 000 UFC (Unité Formant Colonie) par gramme de poudre, de manière préférée inférieur à 1000 UFC par gramme de poudre, et de manière tout particulièrement préférée inférieur à 100 UFC par gramme de poudre. De préférence, la poudre de la formulation pharmaceutique selon l'invention ne contient pas d'*Escherichia coli.* Concernant les autres germes, le taux de champignons et levures est de préférence inférieur à 1000 UFC par gramme de poudre, de manière préférée inférieur à 100 UFC par gramme de poudre, et de manière tout particulièrement préférée inférieur à 50 UFC par gramme de poudre.

La formulation pharmaceutique selon l'invention comprend, ou consiste essentiellement en, du cinéol, de l'amoxicilline, et une huile pharmaceutiquement acceptable, tels que définis ci-dessus.

De préférence, l'amoxicilline et le cinéol sont présents dans la formulation selon l'invention à des concentrations permettant l'administration de doses suffisantes pour obtenir un effet thérapeutique et/ou un effet antibactérien, de préférence un effet synergique, tel qu'illustré dans les exemples ci-après. Pour obtenir un tel effet, l'amoxicilline et le cinéol peuvent être administrés en des quantités thérapeutiquement efficaces ou en des quantités sub-thérapeutiques.

Dans un mode de réalisation préféré, le cinéol et l'amoxicilline sont tous deux administrés en des quantités thérapeutiquement efficaces.

Dans un autre mode de réalisation préféré, l'amoxicilline est administrée en une quantité thérapeutiquement efficace et le cinéol est administré en une quantité sub-thérapeutique.

Dans encore un autre mode de réalisation préféré, l'amoxicilline est administrée en une quantité sub-thérapeutique et le cinéol est administré en une quantité thérapeutiquement efficace.

Dans encore un autre mode de réalisation préféré, l'amoxicilline et le cinéol sont tous deux administrés en des quantités sub-thérapeutiques.

De préférence, l'huile est présente dans la formulation selon l'invention à une concentration suffisante pour permettre l'adsorption du cinéol à la poudre de la formulation.

Selon un mode de réalisation, la formulation pharmaceutique selon l'invention comprend, ou consiste essentiellement en, entre environ 5 mg et environ 100 mg, de préférence entre environ 10 mg et environ 50 mg, de manière encore préférée entre environ 20 mg et environ 40 mg, de cinéol par gramme de poudre ; et/ou entre environ 20 mg et environ 500 mg, de préférence entre environ 50 mg et environ 300 mg, de manière encore préférée entre environ 150 mg et environ 200 mg, d'amoxicilline par gramme de poudre ; et/ou entre environ 2 mg et environ 50 mg, de préférence entre environ 10 mg et environ 25 mg, de manière encore préférée entre environ 15 mg et environ 20 mg, d'huile par gramme de poudre.

De préférence, la formulation pharmaceutique selon l'invention comprend, ou consiste essentiellement, entre environ 5 mg et environ 100 mg de cinéol, entre environ 20 mg et environ 500 mg d'amoxicilline, et entre environ 2 mg et environ 50 mg d'huile par gramme de poudre.

De manière encore préférée, la formulation pharmaceutique suivant l'invention comprend, ou consiste essentiellement, entre environ 10 mg et environ 50 mg de cinéol, entre environ 50 mg et environ 300 mg d'amoxicilline, et entre environ 10 mg et environ 25 mg d'huile par gramme de poudre.

De manière tout particulièrement préférée, la formulation pharmaceutique suivant l'invention comprend, ou consiste essentiellement en, entre environ 20 mg et environ 40 mg, de préférence environ 33 mg, de cinéol par gramme de poudre, entre environ 150 mg et environ 200 mg, de préférence environ 167 mg, d'amoxicilline par gramme de poudre, entre environ 15 mg et environ 20 mg, de préférence environ 17 mg, d'huile par gramme de poudre.

La formulation pharmaceutique selon l'invention comprend, ou consiste essentiellement en, du cinéol, de l'amoxicilline, une huile pharmaceutiquement acceptable et un inhibiteur de β-lactamases, tels que définis ci-dessus.

De préférence, l'inhibiteur de β-lactamases est sélectionné parmi l'acide clavulanique, le sulbactam, le tazobactam et l'aztréonam. De manière encore préférée, l'inhibiteur de β-lactamases est l'acide clavulanique.

Ainsi, la formulation pharmaceutique selon l'invention peut comprendre, ou consister essentiellement en, du cinéol, de l'amoxicilline et une huile pharmaceutiquement acceptable dans des proportions telles que décrites ci-dessus, et un inhibiteur de β-lactamases présent à une concentration suffisante pour permettre son administration en une quantité thérapeutiquement efficace ou en une quantité sub-thérapeutique.

Selon un mode de réalisation, la formulation pharmaceutique selon l'invention comprend, ou consiste essentiellement en, du cinéol, de l'amoxicilline et une huile pharmaceutiquement acceptable dans des proportions telles que décrites ci-dessus, et entre environ 1 mg et environ 100 mg, de préférence entre environ 5 mg et environ 50 mg, de manière encore préférée entre environ 15 mg et environ 25 mg, et de manière tout particulièrement préférée environ 20,8 mg, d'inhibiteur de β-lactamases, de préférence de l'acide clavulanique, par gramme de poudre.

Dans un autre mode de réalisation particulier, la formulation pharmaceutique selon l'invention comprend, ou consiste essentiellement en, du cinéol, de l'amoxicilline et une huile pharmaceutiquement acceptable, dans laquelle la proportion de cinéol est comprise entre environ 0,02 mg et environ 0,5 mg, de préférence entre environ 0,1 mg et environ 0,3 mg, de cinéol par gramme d'amoxicilline, et de manière encore préférée la proportion de cinéol est égale à environ 0,2 mg de cinéol par gramme d'amoxicilline ; et/ou la proportion d'huile est comprise entre environ 0,01 mg et environ 0,5 mg, de préférence entre environ 0,05 mg et environ 0,2 mg, d'huile par gramme d'amoxicilline, et de manière encore préférée la proportion d'huile est égale à environ 0,1 mg d'huile par gramme d'amoxicilline. De préférence, la formulation pharmaceutique selon l'invention comprend en outre un inhibiteur de β-lactamases, de préférence de l'acide clavulanique, la proportion d'inhibiteur de β-lactamases étant comprise entre environ 0,01 mg et environ 0,5 mg, de préférence entre 0,1 mg et environ 0,2 mg, d'inhibiteur de β-lactamases par gramme d'amoxicilline, et de manière encore préférée la proportion d'inhibiteur de β-lactamases est égale à environ 0,125 mg d'inhibiteur de β-lactamases par gramme d'amoxicilline.

Dans des modes de réalisation particuliers, le ratio massique amoxicilline/cinéol est compris entre 2 et 8, de préférence entre 3 et 7, de manière plus particulièrement préférée entre 4 et 6. Dans un mode de réalisation préféré, le ratio massique amoxicilline/cinéol est d'environ 5.

Dans des modes de réalisation particuliers, le ratio massique amoxicilline/inhibiteur de β-lactamases, de préférence acide clavulanique, est compris entre 5 et 11, de préférence entre 6 et 10, de manière plus particulièrement préférée entre 7 et 9. Dans un mode de réalisation préféré, le ratio massique amoxicilline/inhibiteur de β-lactamases, de préférence acide clavulanique, est d'environ 8.

Dans des modes de réalisation particuliers, le ratio massique amoxicilline/huile est compris entre 5 et 15, de préférence entre 7 et 13, de manière plus particulièrement préférée entre 8 et 12. Dans un mode de réalisation préféré, le ratio massique amoxicilline/cinéol est d'environ 10.

Dans des modes de réalisation particuliers, le ratio massique cinéol/huile est compris entre 0,1 et 5, de préférence entre 0.5 et 4, de manière plus particulièrement préférée entre 1 et 3. Dans un mode de réalisation préféré, le ratio massique cinéol/huile est d'environ 2.

La formulation pharmaceutique selon l'invention peut comprendre en outre au moins un autre principe actif.

Ainsi, dans un mode de réalisation particulier, la formulation pharmaceutique selon l'invention comprend, ou consiste essentiellement en, du cinéol, de l'amoxicilline, une huile pharmaceutiquement acceptable, éventuellement un inhibiteur de β-lactamases, de préférence de l'acide clavulanique, tels que définis ci-dessus, et un autre principe actif. De préférence, le principe actif supplémentaire de la formulation selon l'invention est un autre antibiotique, en particulier un antibiotique β-lactamine, et/ou un autre inhibiteur des β-lactamases, et/ou un antifongique, et/ou un antiparasitaire, et/ou un analgésique.

La formulation pharmaceutique selon l'invention peut comprendre, ou consister essentiellement en, du cinéol, de l'amoxicilline, une huile pharmaceutiquement acceptable, et optionnellement un inhibiteur de β-lactamases, de préférence de l'acide clavulanique, en des quantités telles que décrites ci-dessus et un autre principe actif présent à une concentration suffisante pour permettre son administration en une quantité thérapeutiquement efficace ou en une quantité sub-thérapeutique.

## Excipients

La formulation pharmaceutique selon l'invention peut comprendre en outre au moins un excipient ou support pharmaceutiquement acceptable en plus de l'huile pharmaceutiquement acceptable.

Cet excipient ou support pharmaceutiquement acceptable de la formulation selon l'invention est de préférence sélectionné dans le groupe consistant en un édulcorant, un aromatisant, un antiagglomérant, un lubrifiant, un désintégrant, et un mélange de ceux-ci.

Ainsi, dans un mode de réalisation particulier, la formulation pharmaceutique selon l'invention comprend en outre au moins un désintégrant.

Tel qu'utilisé ici, le terme « désintégrant » se réfère à un excipient qui permet d'améliorer la désintégration, c'est-à-dire la séparation des molécules présentes dans la formulation pharmaceutique en milieu liquide, de préférence un milieu aqueux, et leur suspension homogène.

De préférence, le désintégrant est sélectionné dans le groupe constitué des celluloses microcristallines, des carboxyméthylamidons réticulés, des polyvinylpyrrolidones réticulés et des carboxyméthylcelluloses réticulés.

Tel qu'utilisés ici les termes de « carboxyméthylcellulose réticulé » et « croscarmellose » sont équivalents et peuvent être employés l'un pour l'autre.

Dans un mode de réalisation préféré, la formulation pharmaceutique selon l'invention comprend entre environ 10 mg et environ 1000 mg, de préférence entre environ 100 mg et environ 600 mg, de manière encore préférée entre environ 450 mg et environ 550 mg, par exemple environ 513,7 mg, de désintégrant par gramme de poudre.

En particulier, la formulation peut comprendre une cellulose microcristalline, de préférence sous forme d'une poudre constituée de particules ayant un diamètre d'environ 20 à environ 200 $\mu$m, de manière encore préférée un diamètre d'environ 50 à environ 100 $\mu$m. En particulier, la cellulose microcristalline est sélectionnée parmi les celluloses microcristallines de type Avicel®, Emcocel® et Vitacel®, ou des mélanges de celles-ci, de préférence la cellulose microcristalline est sélectionnée parmi les celluloses microcristallines Avicel PH 101,102, 103,104, 112,113, 301 et 302, et de manière tout particulièrement préférée la cellulose microcristalline est de type Avicel PH 112.

Ainsi, dans un mode de réalisation particulier, la formulation pharmaceutique selon l'invention comprend entre environ 100 mg et environ 800 mg, de préférence entre environ 300 mg et environ 500 mg, de manière encore préférée entre environ 400 mg et environ 420 mg, par exemple environ 413,7 mg, de cellulose microcristalline par gramme de poudre.

Alternativement, la formulation peut comprendre une croscarmellose. En particulier, la croscarmellose peut être sous forme d'une poudre formée de particules ayant un diamètre moyen inférieur à 36 pm. De préférence, la croscarmellose est une croscarmellose sodique.

Dans un autre mode de réalisation particulier, la formulation pharmaceutique selon l'invention comprend entre environ 10 mg et environ 250 mg, de préférence entre environ 50 mg et environ 150 mg, de manière encore préférée entre environ 80 mg et 120 mg, par exemple environ 100 mg de croscarmellose par gramme de poudre.

Selon un mode de réalisation particulièrement préféré, la formulation pharmaceutique selon l'invention comprend de la cellulose microcristalline et de la croscarmellose tel que définie ci-dessus. Ainsi, la formulation pharmaceutique selon l'invention peut comprendre entre environ 100 mg et environ 800 mg, de préférence entre environ 300 mg et environ 500 mg, de manière encore préférée entre environ 400 mg et environ 420 mg, par exemple environ 413,7 mg, de cellulose microcristalline par gramme de poudre ; et/ou entre environ 10 mg et environ 250 mg, de préférence entre environ 50 mg et environ 150 mg, de manière encore préférée entre environ 80 mg et environ 120 mg, par exemple environ 100 mg de croscarmellose par gramme de poudre.

La formulation pharmaceutique selon l'invention peut également comprendre au moins un excipient de type antiagglomérant.

Tel qu'utilisé ici, le terme « antiagglomérant » se réfère à un excipient qui limite l'agglutination des particules dans un produit en poudre et ainsi assure sa fluidité.

De préférence, l'antiagglomérant est sélectionné dans le groupe constitué du talc, de la silice et de ses dérivés, du carbonate de sodium, du carbonate d'ammonium, du bicarbonate d'ammonium, du carbonate de magnésium, du ferrocyanure de sodium, du ferrocyanure de potassium, du ferrocyanure de calcium, ou des mélanges de ceux-ci.

De manière encore préférée, l'antiagglomérant est sélectionné dans le groupe constitué de la silice et de ses dérivés, en particulier la silice, la silice colloïdale, le dioxyde de silicium, le silicate de calcium, le silicate de magnésium, le silicate alumino-sodique, le silicate alumino-potassique, le silicate alumino-calcique, le silicate de zinc, le silicate d'aluminium, ou des mélanges de ceux-ci.

De manière tout particulièrement préférée, l'antiagglomérant est de la silice, de préférence de la silice synthétique amorphe, en particulier du syloid® Al-1 FP.

Dans un mode de réalisation préféré, la formulation pharmaceutique selon l'invention comprend entre environ 20 mg et environ 500 mg, de préférence entre environ 50 mg et environ 300 mg, de manière encore préférée entre environ 150 mg et environ 200 mg, par exemple environ 180 mg d'antiagglomérant, de préférence de la silice, par gramme de poudre.

La formulation pharmaceutique selon l'invention peut également comprendre au moins un excipient de type lubrifiant.

Tel qu'utilisé ici, le terme « lubrifiant » se réfère à un excipient destiné à faciliter les étapes de fabrication de la poudre, notamment grâce à ses effets glissant, anti-adhérent et antifriction.

De préférence, le lubrifiant est sélectionné dans le groupe constitué du stéarate de magnésium, du stéarate d'aluminium, du stéarate de calcium, du stéarate de sodium, du stéarate de zinc, du stéarylfumarate de sodium, du propylenglycol, du monostéarate de glycérol ou des mélanges de ceux-ci. De manière encore préférée, le lubrifiant est du stéarate de magnésium.

Selon un mode de réalisation préféré, la formulation pharmaceutique selon l'invention comprend entre environ 1 mg et environ 40 mg, de préférence entre environ 2 mg et environ 15 mg, de manière encore préférée entre environ 4 mg et environ 10 mg, par exemple environ 6 mg de lubrifiant, de préférence du stéarate de magnésium, par gramme de poudre.

La formulation pharmaceutique selon l'invention peut également comprendre au moins un excipient de type édulcorant.

Tel qu'utilisé ici, le terme « édulcorant » se réfère à un excipient destiné à changer le goût d'une formulation pharmaceutique en lui conférant une saveur sucrée.

De préférence, l'édulcorant est sélectionné dans le groupe constitué de l'acésulfame potassium (E950), l'alitame (E956), l'aspartame (E951), la cyclamate (E952), la néotame, la saccharine (E954), le sel d'aspartame-acésulfame (E962), le sucralose, la thaumatine, les polyols, la brazzéine, la curculine, la glycyrrhizine, un hydrolysat d'amidon hydrogéné, la mabinline, la miraculine, la monelline, la pentadine, le stevia, le tagatose, le tréhalose, l'isomaltulose, l'érythritol, et des mélanges de ceux-ci. De manière particulièrement préférée, l'édulcorant est l'aspartame.

Selon un mode de réalisation préféré, la formulation pharmaceutique selon l'invention comprend entre environ 1 mg et environ 60 mg, de préférence entre environ 3 mg et environ 20 mg, de manière encore préférée la formulation pharmaceutique de l'invention comprend entre environ 8 mg et environ 15 mg, par exemple environ 12 mg d'édulcorant, de préférence de l'aspartame, par gramme de poudre.

La formulation pharmaceutique selon l'invention peut également comprendre au moins un excipient de type aromatisant.

Tel qu'utilisé ici, le terme « aromatisant » se réfère à un excipient destiné à changer le goût d'une formulation pharmaceutique en lui conférant un arôme.

De préférence, l'aromatisant est sélectionné dans le groupe constitué de l'arôme de fraise, de framboise, de cerise, de banane, de citron, d'orange, de pêche, de pomme, de caramel, ou des mélanges de ceux-ci. De manière encore préféré, l'aromatisant est un mélange d'arômes citron, fraise et pêche.

Selon un mode de réalisation préféré, la formulation pharmaceutique selon l'invention comprend entre environ 1 mg et environ 100 mg, de préférence entre environ 10 mg et environ 50 mg, de manière encore préférée entre environ 20 mg et environ 40 mg, par exemple environ 30 mg, d'aromatisant par gramme de poudre.

Afin de prévenir les risques de contamination microbiologique, la formulation pharmaceutique selon l'invention peut également comprendre un agent conservateur.

Les agents conservateurs utilisables dans la formulation selon l'invention comprennent, sans y être limités, l'acide benzoïque et ses sels sodiques ou potassique tels que le benzoate de sodium ; les parabènes tels que le méthylparabène, le propylparabène ou le butylparabène ; l'acide sorbique et ses sels sodiques ou potassique tels que le sorbate de potassium ; des ammoniums quaternaires tels que le chlorure de benzalkonium ; des dérivés mercuriels tels que les sels de phénylmercure (acétate, borate ou nitrate) ou le thiomersal ; et une combinaison de ceux-ci.

La formulation pharmaceutique suivant l'invention peut également comprendre un excipient de type tampon pH, tels que différents acides et leurs sels, comme par exemple l'acide citrique, le citrate sodique et l'acide succinique.

La formulation peut encore comprendre un colorant, notamment afin d'augmenter son acceptabilité vis-à-vis des enfants. De préférence, le colorant est utilisé pour renforcer la crédibilité de l'arôme (par exemple un colorant rose pour un arôme fraise).

Dans un mode de réalisation particulièrement préféré, la formulation pharmaceutique suivant l'invention ne comprend pas de détergent. Le terme de « détergent » (ou « surfactant »), tel qu'employé ici se réfère à une molécule amphiphile dotée de propriétés tensioactives. Des détergents couramment utilisés dans les formulations pharmaceutiques sont par exemple des détergents ioniques tel que le sodium dodecyl sulfate (SDS), le desoxycholate et le cholate, des détergents non-ionique tel que le triton X-100, le n-Dodecyl β-D-Maltopyranoside (DDM), la digitonine, le tween 20 et le tween 80, et des détergents amphotériques tel que le 3-[(3-cholamidopropyl)diméthylammonio]-1-propanesulfonate (CHAPS) ou le 3-[diméthylammonio]-1-propanesulfonate.

Dans un autre mode de réalisation préféré, la formulation pharmaceutique selon l'invention est sous forme d'une poudre comprenant, ou consistant essentiellement en, ou consistant en, du cinéol, de l'amoxicilline, une huile pharmaceutiquement acceptable, et au moins un édulcorant, un aromatisant, un antiagglomérant, un lubrifiant et/ou un désintégrant tels que définis ci-dessus et, optionnellement, un inhibiteur de β-lactamases, de préférence de l'acide clavulanique, tel que défini ci-dessus.

De préférence, la formulation pharmaceutique selon l'invention est sous forme d'une poudre comprenant, ou consistant essentiellement en, ou consistant en, du cinéol, de l'amoxicilline, et de l'huile dans les proportions décrites ci-dessus, et entre environ 1 mg et environ 60 mg d'édulcorant, de préférence de l'aspartame, et/ou entre environ 1 mg et environ 60 mg d'aromatisant, et/ou entre environ 20 mg et environ 500 mg d'antiagglomérant, de préférence de la silice, et/ou entre environ 1 mg et environ 40 mg de lubrifiant, de préférence du stéarate de magnésium, et/ou entre environ 10 mg et environ 1000 mg de désintégrant, de préférence un mélange de cellulose microcristalline et de croscarmellose, par gramme de poudre et, optionnellement, un inhibiteur de β-lactamases, de préférence de l'acide clavulanique, dans les proportions décrites ci-dessus.

De manière encore préférée, la formulation pharmaceutique suivant l'invention est sous forme d'une poudre comprenant, ou consistant essentiellement en, ou consistant en, du cinéol, de l'amoxicilline, et de l'huile dans les proportions décrites ci-dessus, et entre environ 3 mg et environ 20 mg d'édulcorant, de préférence de l'aspartame, et/ou entre environ 10 mg et environ 50 mg d'aromatisant, et/ou entre environ 50 mg et environ 300 mg d'antiagglomérant, de préférence de la silice, et/ou entre environ 2 mg et environ 15 mg de lubrifiant, de préférence du stéarate de magnésium, et/ou entre environ 100 mg et environ 600 mg de désintégrant, de préférence un mélange de cellulose microcristalline et de croscarmellose, par gramme de poudre, et, optionnellement, un inhibiteur de β-lactamases, de préférence de l'acide clavulanique, dans les proportions décrites ci-dessus.

De manière tout particulièrement préférée, la formulation pharmaceutique suivant l'invention est sous forme d'une poudre comprenant, ou consistant essentiellement en, ou consistant en, du cinéol, de l'amoxicilline, et de l'huile dans les proportions décrites ci-dessus, et entre environ 8 mg et environ 15 mg, par exemple environ 12 mg, d'édulcorant, de préférence de l'aspartame, et/ou entre environ 20 mg et environ 40 mg, par exemple environ 30 mg, d'aromatisant par gramme de poudre, et/ou entre environ 150 mg et environ 200 mg, par exemple environ 180 mg, d'antiagglomérant, de préférence de la silice, et/ou entre environ 4 mg et environ 8 mg, par exemple environ 6 mg de lubrifiant, de préférence du stéarate de magnésium, et/ou entre environ 450 mg et environ 550 mg, par exemple environ 513,7 mg, de désintégrant, par exemple environ 100 mg de croscarmellose et environ 413,7 mg de cellulose microcristalline, et, optionnellement, un inhibiteur de β-lactamases, de préférence de l'acide clavulanique, dans les proportions décrites ci-dessus.

**Conditionnement, stabilité et voie d'administration de la formulation selon l'invention**

Les inventeurs ont démontré que la formulation pharmaceutique selon l'invention est particulièrement stable. La stabilité d'un produit se caractérise par le fait que la qualité dudit produit ne varie pas pendant un intervalle de temps donné sous l'effet de divers facteurs environnementaux, en particulier de la température et de l'humidité (norme ICH). L'absence de variation peut être constatée pour un pourcentage final en amoxicilline, en cinéol, et éventuellement en acide clavulanique n'ayant pas varié, de préférence, de plus de 5 % par rapport aux valeurs initiales.

Ainsi, dans un mode de réalisation particulier, la formulation pharmaceutique selon l'invention est stable pendant au moins 18 mois, de préférence pendant au moins 24 mois, de manière encore préférée pendant au moins 36 mois à une température d'environ 25°C et sous une humidité relative d'au moins environ 60 %.

Dans un autre mode de réalisation particulier, la formulation pharmaceutique selon l'invention est stable pendant au moins 6 mois, de préférence pendant au moins 12 mois, de manière encore préférée pendant au moins 24 mois à une température d'environ 30°C et sous une humidité relative d'au moins environ 65 %.

Dans encore un autre mode de réalisation particulier, la formulation pharmaceutique selon l'invention est stable pendant au moins 3 mois, de préférence pendant au moins 6 mois, de manière encore préférée pendant au moins 12 mois à une température d'environ 40°C et sous une humidité relative d'au moins environ 75 %.

Des enceintes climatiques, bien connues de l'homme du métier, permettent de soumettre la formulation pharmaceutique selon l'invention aux conditions de température et d'humidité relative mentionnées ci-dessus.

Tel qu'utilisé ici, le terme « humidité relative » se réfère au rapport de la pression partielle de la vapeur d'eau contenue dans l'air sur la pression de vapeur saturante (ou tension de vapeur) à la même température.

La formulation pharmaceutique selon l'invention est destinée à une administration par voie orale.

De préférence, la formulation pharmaceutique est administrée après suspension dans un solvant aqueux puis mélange, de préférence le solvant aqueux est de l'eau. De manière particulièrement préférée, la formulation pharmaceutique est mise en solution extemporanément. La formulation pharmaceutique selon l'invention est stable après suspension dans un milieu aqueux, pendant au moins 48 h, de préférence pendant au moins 72 h, de manière encore préférée pendant au moins 96 h à une température inférieure ou égale à environ 20°C et sous une humidité relative inférieure ou égale à environ 15 %.

Après mise en suspension dans un solvant aqueux, de préférence de l'eau, la formulation pharmaceutique de l'invention peut former une solution au pH légèrement acide. De préférence, le pH de la solution formée par la suspension de la formulation pharmaceutique de l'invention dans un solvant aqueux est compris entre un pH d'environ 5 et un pH d'environ 7.

La formulation pharmaceutique selon l'invention peut être conditionnée dans un récipient unidose ou multi-dose, de préférence dans un récipient unidose.

Dans un mode de réalisation préféré, la formulation pharmaceutique selon l'invention est conditionnée dans un récipient unidose contenant entre environ 1 g et environ 150 g de poudre, de manière encore préférée entre environ 1 g et environ 50 g de poudre, de manière toujours préférée entre environ 1 g et environ 10 g de poudre, et de manière plus particulièrement préférée environ 3 g de poudre.

Lorsque la formulation pharmaceutique est conditionnée dans un récipient unidose, lesdits récipients unidoses peuvent être secondairement conditionnés dans une boîte. Une boîte peut contenir par exemple entre 3 et 31 récipients unidoses, de préférence entre 5 et 21 récipients unidoses, de manière encore préféré entre 7 et 14 récipients uni-doses.

Dans un autre mode de réalisation, la formulation pharmaceutique selon l'invention est conditionnée dans un récipient multi-dose. Ledit récipient peut contenir par exemple entre environ 10 grammes et environ 500 grammes de poudre, de préférence entre environ 20 grammes et environ 200 grammes de poudre, de manière encore préférée entre environ 30 grammes de poudre et environ 100 grammes de poudre, et de manière tout particulièrement préférée environ 50 grammes de poudre.

Lorsque la formulation pharmaceutique est conditionnée dans un récipient multi-dose, celui-ci peut être secondairement conditionné dans une boîte, éventuellement accompagné d'un doseur, par exemple une cuillère, permettant de prélever une quantité déterminée de poudre, de préférence d'environ 1 mg à environ 30 mg de poudre, de manière encore préférée d'environ 2 mg à environ 20 mg de poudre, et de manière encore préférée d'environ 3 à environ 12 mg de poudre. En particulier, le doseur peut permettre de prélever environ 3 g, environ 6 g, environ 9 g, environ 12 g, environ 15 g et/ou environ 18 g de poudre.

### Combinaison de cinéol et d'amoxicilline

Dans un second aspect non revendiqué, la présente demande divulgue une combinaison comprenant, ou consistant essentiellement en, du cinéol et de l'amoxicilline pour une utilisation dans le traitement d'une infection bactérienne chez un sujet.

De préférence, dans la combinaison divulguée, l'amoxicilline et le cinéol sont administrés à des doses permettant d'obtenir un effet thérapeutique et/ou un effet antibactérien, de préférence synergique, tel qu'illustré dans les exemples ci-après. Pour obtenir un tel effet, l'amoxicilline et/ou le cinéol peuvent être administrés en des quantités thérapeutiquement efficaces ou en des quantités sub-thérapeutiques.

Dans un aspect, le cinéol et l'amoxicilline sont tous deux administrés en des quantités thérapeutiquement efficaces.

Dans un autre aspect, l'amoxicilline est administrée en une quantité thérapeutiquement efficace et le cinéol est administré en une quantité sub-thérapeutique.

Dans encore un autre aspect, l'amoxicilline est administrée en une quantité sub-thérapeutique et le cinéol est administré en une quantité thérapeutiquement efficace.

Dans encore un autre aspect, l'amoxicilline et le cinéol sont tous deux administrés en des quantités sub-thérapeutiques.

Selon un aspect, le cinéol peut être administré au sujet à une dose comprise entre environ 0,1 mg/kg/jour et environ 50 mg/kg/jour, de préférence entre environ 0,5 mg/kg/jour et environ 20 mg/kg/jour, et de manière tout particulièrement préférée entre environ 1 mg/kg/jour et environ 10 mg/kg/jour de poids du sujet ; et/ou l'amoxicilline peut être administrée au sujet à une dose comprise entre environ 5 mg/kg/jour et environ 200 mg/kg/jour, de préférence entre environ 10 mg/kg/jour et environ 100 mg/kg/jour, et de manière tout particulièrement préférée entre environ 15 mg/kg/jour et environ 50 mg/kg de poids du sujet par jour.

De préférence, le cinéol de la combinaison divulguée dans la présente demande est administré au sujet à une dose comprise entre environ 0,1 mg/kg/jour et environ 50 mg/kg/jour et l'amoxicilline de la combinaison divulguée est administrée au sujet à une dose comprise entre environ 5 mg/kg/jour et environ 200 mg/kg de poids du sujet par jour.

De manière encore préférée, le cinéol de la combinaison divulguée dans la présente demande est administré au sujet à une dose comprise entre environ 0,5 mg/kg/jour et environ 20 mg/kg/jour et l'amoxicilline de la combinaison divulguée est administrée au sujet à une dose comprise entre environ 10 mg/kg/jour et environ 100 mg/kg de poids du sujet par jour.

De manière tout particulièrement préférée, le cinéol de la combinaison divulguée dans la présente demande est administré au sujet à une dose comprise entre environ 1 mg/kg/jour et environ 10 mg/kg/jour et l'amoxicilline de la combinaison divulguée est administrée au sujet à une dose comprise entre environ 15 mg/kg/jour et environ 50 mg/kg de poids du sujet par jour.

Dans un aspect, la combinaison divulguée dans la présente demande comprend, ou consiste essentiellement en, du cinéol, de l'amoxicilline et un inhibiteur de β-lactamases, de préférence sélectionné parmi l'acide clavulanique, le sulbactam, le tazobactam et l'aztréonam, et des mélanges de ceux-ci, pour une utilisation dans le traitement d'une infection bactérienne chez un sujet. De préférence, l'inhibiteur de β-lactamases est l'acide clavulanique.

Ainsi, le cinéol et l'amoxicilline de la combinaison divulguéepeuvent être administrées au sujet en des quantités telles que décrites ci-dessus et l'inhibiteur de β-lactamase peut être administré au sujet en une quantité thérapeutiquement efficace ou en une quantité sub-thérapeutique.

Selon un aspect particulier, le cinéol et l'amoxicilline de la combinaison selon l'invention sont administrées au sujet à des doses telles que décrites ci-dessus et l'inhibiteur de β-lactamases, de préférence de l'acide clavulanique, est administrée au sujet à une dose comprise entre environ 0,1 mg/kg/jour et environ 50 mg/kg/jour, de préférence entre environ 0,5 mg/kg/jour et environ 20 mg/kg/jour, et de manière tout particulièrement préférée entre environ 1 mg/kg/jour et environ 10 mg/kg de poids du sujet par jour.

Dans un autre aspect particulier, la combinaison divulguée dans la présente demande comprend, ou consiste essentiellement en, du cinéol et de l'amoxicilline, la proportion de cinéol étant comprise entre environ 0,02 mg et environ 0,5 mg, de préférence entre environ 0,1 mg et environ 0,3 mg, de cinéol par gramme d'amoxicilline, et de manière encore préférée la proportion de cinéol est égale à environ 0,2 mg de cinéol par gramme d'amoxicilline. De préférence, la combinaison divulguée comprend en outre un inhibiteur de β-lactamases, de préférence de l'acide clavulanique, la proportion d'inhibiteur de β-lactamases étant comprise entre environ 0,01 mg et environ 0,5 mg, de préférence entre 0,1 mg et environ 0,2 mg, d'inhibiteur de β-lactamases par gramme d'amoxicilline, et de manière encore préférée la proportion d'inhibiteur de β-lactamases est égale à environ 0,125 mg d'inhibiteur de β-lactamases par gramme d'amoxicilline.

La combinaison divulguée dans la présente demande peut comprendre en outre au moins un autre principe actif. De préférence, le principe actif supplémentaire de la combinaison divulguée est un autre antibiotique, en particulier un antibiotique β-lactamine, et/ou un autre inhibiteur des β-lactamases, et/ou un antifongique, et/ou un antiparasitaire, et/ou un analgésique.

Ainsi, le cinéol, l'amoxicilline, et optionnellement un inhibiteur de β-lactamases, de préférence de l'acide clavulanique, de la combinaison divulguée peuvent être administrées au sujet en des quantités telles que décrites ci-dessus et le ou les autres principes actifs peuvent être administrés au sujet en des quantités thérapeutiquement efficaces ou en des quantités sub-thérapeutiques.

## Conditionnement et voie d'administration de la combinaison selon l'invention

Les principes actifs de la combinaison divulguée dans la présente demande peuvent être administrés au sujet de façon simultanée, séquentielle, séparée ou un mélange de ces modes d'administration.

Lorsque des principes actifs de la combinaison divulguée sont administrés de façon séquentielle ou séparée, le ou les intervalles de temps sont de préférence choisis afin de permettre l'obtention de l'effet thérapeutique recherché, avec de préférence un effet synergique entre le cinéol et l'amoxicilline.

De préférence, les principes actifs de la combinaison divulguée sont administrés de façon simultanée. Lorsque l'administration des principes actifs de la combinaison divulguée est simultanée, elle se fait de préférence par l'administration d'une seule formulation comprenant l'ensemble des principes actifs de la combinaison.

Lorsque la combinaison divulguée comprend plus de deux principes actifs, certains principes actifs peuvent être administrés de façon simultanée, certains principes actifs peuvent être administrés de façon séquentielle, et/ou certains principes actifs peuvent être administrés de façon séparée. Par exemple, lorsque la combinaison divulguée comprend trois principes actifs, deux principes actifs peuvent être administrés simultanément et le troisième administré de façon séquentielle ou séparée, de préférence de façon séquentielle.

Dans un aspect particulier, la combinaison divulguée comprend du cinéol et de l'amoxicilline administrés au sujet de façon simultanée, de préférence à partir d'une composition pharmaceutique comprenant du cinéol et de l'amoxicilline, alternativement à partir de deux compositions pharmaceutiques, l'une comprenant du cinéol et l'autre comprenant de l'amoxicilline.

Dans un autre aspect particulier, la combinaison divulguée comprend du cinéol et de l'amoxicilline administrés au sujet de façon séquentielle ou séparée, de préférence de façon séquentielle, à partir de deux compositions pharmaceutiques, l'une comprenant du cinéol et l'autre comprenant de l'amoxicilline.

Dans encore un autre aspect particulier, la combinaison divulguée comprend du cinéol, de l'amoxicilline et un inhibiteur de β-lactamases, de préférence de l'acide clavulanique, administrés au sujet de façon simultanée. Le cinéol, l'amoxicilline et l'inhibiteur de β-lactamases peuvent être administrés au sujet simultanément à partir :

(a) d'une composition pharmaceutique comprenant du cinéol, de l'amoxicilline et un inhibiteur de β-lactamases ;

(b) d'une composition pharmaceutique comprenant du cinéol et d'une composition pharmaceutique comprenant de l'amoxicilline et un inhibiteur de β-lactamases ;

(c) d'une composition pharmaceutique comprenant de l'amoxicilline et d'une composition pharmaceutique comprenant du cinéol et un inhibiteur de β-lactamases ;

(d) d'une composition pharmaceutique comprenant un inhibiteur de β-lactamases, et d'une composition pharmaceutique comprenant du cinéol et de l'amoxicilline; ou

(e) d'une composition pharmaceutique comprenant du cinéol, d'une composition pharmaceutique comprenant de l'amoxicilline, et d'une composition pharmaceutique comprenant un inhibiteur de β-lactamases.

Dans encore un autre aspect particulier, la combinaison divulguée comprend du cinéol, de l'amoxicilline et un inhibiteur de β-lactamases, de préférence de l'acide clavulanique, administrés au sujet de façon séquentielle ou séparée, de préférence de façon séquentielle, à partir d'une composition pharmaceutique comprenant du cinéol, d'une composition pharmaceutique comprenant de l'amoxicilline, et d'une composition pharmaceutique comprenant un inhibiteur de β-lactamases.

Dans encore un autre aspect particulier, la combinaison divulguée comprend du cinéol, de l'amoxicilline et un inhibiteur de β-lactamases, de préférence de l'acide clavulanique, dans laquelle l'amoxicilline et l'inhibiteur de β-lactamases sont administrés au sujet simultanément et le cinéol est administré au sujet de façon séquentielle ou séparée, de préférence de façon séquentielle, à partir :

(a) d'une composition pharmaceutique comprenant du cinéol, d'une composition pharmaceutique comprenant de l'amoxicilline, et d'une composition pharmaceutique comprenant un inhibiteur de β-lactamases ; ou
(b) d'une composition pharmaceutique comprenant du cinéol et d'une composition pharmaceutique comprenant de l'amoxicilline et un inhibiteur de β-lactamases.

Dans encore un autre aspect particulier, la combinaison divulguée comprend du cinéol, de l'amoxicilline et un inhibiteur de β-lactamases, de préférence de l'acide clavulanique, dans laquelle le cinéol et l'inhibiteur de β-lactamases sont administrés au sujet simultanément et l'amoxicilline est administrée au sujet de façon séquentielle ou séparée, de préférence de façon séquentielle, à partir :

(a) d'une composition pharmaceutique comprenant du cinéol, d'une composition pharmaceutique comprenant de l'amoxicilline, et d'une composition pharmaceutique comprenant un inhibiteur de β-lactamases ; ou
(b) d'une composition pharmaceutique comprenant de l'amoxicilline et d'une composition pharmaceutique comprenant du cinéol et un inhibiteur de β-lactamases.

Dans encore un autre aspect particulier, la combinaison divulguée comprend du cinéol, de l'amoxicilline et un inhibiteur de β-lactamases, de préférence de l'acide clavulanique, dans laquelle l'amoxicilline et le cinéol sont administrés au sujet simultanément et l'inhibiteur de β-lactamases est administré au sujet de façon séquentielle ou séparée, de préférence de façon séquentielle, à partir :

(a) d'une composition pharmaceutique comprenant du cinéol, d'une composition pharmaceutique comprenant de l'amoxicilline, et d'une composition pharmaceutique comprenant un inhibiteur de β-lactamases ; ou
(b) d'une composition pharmaceutique comprenant un inhibiteur de β-lactamases, et d'une composition pharmaceutique comprenant du cinéol et de l'amoxicilline.

Dans un aspect, l'amoxicilline et un inhibiteur de β-lactamases, de préférence de l'acide clavulanique, sont administrés au sujet de façon simultanée, de préférence à partir d'une composition pharmaceutique comprenant de l'amoxicilline et un inhibiteur de β-lactamases.
Dans un autre aspect, le cinéol, l'amoxicilline et optionnellement un inhibiteur de β-lactamases, de préférence de l'acide clavulanique, sont administrés au sujet simultanément, de préférence à partir d'une composition pharmaceutique comprenant du cinéol, de l'amoxicilline et optionnellement un inhibiteur de β-lactamases, ou à partir d'une composition pharmaceutique comprenant du cinéol et d'une composition pharmaceutique comprenant de l'amoxicilline et optionnellement un inhibiteur de β-lactamases.
Dans encore un autre aspect, le cinéol est administré au sujet séquentiellement ou séparément, de préférence séquentiellement par rapport à l'amoxicilline et/ou, optionnellement, par rapport à l'inhibiteur de β-lactamases, de préférence de l'acide clavulanique.
Les principes actifs de la combinaison peuvent être administrés au sujet par des voies identiques ou différentes. Les voies d'administration dépendent, en général, des formulations pharmaceutiques utilisées. Les principes actifs de la combinaison selon l'invention sont, de préférence, administrés au sujet par voie parentérale ou par voie entérale, de préférence par voie entérale, de manière encore préférée par voie orale ou rectale. De manière particulièrement préférée, les principes actifs de la combinaison sont administrés au sujet par voie orale.
Dans un aspect particulier, l'amoxicilline et, optionnellement, un inhibiteur de β-lactamases, de préférence de l'acide clavulanique, sont administrés au sujet par voie orale et le cinéol est administré au sujet par voie rectale.
Dans un aspect préféré, l'amoxicilline, le cinéol et, optionnellement, un inhibiteur de β-lactamases, de préférence de l'acide clavulanique, sont administrés au sujet par voie orale.
Les principes actifs de la combinaison, en particulier l'amoxicilline, le cinéol, et éventuellement un inhibiteur de β-lactamases, de préférence de l'acide clavulanique, peuvent être administrés au sujet sous la forme de toute formulation pharmaceutique, de préférence compatible avec une administration par voie parentérale ou par voie entérale, de manière encore préférée compatible avec une administration par voie orale ou par voie rectale, de manière tout particulièrement préférée compatible avec une administration par voie orale.

Les principes actifs de la combinaison en particulier l'amoxicilline, le cinéol, et éventuellement un inhibiteur de β-lactamases, de préférence de l'acide clavulanique, peuvent ainsi être formulés sous forme de comprimés, capsules, gélules, granulats, poudre, suspensions, émulsions, solutions, polymères, nanoparticules, microsphères, suppositoires, capsules rectales, lavements, gels, pates, onguents, crèmes, emplâtres, potions, injectables, implants, sprays ou aérosols.

Dans un aspect, les principes actifs de la combinaison selon l'invention sont formulés sous forme de poudre, de préférence des poudres pour suspensions aqueuses buvables.

Dans un aspect particulier, les principes actifs sont formulés sous forme de suppositoires ou de capsules rectales.

Dans un autre aspect particulier, le cinéol est formulé sous forme d'huile, ou sous forme de suppositoire ou de capsule rectale, de préférence sous forme d'huile encapsulée, et les autres principes actifs sont formulés sous forme de poudres, de préférence des poudres pour suspensions aqueuses buvables.

## Traitement d'une pathologie infectieuse chez un sujet

Dans un autre aspect, la présente invention concerne également une formulation pharmaceutique selon l'invention pour une utilisation dans le traitement d'une pathologie infectieuse chez un sujet. Elle concerne aussi une formulation pharmaceutique selon l'invention pour la préparation d'un médicament destiné au traitement d'une pathologie infectieuse. Elle concerne également une méthode de traitement comprenant l'administration d'une quantité thérapeutiquement efficace de la formulation selon l'invention à un sujet en ayant besoin, en particulier un sujet souffrant d'une pathologie infectieuse.

La présente demande concerne encore une combinaison telle que divulguée ici pour une utilisation dans le traitement pathologie infectieuse, de préférence une infection bactérienne, chez un sujet..

De préférence, la pathologie infectieuse est d'origine bactérienne, de manière encore préférée la pathologie infectieuse est causée par une ou plusieurs bactéries résistantes aux antibiotiques, en particulier aux antibiotiques de la famille des β-lactamines. De préférence, le terme « bactérie résistante aux antibiotiques» se réfère à une bactérie qui, selon les cas, est insensible ou peu sensible aux antibiotiques. De même, une bactérie résistante aux antibiotiques de la famille des β-lactamines est insensible ou peu sensible aux antibiotiques de cette famille. Une infection bactérienne par une bactérie résistante aux antibiotiques de la famille des β-lactamines ne sera donc pas traitée efficacement par des antibiotiques de cette famille.

Dans un mode de réalisation préféré, la pathologie infectieuse est causée par une bactérie résistante à l'amoxicilline. De préférence, la bactérie responsable de la pathologie infectieuse est au moins partiellement résistante à une combinaison d'amoxicilline et d'acide clavulanique.

Dans un autre mode de réalisation préféré, la bactérie responsable de la pathologie infectieuse est une bactérie productrice de β-lactamases. Les β-lactamases sont des enzymes d'inactivation dites à sérine active (classes A, C et D) ou des métalloenzymes (classe B) dont les substrats sont les antibiotiques β-lactamines. On distingue des β-lactamases à spectre étroit et des β-lactamases à spectre étendu (BLSE) qui sont capables d'inhiber un grand nombre d'antibiotiques β-lactamines différents, en particulier les pénicillines, dont l'amoxicilline, les céphalosporines de 1ère, 2ème, 3ème (ex. céfotaxime, ceftazidime) et 4ème (ex. céfépime) génération et les monobactames (ex. aztréonam).

Dans un mode de réalisation particulièrement préféré, la bactérie responsable de la pathologie infectieuse est une bactérie productrice de β-lactamases à spectre étendu (BLSE). En particulier, une bactérie productrice de BLSE insensibles à l'acide clavulanique. Alternativement, la bactérie produit des BLSE qui ne sont que partiellement inhibées par l'acide clavulanique. Les bactéries BLSE peuvent être détectées par des tests bien connus de l'homme du métier comme le test du double disque, la méthode des disques combinés et l'E-test BLSE.

L'infection bactérienne traitée par la formulation pharmaceutique de l'invention peut être sélectionnée dans le groupe constitué des cystites, en particulier les cystites aiguës récidivantes, des sinusites bactériennes, en particulier les sinusites maxillaires aiguës, des otites, en particulier les otites moyennes aiguës, des bronchites, en particulier les bronchites chroniques et/ou aiguës, des bronchopneumopathies, en particulier les bronchopneumopathies chroniques et/ou aiguës, des pyélonéphrites, des infections gynécologiques hautes, des parodontites, des infections stomatologiques sévères, en particulier les abcès, les phlegmons et les cellulites, des morsures animales, des infections des os et des articulations, en particulier l'ostéomyélite, des endocardites, des péricardites, des septicémies, et des infections dermiques. De préférence, ladite infection bactérienne est une cystite, de manière encore préférée une cystite résistante aux antibiotiques de la famille des β-lactamines, de manière encore préférée une cystite résistante à l'amoxicilline, et de manière tout particulièrement préférée, une cystite résistante à une combinaison d'amoxicilline et d'acide clavulanique

Les termes de « sujet » et de « patient » sont équivalents et peuvent indifféremment être employés l'un pour l'autre dans le cadre de la présente invention. Tels qu'utilisé ici, le terme de « sujet » fait référence à un animal, de préférence à un mammifère, de manière encore préférée le terme de « sujet » fait référence à un humain.

Ainsi, dans un mode de réalisation particulier, le sujet auquel est administrée la formulation ou combinaison selon l'invention est un humain. Il peut ainsi s'agir d'un nouveau-né, d'un enfant, d'un adolescent, d'un adulte ou d'une personne âgée. Tel qu'utilisé ici, le terme de « nouveau-né » se réfère à un être humain âgé de moins de 12 mois, de préférence âgé de moins de 6 mois, de manière encore préférée âgé de moins de 3 mois. Tel qu'utilisé dans la présente invention, le terme « enfant » se réfère à un être humain âgé de 1 à 12 ans, de préférence à un être humain âgé de 1 à 8 ans, et de manière encore préférée à un être humain âgé de 1 à 5 ans. Tel qu'utilisé ici, le terme « d'adulte » se réfère à un être humain âgé de 12 à 60 ans, de préférence à un être humain âgé de 15 à 60 ans, de manière encore préférée à un être humain âgé de 18 à 60 ans. Tel qu'utilisé dans la présente invention, le terme de « personne âgée » se réfère à un être humain âgé de 60 ans ou plus, de préférence à un être humain âgé de 65 ans ou plus, et de manière encore préférée à un être humain âgé de 70 ans ou plus.

Dans un mode de réalisation préféré, le sujet auquel est administrée la formulation selon l'invention est un humain adulte.

Dans le domaine des applications vétérinaires, le sujet de l'invention peut être un animal non-humain, de préférence un animal de compagnie ou d'élevage, de manière encore préféré un animal sélectionné dans le groupe constitué des chiens, chats, bovins, ovins, lapins, porcs, caprins, équidés, rongeurs, en particulier hamsters et cochons d'inde, primates non-humains et volailles, de préférence poulets de chair, poules pondeuses, coqs et poules reproducteurs, pintades, dindons, cailles, canards, oies et pigeons.

Dans un mode de réalisation tout particulièrement préférée, la présente invention concerne une formulation selon l'invention dans le traitement d'une cystite résistante aux antibiotiques de la famille des β-lactamines chez un humain.

**Posologie**

La formulation pharmaceutique selon l'invention, utilisée dans le traitement d'une pathologie infectieuse, de préférence une infection bactérienne, chez un sujet, peut être administrée à raison d'une dose unique (une seule administration), ou à raison de plusieurs doses (plusieurs administrations) en fonction du sujet, de son âge, de son état de santé et de l'infection à traiter. Lorsque plusieurs doses (plusieurs administrations) de la formulation pharmaceutique selon l'invention sont administrées, celles-ci peuvent être réparties sur un ou plusieurs jours. La formulation pharmaceutique selon l'invention peut ainsi être administrée à raison d'une dose unique (une administration) par jour d'administration du traitement. De préférence, le sujet reçoit plusieurs doses (plusieurs administrations) de la formulation pharmaceutique selon l'invention par jour d'administration du traitement. De manière encore préférée, le sujet reçoit trois doses (trois administrations) de la formulation pharmaceutique selon l'invention par jour d'administration du traitement, de préférence matin, midi et soir.

Le nombre de doses (ou administrations) reçues par le sujet par jour d'administration peut également varier au cours du temps. Ainsi, peuvent alterner des périodes où le sujet reçoit une dose unique (une seule administration) par jour d'administration du traitement avec des périodes où le sujet reçoit plusieurs doses (plusieurs administrations) par jour d'administration du traitement, de préférence trois doses (trois administrations) réparties matin, midi et soir. En particulier, le sujet peut recevoir une dose (une administration) en général unique dite « dose de charge » le premier jour du traitement avant de recevoir plusieurs doses (plusieurs administrations) dites « doses d'entretien » par jour de traitement les jours suivants, de préférence trois doses (trois administrations) d'entretien par jour de traitement, de préférence matin, midi et soir.

La dose de charge, en général administrée au sujet le premier jour du traitement, de préférence en une seule prise, est une dose de la formulation selon l'invention qui est de préférence supérieure ou égale aux doses d'entretien, de manière encore préférée la dose de charge est supérieure ou égale à deux doses d'entretien lorsque le sujet reçoit au moins deux doses d'entretien par jour de traitement. De préférence, la dose de charge est comprise entre environ 5 g et environ 20 g, de manière encore préférée entre environ 10 g et environ 15 g, de manière tout particulièrement préférée la dose de charge est d'environ 12 g. De préférence, la dose d'entretien est comprise entre environ 1 g et environ 15 g, de manière encore préférée entre environ 3 g et environ 6 g, de manière tout particulièrement préférée la dose d'entretien est d'environ 3 g ou d'environ 6 g.

La formulation pharmaceutique selon l'invention peut être administrée tous les jours, tous les deux jours, ou une fois par semaine, de préférence tous les jours. La périodicité de prise de la formulation ou de la combinaison dépend de différents paramètres et peut être aisément définie par l'homme du métier selon, par exemple, la durée globale prévue de prise de la formulation pharmaceutique ou de la combinaison, l'âge du sujet et/ou la gravité de l'infection, de préférence bactérienne, à prévenir ou à traiter. La périodicité de prise (d'administration) de la formulation pharmaceutique ou de la combinaison peut également évoluer au cours du temps, chez un sujet donné, notamment en fonction de l'évolution de son infection, et/ou de son état de santé global.

En particulier, la formulation pharmaceutique selon l'invention peut être administrée au sujet pendant une période allant de 1 jour à environ 3 mois, 2 mois, 1 mois, 3 semaines, 2 semaines, 1 semaine, 5 jours, 3 jours, 2 jours, de préférence pendant une période d'environ 2 à environ 21 jours, de manière encore préférée pendant une période d'environ 7 à environ 14 jours. Dans un mode de réalisation particulièrement préféré, la formulation pharmaceutique l'invention est administrée au sujet pendant une période d'environ 7 jours. Alternativement, la formulation pharmaceutique selon l'invention peut être administrée pendant toute la durée de l'infection. Dans le cas d'une infection urinaire, la formulation selon l'invention peut être administrée jusqu'à l'obtention d'une urine stérile chez le sujet.

Dans un mode de réalisation particulier, la formulation pharmaceutique selon l'invention peut être administrée durant plusieurs mois, éventuellement durant plusieurs années, par exemple dans le cas d'infections bactériennes chroniques.

La formulation pharmaceutique selon l'invention peut être administrée au sujet à raison d'environ 1 à environ 150 grammes, de préférence d'environ 1 à environ 50 grammes, de manière encore préférée d'environ 2 à environ 30 grammes, de manière tout particulièrement préférée d'environ 3 à environ 20 grammes par jour de traitement. Par exemple, environ 9 grammes de la formulation pharmaceutique selon l'invention peuvent être administrés au sujet par jour de traitement. Dans un autre exemple, environ 18 grammes de la formulation suivant l'invention peuvent être administrés au sujet par jour de traitement.

## Composition pharmaceutique de cinéol et d'amoxicilline

La demande concerne également, dans un autre aspect non revendiqué, une composition pharmaceutique comprenant, ou consistant essentiellement en, du cinéol, de l'amoxicilline et un excipient ou support pharmaceutiquement acceptable, et optionnellement un inhibiteur de β-lactamases.

Dans un mode de réalisation préférée, l'inhibiteur de β-lactamases est sélectionné parmi l'acide clavulanique, le sulbactam, le tazobactam et l'aztréonam, de préférence, l'inhibiteur de β-lactamases est l'acide clavulanique.

Dans un aspect particulier, la composition divulguée comprend, ou consiste essentiellement en, du cinéol, de l'amoxicilline, optionnellement un inhibiteur de β-lactamases, de préférence de l'acide clavulanique, et un autre principe actif. De préférence, le principe actif supplémentaire de la composition divulguée est un autre antibiotique, en particulier un antibiotique β-lactamine, et/ou un autre inhibiteur des β-lactamases, et/ou un antifongique, et/ou un antiparasitaire, et/ou un analgésique.

De préférence, la composition divulguée comprend, ou consiste essentiellement en, des principes actifs, de préférence de l'amoxicilline et du cinéol, présents à des concentrations permettant l'administration de doses suffisantes pour obtenir un effet thérapeutique et/ou un effet antibactérien, de préférence un effet synergique, tel qu'illustré dans les exemples ci-après. Pour obtenir un tel effet, l'amoxicilline et le cinéol peuvent être administrés en des quantités thérapeutiquement efficaces ou en des quantités sub-thérapeutiques.

Dans un aspect, le cinéol et l'amoxicilline sont tous deux administrés en des quantités thérapeutiquement efficaces.

Dans un autre aspect, l'amoxicilline est administrée en une quantité thérapeutiquement efficace et le cinéol est administré en une quantité sub-thérapeutique.

Dans encore un autre aspect, l'amoxicilline est administrée en une quantité sub-thérapeutique et le cinéol est administré en une quantité thérapeutiquement efficace.

Dans encore un autre aspect, l'amoxicilline et le cinéol sont tous deux administrés en des quantités sub-thérapeutiques.

Lorsqu'ils sont présents dans la composition selon l'invention, l'inhibiteur de β-lactamases et/ou le principe actif supplémentaire peuvent être administrés en des quantités thérapeutiquement efficaces ou en des quantités sub-thérapeutiques.

La composition divulguée peut comprendre, ou consister essentiellement en, entre environ 5 mg et environ 100 mg, de préférence entre environ 10 mg et environ 50 mg, de manière encore préférée entre environ 20 mg et environ 40 mg, de manière tout particulièrement préférée environ 33 mg, de cinéol par gramme de composition ; et/ou entre environ 20 mg et environ 500 mg, de préférence entre environ 50 mg et environ 300 mg, de manière encore préférée entre environ 150 mg et environ 200 mg, de manière tout particulièrement préférée environ 167 mg, d'amoxicilline par gramme de composition ; et/ou optionnellement entre environ 1 mg et environ 100 mg, de préférence entre environ 5 mg et environ 50 mg, de manière encore préférée entre environ 15 mg et environ 25 mg, et de manière tout particulièrement préférée environ 21 mg, d'inhibiteur de β-lactamases, de préférence de l'acide clavulanique par gramme de composition.

De préférence, la composition divulguée comprend, ou consiste essentiellement, entre environ 5 mg et environ 100 mg de cinéol, entre environ 20 mg et environ 500 mg d'amoxicilline, et optionnellement entre environ 1 mg et environ 100 mg d'inhibiteur de β-lactamases, de préférence de l'acide clavulanique, par gramme de composition.

De manière encore préférée, la composition divulguée comprend, ou consiste essentiellement, entre environ 10 mg et environ 50 mg de cinéol, entre environ 50 mg et environ 300 mg d'amoxicilline, et optionnellement entre environ 5 mg et environ 50 mg d'inhibiteur de β-lactamases, de préférence de l'acide clavulanique, par gramme de composition.

De manière tout particulièrement préférée, la composition divulguée comprend, ou consiste essentiellement, entre environ 20 mg et environ 40 mg, de préférence environ 33 mg, de cinéol, et entre environ 150 mg et environ 200 mg, de préférence environ 167 mg, d'amoxicilline, et optionnellement entre environ 15 mg et environ 25 mg, et de manière tout particulièrement préférée environ 21 mg, d'inhibiteur de β-lactamases, de préférence de l'acide clavulanique, par gramme de composition.

La composition divulguée peut être sous forme de comprimés, capsules, gélules, granulats, poudre, suspensions, émulsions, solutions, polymères, nanoparticules, microsphères, suppositoires, capsules rectales, lavements, gels, pates, onguents, crèmes, emplâtres, potions, injectables, implants, sprays ou aérosols. De préférence, la composition pharmaceutique divulguée est sous la forme d'une poudre, plus particulièrement une poudre pour suspension buvable.

De préférence, les caractéristiques techniques de la poudre sont telle que définie précédemment pour la formulation pharmaceutique selon l'invention.

La composition divulguée comprend en outre au moins un excipient ou support pharmaceutiquement acceptable. L'homme du métier peut aisément définir les excipients nécessaires à une composition en fonction de la forme pharmaceutique choisie.

En particulier, lorsque la composition divulguée est sous forme de poudre, l'excipient ou support pharmaceutiquement acceptable de la composition divulguée est de préférence sélectionné dans le groupe consistant en un édulcorant, un aromatisant, un antiagglomérant, un lubrifiant, un désintégrant, un adsorbant, et un mélange de ceux-ci.

De préférence, lorsque la composition selon l'invention est sous forme de poudre, la composition suivant l'invention comprend au moins un édulcorant, de préférence de l'aspartame, un aromatisant, un antiagglomérant, de préférence de la silice, un lubrifiant, de préférence du stéarate de magnésium, un désintégrant, de préférence un mélange de croscarmellose et de cellulose microcristalline, et un adsorbant, de préférence de l'huile. Une telle composition peut en outre comprendre un conservateur, un colorant et/ou un tampon pH.

Ainsi, dans un mode de réalisation préférée, la composition selon l'invention est sous forme d'une poudre comprenant, ou consistant essentiellement en, ou consistant en, du cinéol, de l'amoxicilline, et au moins un édulcorant, un aromatisant, un antiaggloméant, un lubrifiant, un désintégrant et/ou un adsorbant tels que définis ci-dessus et un inhibiteur de β-lactamases, de préférence de l'acide clavulanique, tel que défini ci-dessus.

De préférence, la composition est une poudre comprenant, ou consistant essentiellement en, ou consistant en, du cinéol et de l'amoxicilline dans les proportions décrites ci-dessus, et entre environ 1 mg et environ 60 mg d'édulcorant, de préférence de l'aspartame, et/ou entre environ 1 mg et environ 60 mg d'aromatisant, et/ou entre environ 20 mg et environ 500 mg d'antiaggloméant, de préférence de la silice, et/ou entre environ 1 mg et environ 40 mg de lubrifiant, de préférence du stéarate de magnésium, et/ou entre environ 10 mg et environ 1000 mg de désintégrant, de préférence un mélange de cellulose microcristalline et de croscarmellose, et/ou entre environ 2 mg et environ 50 mg d'adsorbant, de préférence de l'huile, par gramme de poudre, et optionnellement un inhibiteur de β-lactamases, de préférence de l'acide clavulanique, dans les proportions décrites ci-dessus.

La composition selon l'invention peut notamment comprendre au moins un excipient de type adsorbant. Tel qu'utilisé ici, le terme « adsorbant » se réfère à un excipient capable de fixer des molécules liquides, par exemple des molécules de cinéol, sur un support solide, par exemple poudreux, dans une composition pharmaceutique. De préférence, l'adsorbant est une huile pharmaceutiquement acceptable. L'huile pharmaceutiquement acceptable est telle que décrite précédemment.

Dans un autre mode de réalisation particulièrement préféré, la composition selon l'invention ne comprend pas de détergent. Le détergent est tel que décrit précédemment dans le chapitre sur les excipients.

La demande concerne également une composition pharmaceutique divulguée pour une utilisation dans le traitement d'une pathologie infectieuse, de préférence une infection bactérienne, chez un sujet.

Elle concerne également une méthode de traitement comprenant l'administration d'une quantité thérapeutiquement efficace de la composition pharmaceutique divulguée à un sujet en ayant besoin, en particulier un sujet souffrant d'une pathologie infectieuse, de préférence une infection bactérienne.

Les modes de réalisations et définitions décrits pour l'utilisation d'une combinaison divulguée ou d'une formulation pharmaceutique suivant l'invention dans le traitement d'une pathologie infectieuse, de préférence une infection bactérienne, chez un sujet ainsi que concernant la posologie de cette combinaison ou formulation, sont également à prendre en considération dans cet aspect.

De préférence, la pathologie infectieuse est causée par une ou plusieurs bactéries résistantes aux antibiotiques, de préférence aux antibiotiques de la famille des β-lactamines, de manière encore préférée à l'amoxicilline et de manière tout particulièrement préférée à une ou plusieurs bactéries au moins partiellement résistantes à une combinaison d'amoxicilline et d'acide clavulanique.

Dans un mode de réalisation tout particulièrement préféré, la présente demande concerne une composition pharmaceutique telle que divulguée dans la présente demande pour une utilisation dans le traitement d'une cystite, en particulier une cystite résistante aux antibiotiques, de préférence aux antibiotiques de la famille des β-lactamines, de manière encore préférée à l'amoxicilline et de manière tout particulièrement préférée à une combinaison d'amoxicilline et d'acide clavulanique.

**Trousse**

La demande concerne encore, dans un autre aspect non revendiqué, une trousse pour le traitement d'une pathologie infectieuse, de préférence une infection bactérienne, chez un sujet comprenant :

(a) une composition pharmaceutique comprenant, ou consistant essentiellement en, du cinéol et une composition pharmaceutique comprenant, ou consistant essentiellement en, de l'amoxicilline ;
(b) une composition pharmaceutique comprenant, ou consistant essentiellement en, du cinéol et une composition pharmaceutique comprenant, ou consistant essentiellement en, de l'amoxicilline et un inhibiteur de β-lactamases, de préférence de l'acide clavulanique ;
(c) une composition pharmaceutique comprenant, ou consistant essentiellement en, de l'amoxicilline et une composition pharmaceutique comprenant, ou consistant essentiellement en, du cinéol et un inhibiteur de β-lactamases, de préférence de l'acide clavulanique ;
(d) une composition pharmaceutique comprenant, ou consistant essentiellement en, un inhibiteur de β-lactamases, de préférence de l'acide clavulanique, et une composition pharmaceutique comprenant, ou consistant essentiellement en, du cinéol et de l'amoxicilline ; ou
(e) une composition pharmaceutique comprenant, ou consistant essentiellement en, du cinéol, une composition pharmaceutique comprenant, ou consistant essentiellement en, de l'amoxicilline, et une composition pharmaceutique comprenant, ou consistant essentiellement en, un inhibiteur de β-lactamases, de préférence de l'acide clavulanique ; et
(f) éventuellement, un guide contenant des instructions pour l'utilisation d'une telle trousse.

De préférence, chaque composition comprise dans la trousse est dans un récipient, un contenant, et/ou un emballage distinct.

Dans un aspect particulier, au moins une composition pharmaceutique de la trousse divulguée comprend en outre au moins un autre principe actif. De préférence, le principe actif supplémentaire divulguée est un autre antibiotique, en particulier un antibiotique β-lactamine, et/ou un autre inhibiteur de β-lactamases, et/ou un antifongique, et/ou un antiparasitaire, et/ou un analgésique.

De préférence, les principes actifs des compositions de la trousse selon l'invention, en particulier l'amoxicilline et le cinéol, sont présents à des concentrations permettant l'administration de doses suffisantes pour obtenir un effet thérapeutique et/ou un effet antibactérien, de préférence un effet synergique lorsque lesdits principes actifs sont présents dans une même composition ou lorsque, présents dans des compositions différentes, ils sont utilisées simultanément, séquentiellement ou séparément. Pour obtenir un tel effet, le cinéol et l'amoxicilline peuvent être administrés en des quantités thérapeutiquement efficaces ou en des quantités sub-thérapeutiques.

Dans un aspect préféré, le cinéol et l'amoxicilline sont tous deux administrés en des quantités thérapeutiquement efficaces.

Dans un autre aspect préféré, l'amoxicilline est administrée en une quantité thérapeutiquement efficace et le cinéol est administré en une quantité sub-thérapeutique.

Dans encore un autre aspect préféré, l'amoxicilline est administrée en une quantité sub-thérapeutique et le cinéol est administré en une quantité thérapeutiquement efficace.

Dans encore un autre aspect préféré, l'amoxicilline et le cinéol sont tous deux administrés en des quantités sub-thérapeutiques.

Lorsqu'ils sont présents dans les compositions de la trousse selon l'invention, l'inhibiteur de β-lactamases et/ou le principe actif supplémentaire peuvent être administrés en des quantités thérapeutiquement efficaces ou en des quantités sub-thérapeutiques.

La composition pharmaceutique de la trousse divulguée comprenant du cinéol peut comprendre entre environ 5 mg et environ 100 mg, de préférence entre environ 10 mg et environ 50 mg, de manière encore préférée entre environ 20 mg et environ 40 mg, de manière tout particulièrement préférée environ 33 mg, de cinéol par gramme de composition.

La composition pharmaceutique de la trousse divulguée comprenant de l'amoxicilline peut comprendre entre environ 20 mg et environ 500 mg, de préférence entre environ 50 mg et environ 300 mg, de manière encore préférée entre environ 150 mg et environ 200 mg, de manière tout particulièrement préférée environ 167 mg, d'amoxicilline par gramme de composition.

Lorsqu'une composition pharmaceutique de la trousse divulguée comprend un inhibiteur de β-lactamases, de préférence de l'acide clavulanique, ladite composition peut comprendre entre environ 1 mg et environ 100 mg, de préférence entre environ 5 mg et environ 50 mg, de manière encore préférée environ 15 mg et environ 25 mg, et de manière tout particulièrement préférée environ 21 mg, d'inhibiteur de β-lactamases par gramme de composition.

Les compositions de la trousse divulguée peuvent être sous forme de comprimés, capsules, gélules, granulats, poudre, suspensions, émulsions, solutions, polymères, nanoparticules, microsphères, suppositoires, capsules rectales, lavements, gels, pates, onguents, crèmes, emplâtres, potions, injectables, implants, sprays ou aérosols.

De préférence, les compositions pharmaceutiques divulguée sont sous la forme de poudres, de suppositoires ou de capsules rectales, de préférence sous forme de poudres, de manière encore préférée sous forme de poudres sèches, plus particulièrement de poudres pour suspensions buvables.

Lorsque le cinéol est le seul principe actif d'une composition, il peut être sous forme d'huile, en particulier d'huile encapsulée.

La ou les compositions pharmaceutiques de la trousse divulguée comprennent de préférence au moins un excipient ou support pharmaceutiquement acceptable. L'homme du métier peut aisément définir les excipients nécessaires à une composition en fonction de la forme pharmaceutique choisie. Les excipients ou support pharmaceutiquement acceptables sont tels que définis ci-dessus pour la composition pharmaceutique.

La demande concerne, dans encore un autre aspect non revendiqué, une trousse divulguée pour une utilisation dans le traitement d'une pathologie infectieuse, de préférence une infection bactérienne, chez un sujet.

Les modes de réalisations et définitions décrits pour l'utilisation d'une formulation ou d'une combinaison divulguée dans le traitement d'une pathologie infectieuse chez un sujet ainsi que concernant l'administration et la posologie de cette formulation ou combinaison, sont également à prendre en considération dans cet aspect.

De préférence, la pathologie infectieuse est causée par une ou plusieurs bactéries résistantes aux antibiotiques, de préférence aux antibiotiques de la famille des β-lactamines, de manière encore préférée à l'amoxicilline et de manière tout particulièrement préférée par une ou plusieurs bactéries au moins partiellement résistantes à une combinaison d'amoxicilline et d'acide clavulanique.

Dans un aspect, la présente demande concerne une composition pharmaceutique telle que divulguée dans la présente demande pour une utilisation dans le traitement d'une cystite, en particulier une cystite résistante aux antibiotiques, de préférence aux antibiotiques de la famille des β-lactamines, de manière encore préférée à l'amoxicilline et de manière tout particulièrement préférée à une combinaison d'amoxicilline et d'acide clavulanique.

**Procédé de fabrication de la formulation pharmaceutique**

La présente invention concerne également, dans un troisième aspect, un procédé de fabrication de la composition ou formulation pharmaceutique selon l'invention comprenant :

- l'obtention d'une solution de mouillage par mélange de cinéol et d'une huile pharmaceutiquement acceptable ;
- le mouillage d'une poudre comprenant de l'amoxicilline par la solution de mouillage afin d'obtenir une préparation poudreuse comprenant de l'amoxicilline, du cinéol et de l'huile.

La présente invention concerne également la composition ou formulation pharmaceutique obtenue par le procédé selon l'invention.

Tel qu'utilisé ici, le terme « solution de mouillage » se réfère à solution destinée à mouiller ou humidifier une poudre sèche. L'opération de mouillage peut en particulier être réalisée par pulvérisation de la solution de mouillage sur la poudre à mouiller.

L'étape d'obtention d'une solution de mouillage du procédé selon l'invention peut être réalisée en mélangeant du cinéol et une huile pharmaceutiquement acceptable tels que décrits précédemment dans des proportions massiques huile sur cinéol comprises entre environ 0,1 et environ 1, de préférence entre environ 0,2 et environ 0,8, de manière encore préférée entre environ 0,4 et environ 0,6. De manière tout particulièrement préférée la proportion massique huile sur cinéol est d'environ 0,5. Par exemple, environ 50 mg d'huile est mélangée avec environ 100 mg de cinéol, ou encore environ 16,7 mg d'huile est mélangée avec environ 33,3 mg de cinéol.

De préférence, le mélange de cinéol et d'huile pour obtenir une solution de mouillage est réalisé dans une enceinte fermée, de préférence à une température ne dépassant pas environ 20°C pour éviter l'évaporation du cinéol, mais suffisante maintenir le mélange à l'état liquide.

Dans un mode de réalisation particulier, la poudre comprenant de l'amoxicilline et destinée à être mouillée par la solution de mouillage, comprend également un inhibiteur de β-lactamase, de préférence sélectionné dans le groupe constitué de l'acide clavulanique, du sulbactam, du tazobactam, de l'aztréonam et des sels pharmaceutiquement acceptables de ceux-ci, de manière encore préférée l'inhibiteur de β-lactamase est l'acide clavulanique.

De préférence, la poudre comprenant de l'amoxicilline et destinée à être mouillée par la solution de mouillage, comprend également au moins un excipient ou support pharmaceutiquement acceptable, de préférence sélectionné dans le groupe consistant en un édulcorant, un aromatisant, un antiagglomérant, un lubrifiant, un désintégrant, et un mélange de ceux-ci. De manière encore préférée, la poudre comprenant de l'amoxicilline comprend au moins un édulcorant, un aromatisant, un antiagglomérant, un lubrifiant et un désintégrant tels que définis ci-dessus.

Dans un autre mode de réalisation particulier, la poudre comprenant de l'amoxicilline et destinée à être mouillée par la solution de mouillage, comprend également de l'acide clavulanique, de la silice, de la silice colloïdale, de l'aspartame, de la croscarmellose, de la cellulose microcristalline, du stéarate de magnésium et un aromatisant tels que définis ci-dessus.

De préférence, le procédé selon l'invention comprend encore une étape de compactage de la poudre comprenant de l'amoxicilline avant qu'elle ne soit mouillée par la solution de mouillage. Les granulés obtenus par compactage sont de préférence calibrés puis fractionnés avant mouillage par la solution de mouillage.

Dans un autre mode de réalisation, le procédé de fabrication de la composition ou formulation pharmaceutique selon l'invention comprend :

- l'obtention d'une solution de mouillage par mélange de cinéol et d'une huile pharmaceutiquement acceptable ;
- le mouillage d'une poudre comprenant de l'amoxicilline par la solution de mouillage afin d'obtenir une préparation poudreuse comprenant l'amoxicilline, le cinéol et l'huile ;
- le mélange de la préparation obtenue à l'étape précédente avec une poudre comprenant un inhibiteur de β-lactamase, de préférence sélectionné dans le groupe constitué de l'acide clavulanique, du sulbactam, du tazobactam, de l'aztréonam et des sels pharmaceutiquement acceptables de ceux-ci, de manière encore préférée l'inhibiteur de β-lactamase est l'acide clavulanique ;

  - le tamisage de la poudre ainsi obtenue.

Dans ce procédé, la poudre comprenant de l'amoxicilline et/ou la poudre comprenant l'inhibiteur de β-lactamase, de préférence de l'acide clavulanique, peuvent en outre comprendre un désintégrant tel que décrit ci-dessus. De préférence deux désintégrants, en particulier de la cellulose microcristalline et de la croscarmellose. Dans un mode de réalisation préféré, la poudre comprenant de l'amoxicilline et la poudre comprenant un inhibiteur de β-lactamase, de préférence de l'acide clavulanique, comprennent deux désintégrants, de la cellulose microcristalline et de la croscarmellose.

La poudre comprenant de l'amoxicilline et/ou la poudre comprenant un inhibiteur de β-lactamase, de préférence de l'acide clavulanique, peuvent en outre comprendre un antiagglomérant tel que décrit ci-dessus. Dans un mode de réalisation préféré, la poudre comprenant de l'amoxicilline et la poudre comprenant un inhibiteur de β-lactamase, de préférence de l'acide clavulanique, comprennent toutes deux un antiagglomérant, de préférence de la silice.

Dans un mode de réalisation tout particulièrement préféré, la poudre comprenant de l'amoxicilline et la poudre comprenant un inhibiteur de β-lactamase, de préférence de l'acide clavulanique, comprennent toutes deux de la cellulose microcristalline, de la croscarmellose et de la silice.

Ainsi, le procédé selon l'invention peut comprendre une étape de tamisage puis de mélange du ou des désintégrants et de l'antiagglomérant. Alternativement, le ou les désintégrants et l'antiagglomérant peuvent être mélangés avant que le mélange ne soit tamisé. En particulier, la cellulose microcristalline, la croscarmellose et la silice peuvent être mélangés dans des proportions d'environ 1000 mg à environ 1500 mg, de préférence environ 1241 mg, de cellulose microcristalline pour d'environ 200 mg à environ 400 mg, de préférence environ 300 mg, de croscarmellose et d'environ 400 mg à environ 600 mg, de préférence environ 540 mg, de silice.

Le procédé selon l'invention peut également comprendre une étape de mélange de ces excipients avec la poudre comprenant de l'amoxicilline et/ou avec la poudre comprenant de l'acide clavulanique, de préférence une partie de ces excipients est mélangé avec la poudre comprenant de l'amoxicilline et l'autre partie de ces excipients avec la poudre comprenant de l'acide clavulanique.

Dans un autre mode de réalisation préféré, la poudre comprenant un inhibiteur de β-lactamase, de préférence de l'acide clavulanique, comprend encore de la silice colloïdale, de préférence dans une proportion massique par rapport à l'acide clavulanique comprise entre environ 0,7 et environ 1,3, de préférence entre environ 0,9 et environ 1,1, de manière encore préférée d'environ 1. De préférence, le mélange d'acide clavulanique et de silice colloïdale est réalisée avant l'ajout de tout autre excipient ou mélange avec la poudre comprenant de l'amoxicilline.

De préférence, le procédé selon l'invention comprend encore une étape de compactage de la poudre comprenant de l'amoxicilline avant qu'elle ne soit mouillée par la solution de mouillage. Les granulés obtenus par compactage sont de préférence calibrés puis fractionnés avant mouillage par la solution de mouillage.

De même, le procédé selon l'invention comprend de préférence une étape de compactage de la poudre comprenant un inhibiteur de β-lactamase, de préférence de l'acide clavulanique, avant qu'elle ne soit mélangée au mélange de la poudre d'amoxicilline mouillée par la solution de mouillage. Les granulés obtenus par compactage sont de préférence calibrés puis fractionnés avant mouillage par la solution de mouillage.

Au cours de l'étape de mouillage de la poudre comprenant de l'amoxicilline par la solution de mouillage, la solution de mouillage peut être est utilisée à raison d'environ 100 à environ 200 mg, de préférence environ 150 mg, de solution de mouillage pour une poudre comprenant d'environ 400 à environ 600 mg, de préférence environ 500 mg, d'amoxicilline

Au cours de l'étape de mélange de la préparation d'amoxicilline, cinéol et huile avec la poudre comprenant un inhibiteur de β-lactamase, de préférence de l'acide clavulanique, la préparation d'amoxicilline, cinéol et huile est mélangée avec une poudre comprenant d'environ 550 mg à environ 750 mg, de préférence environ 650 mg, d'inhibiteur de β-lactamase.

Optionnellement, le procédé de l'invention peut comprendre, après l'étape de mélange de la préparation d'amoxicilline cinéol et huile avec la poudre comprenant l'acide clavulanique, une étape supplémentaire d'ajout d'un édulcorant, d'un aromatisant et/ou d'un lubrifiant tels que décrits ci-dessus. De préférence, un édulcorant, un aromatisant et un lubrifiant sont ajoutés. De préférence, l'édulcorant est l'aspartame et le lubrifiant est le stéarate de magnésium.

Au cours de cette étape, ces excipients supplémentaires peuvent être ajoutés dans des proportions d'environ 30 mg à environ 50 mg, de préférence environ 36 mg, d'édulcorant, de préférence de l'aspartame, d'environ 15 mg à environ 25 mg, de préférence environ 18 mg, de lubrifiant, de préférence du magnésium stéarate, et/ou d'environ 70 mg à environ 110 mg, de préférence 90 mg, d'aromatisant pour 3 grammes de formulation pharmaceutique.

Après l'étape d'ajout d'un édulcorant, d'un aromatisant et/ou d'un lubrifiant, la préparation obtenue est mélangée jusqu'à obtention d'une poudre homogène.

Dans un mode de réalisation préférée, l'étape de tamisage finale est réalisée avec une grille de tamis ayant des ouvertures d'un diamètre d'au plus 5 mm, de préférence d'au plus 2,5 mm, de manière encore préféré d'au plus 1,25 mm.

Optionnellement, le procédé de l'invention peut comprendre une étape supplémentaire de conditionnement de la poudre tamisée obtenue par le procédé de l'invention dans un récipient unidose ou multi-dose, de préférence dans un récipient unidose.

Dans un mode de réalisation préféré, la poudre tamisée obtenue par le procédé de l'invention est conditionnée dans un récipient unidose contenant entre environ 1 g et environ 150 g de poudre, de manière encore préférée entre environ 1 g et environ 50 g de poudre, de manière toujours préférée entre environ 1 g et environ 10 g de poudre, et de manière plus particulièrement préférée environ 3 g de poudre.

Dans un autre mode de réalisation, la poudre tamisée obtenue par le procédé de l'invention est conditionnée dans un récipient multi-dose comprenant par exemple entre environ 10 grammes et environ 500 grammes de poudre, de préférence entre environ 20 grammes et environ 200 grammes de poudre, de manière encore préférée entre environ 30 grammes et environ 100 grammes de poudre, et de manière tout particulièrement préférée environ 50 grammes de poudre.

Optionnellement, le procédé de l'invention peut comprendre une étape supplémentaire de conditionnement secondaire du conditionnement primaire de la poudre tamisée obtenue par le procédé de l'invention.

Dans un mode de réalisation préféré, lesdits récipients unidoses contenant la poudre tamisée sont secondairement conditionnés dans une boîte. En particulier, Une boîte peut contenir, par exemple, entre 3 et 31 récipients unidoses, de préférence entre 5 et 21 récipients unidoses, de manière encore préféré entre 7 et 14 récipients unidoses.

Dans un autre mode de réalisation, ledit récipient multi-dose contenant la poudre tamisée est secondairement conditionné dans une boîte, éventuellement accompagné d'un doseur, par exemple une cuillère, permettant de prélever une quantité déterminée de poudre, de préférence d'environ 1 mg à environ 30 mg de poudre, de manière encore préférée d'environ 2 mg à environ 20 mg de poudre, et de manière tout particulièrement préférée d'environ 3 à environ 12 mg de poudre. En particulier, le doseur peut permettre de prélever environ 3 g, environ 6 g, environ 9 g, environ 12 g, environ 15 g et/ou environ 18 g de poudre.

**Complexe moléculaire**

L'amoxicilline est un antibiotique sensible aux β-lactamases. Les β-lactamases, produites par les bactéries résistantes à l'amoxicilline, reconnaissent et inactivent le noyau β-lactame de l'amoxicilline. Lorsqu'elle est mise en solution, de préférence dans un solvant aqueux, l'amoxicilline peut former transitoirement des complexes de deux molécules d'amoxicilline. La formation de ces complexes est trop transitoire pour avoir un quelconque effet protecteur contre les β-lactamases. Par contre, lorsque la formulation pharmaceutique de l'invention est mise en solution, des complexes d'amoxicillines stables comprenant au moins trois molécules d'amoxicilline se forment et protègent l'antibiotique de l'action des β-lactamases. La formation de ces complexes peut également être obtenue par la mise en solution de l'amoxicilline en présence de cinéol.

Ainsi, dans un dernier aspect non revendiqué, la demande concerne encore un complexe moléculaire comprenant plus de deux molécules d'amoxicilline organisées de façon linéaire ou en anneau et interagissant les unes avec les autres par des liaisons non-covalentes.

De préférence, le complexe moléculaire divulgué ici est formé uniquement de molécules d'amoxicilline.

Le complexe moléculaire divulgué ici est formé d'au moins trois molécules d'amoxicilline, de préférence de trois à six molécules d'amoxicilline, de manière encore préférée de trois ou quatre molécules d'amoxicilline et de manière tout particulièrement préférée de quatre molécules d'amoxicilline.

Les molécules d'amoxicilline du complexe moléculaire divulgué ici peuvent être organisées de façon linéaire ou en anneau. De préférence, elles sont organisées en anneau de sorte que chaque molécule d'amoxicilline interagit avec deux autres molécules d'amoxicilline.

Dans un aspect particulier, les molécules d'amoxicilline peuvent librement passer d'une organisation en complexe linéaire à une organisation en complexe en anneau par rupture ou formation de liaisons non-covalentes.

Le complexe moléculaire divulgué ici peut être obtenu par la mise en solution d'amoxicilline en présence de cinéol dans un solvant aqueux. De préférence, le complexe moléculaire divulgué ici est obtenu par la mise en solution d'amoxicilline en présence de cinéol dans un solvant aqueux en l'absence de détergent.

Dans un aspect particulier, le complexe moléculaire divulgué ici est obtenu par la mise en solution de la formulation pharmaceutique de l'invention dans un solvant aqueux.

De préférence, les molécules d'amoxicilline du complexe moléculaire divulgué ici ne sont pas reconnues par les β-lactamases. Ainsi, le complexe moléculaire divulgué ici peut être utilisé dans le traitement des bactéries considérées comme résistantes à l'amoxicilline.

Dans un aspect particulier, le complexe moléculaire divulgué ici peut être obtenu en milieu aqueux lorsque le ratio massique de l'amoxicilline sur le cinéol est compris entre environ 0,01 et environ 1000, de préférence entre environ 0,1 et environ 100, de manière encore préférée entre environ 1 et environ 10, et de manière tout particulièrement préférée lorsque le ratio massique de l'amoxicilline sur le cinéol est d'environ 5.

La présente demande concerne également l'utilisation du complexe moléculaire divulgué ici en tant que médicament. Elle concerne également le complexe moléculaire divulgué ici pour une utilisation dans le traitement d'une pathologie infectieuse chez un sujet. Elle concerne en outre le complexe moléculaire divulgué ici pour la préparation d'un médicament destiné au traitement d'une pathologie infectieuse. Elle concerne également une méthode de traitement comprenant l'administration d'une quantité thérapeutiquement efficace du complexe moléculaire divulgué ici à un sujet en ayant besoin, en particulier un sujet souffrant d'une pathologie infectieuse.

Les modes de réalisation décrits pour la formulation ou la combinaison divulgué ici sont également à prendre en compte dans cet aspect.

Bien qu'ayant des sens différents, les termes « comprenant », « ayant », « contenant » et « consistant en » peuvent être remplacés l'un par l'autre dans toute la description de l'invention.

D'autres caractéristiques et avantages de l'invention apparaîtront mieux à la lecture des exemples suivants donnés à titre illustratif et non limitatif.

## Exemples

### Exemple 1 - Etude *in vitro* de l'activité antibactérienne de la combinaison d'amoxicilline et de cinéol

**Matériel et méthodes**

Matériel biologique, milieu de culture et agents antimicrobiens

Les six souches testées lors de cette étude sont des isolats cliniques purifiés et identifiés au laboratoire de bactériologie du centre hospitalier universitaire Hassan II (CHU, Fès, Maroc). Trois des six souches bactériennes testées sont des souches d'*Escherichia coli* BLSE (P956, P933 et P7847) et les trois autres souches bactériennes testées sont des souches de *Klebsiella pneumoniæ* BLSE (H1878, H2001 et H1893).

Pour chaque test effectué, des précultures des souches bactériennes (auparavant congelées à -20°C) de 24h à 37°C ont été préparées. A partir de ces précultures, des inoculums bactériens à $2 \times 10^7$ UFC (Unité formant une colonie)/ml ont été préparés en ajustant la densité optique à 540 nm.

Des milieux de culture Mueller Hinton (MH) gélosés et liquides ont été fournis par BIOKAR (France). Le premier a été utilisé pour la croissance des souches et le deuxième, additionné de 20% (v/v) de glycérol, a été utilisé pour la conservation des souches. La préparation des deux milieux a été effectuée, selon les instructions du fournisseur.

L'amoxicilline (AMX) et le cinéol ont été fournis par Sigma Aldrich (France). Une solution mère d'AMX (400 μg/ml) a été réalisée après mise en solution de 40 mg d'antibiotique dans 100 ml d'eau distillée stérile. À partir de cette solution mère, des séries de dilutions ont été effectuées. Les concentrations de cinéol utilisées ont été préparées par émulsion de cinéol pur dans 0,2 % (v/v) d'agar agar selon la méthode décrite par Remmal A et al (J. Essent. Oil. Res [Book], 1993, 5 : pp. 1179-1184).

Evaluation des pourcentages d'inhibition de l'association de l'AMX avec le cinéol

Les concentrations inhibitrices partielles (CIP) sont les concentrations d'agents antibactériens inhibant la croissance d'un pourcentage donné (90%, 75%, 50%, 40%...) de la population bactérienne étudiée. Les CIP de l'AMX et du cinéol vis-à-vis des six souches bactériennes utilisés ont été déterminées par une technique de microdilution en microplaque se basant sur la surveillance de la croissance bactérienne par mesure de la densité optique (Casey JT et al, J Microbiol Meth [Book], 2004, 58 : pp. 327- 334 ; Patton T et al, J Microbiol Meth [Book], 2006, 64 : pp. 84- 95).

Des microplaques stériles à fond en U de 96 cupules et d'une capacité de 200 μl ont été utilisées. Pour chaque microplaque, deux rangées témoins ont été réalisées:

➢ Une rangée contenant 200 μl du milieu liquide MH (Témoin de stérilité et témoin négatif).
➢ Une rangée contenant 150 μl du milieu liquide MH et 50 μl de l'inoculum bactérien à 2x10^7 UFC/ml (Témoin positif).

Pour l'AMX et le cinéol, deux rangées ont été réalisées contenant 100 μl de milieu liquide MH, 50 μl de l'inoculum bactérien à 2x10^7 UFC/ml et 50 μl de concentrations décroissantes d'agents antimicrobiens. Les concentrations d'AMX utilisées sont de : 50-25-12,5-6,25-3,1-1,6-0,8-0,4-0,20-0,10-0,05-0,025 μg/ml. Les concentrations de cinéol utilisées sont de : 100-50-25-12,5-6,25-3,1-1,6-0,8-0,4-0,20-0,10-0,05 μl/ml. Les densités optiques (DO) ont été déterminées à 540 nm à l'aide d'un spectrophotomètre pour microplaques (Versamax, Molecular devices, USA). La DO a été mesurée au temps t=0 et après 22 heures d'incubation à 30°C. Le pourcentage d'inhibition des agents antimicrobiens vis-à-vis de chacune des six souches a été calculé d'après Casey JT et al (J Microbiol Meth [Book], 2004, 58 : pp. 327- 334) selon la formule suivante:

$$\% \text{ d'inhibition} = 1 - \frac{DO_{T22} - DO_{T0}}{DO_{C22} - DO_{C0}} \quad X \quad 100$$

Avec $DO_{T0}$=DO de la cupule test au temps t=0, $DO_{T22}$= DO de la cupule test après 22h d'incubation, $DO_{C0}$= DO de la cupule du témoin positif au temps t=0, $DO_{C22}$= DO de la cupule du témoin positif après 22h d'incubation.

Après avoir déterminé les CIP de l'AMX et du cinéol seuls, le pouvoir antibactérien de l'association d'AMX et de cinéol a été évalué. La même technique de microdilution en microplaque a été utilisée. Les associations AMX-cinéol ont été préparées dans des tubes stériles comme suit :

➢ AMX CIP 50% + cinéol CIP 40%
➢ AMX CIP 50% + cinéol CIP 30%
➢ AMX CIP 50% + cinéol CIP 20%
➢ AMX CIP 25% + cinéol CIP 40%
➢ AMX CIP 25% + cinéol CIP 30%
➢ AMX CIP 25% + cinéol CIP 20%

L'indice de fraction des concentrations inhibitrices (FIC-index) exprimant le degré de synergie de l'association AMX-cinéol a été calculé selon la formule suivante (Odds FC et al, J Antimicrob Chemoth [Book], 2003, 52 : p. 1).

$$\text{FIC-index} = \frac{CIP_{AMX+Cinéol}}{CIP_{AMX \text{ seule}}} + \frac{CIP_{AMX+Cinéol}}{CIP_{Cinéol \text{ seul}}}$$

L'association AMX-cinéol est considérée :

➢ Synergique quand le FIC-index est ≤ 0.5
➢ Additive quand 0.5 < FIC-index < 1
➢ Indifférente quand 1< FIC-index< 2
➢ Antagoniste quand FIC-index > 2.

**Résultats**

Détermination des CIP de l'amoxicilline et du cinéol

L'activité antibactérienne de l'AMX vis-à-vis des trois souches d'*E. coli* et des trois souches de *K. pneumoniae* est comparable (cf. tableau 1). Les concentrations minimales inhibitrices (CMI, concentration minimale permettant d'obtenir une inhibition de 100 %) sont de l'ordre de 50 μg/ml (p>0,05). Le cinéol a également une activité antibactérienne comparable vis-à-vis des trois souches d'*E. coli* et des trois souches de *K. pneumoniae* (cf. tableau 2). Les CMI sont de l'ordre de 100 $\mu$l/ml (p>0,05).

*Tableau 1: Concentrations inhibitrices partielles de l'Amoxicilline*

| Souches | CIP 50% ($\mu$g/ml) | CIP 25% ($\mu$g/ml) |
|---|---|---|
| *Escherichia coli* | | |
| P956 | 1,1$\pm$0,25 | 0,7$\pm$0,14 |
| P933 | 1,3$\pm$0,24 | 0,5$\pm$0,10 |
| P7847 | 1,5$\pm$0,13 | 0,8$\pm$0,17 |
| *Klebsiella pneumoniae* | | |
| H1878 | 1,6$\pm$0,17 | 0,9$\pm$0,09 |
| H2001 | 1,3$\pm$0,12 | 0,6$\pm$0,14 |
| H1893 | 1,7$\pm$0,09 | 0,8$\pm$0,12 |

*Tableau 2: Concentrations inhibitrices partielles du cinéol*

| Souches | CIP 40% ($\mu$l/ml) | CIP 30% ($\mu$l/ml) | CIP 20% ($\mu$l/ml) |
|---|---|---|---|
| *Escherichia coli* | | | |
| P956 | 6,2$\pm$0,20 | 2,6$\pm$0,12 | 1,2$\pm$0,16 |
| P933 | 6,8$\pm$0,14 | 2,3$\pm$0,09 | 1,3$\pm$0,24 |
| P7847 | 6,5$\pm$0,10 | 2,7$\pm$0,12 | 1,2$\pm$0,11 |
| *Klebsiella pneumoniae* | | | |
| H1878 | 7,1$\pm$0,12 | 2,9$\pm$0,12 | 0,4$\pm$0,12 |
| H2001 | 6,9$\pm$0,12 | 3,1$\pm$0,10 | 0,7$\pm$0,09 |
| H1893 | 6,8$\pm$0,09 | 2,7$\pm$0,09 | 0,6$\pm$0,12 |

Evaluation des pourcentages d'inhibition de l'association d'amoxicilline et de cinéol

L'association d'AMX et de cinéol donne des pourcentages d'inhibition supérieurs à l'addition des deux (cf. tableaux 3 et 4). En effet, la CIP 50% de l'AMX (1,1-1,7 $\mu$g/ml) associée à la CIP 40% du cinéol (6,2-7,1 $\mu$l/ml) donnent 100% d'inhibition de croissance pour toutes les souches testées.

*Tableau 3: Pourcentages d'inhibition des associations de l'AMX (CIP 50%) avec le cinéol (CIP 40%, CIP 30% et CIP 20%)*

| Souches | AMX + Cinéol CIP 50 % + CIP 40 % | AMX + Cinéol CIP 50 % + CIP 30 % | AMX + Cinéol CIP 50 % + CIP 20 % |
|---|---|---|---|
| *Escherichia coli* | | | |
| P956 | 100,0$\pm$0,0 | 92,5$\pm$0,56 | 80,4$\pm$0,69 |
| P933 | 100,0$\pm$0,0 | 95,2$\pm$0,75 | 81,5$\pm$0,59 |
| P7847 | 100,0$\pm$0,0 | 90,8$\pm$1,29 | 79,4$\pm$0,47 |
| *Klebsiella pneumoniae* | | | |
| H1878 | 100,0$\pm$0,0 | 90,2$\pm$0,68 | 82,8$\pm$0,94 |
| H2001 | 100,0$\pm$00 | 93,7$\pm$0,80 | 81,2$\pm$0,46 |
| H1893 | 100,0$\pm$0,0 | 90,5$\pm$0,25 | 85,2$\pm$0,41 |

*Tableau 4 : Pourcentages d'inhibition des associations de l'AMX (CI 25%) avec le cinéol (CIP 40%, CIP 30% et CIP 20%)*

| Souches | AMX + Cinéol CIP 25 % + CIP 40 % | AMX + Cinéol CIP 25 % + CIP 30 % | AMX + Cinéol CIP 25 % + CIP 20 % |
|---|---|---|---|
| *Escherichia coli* | | | |
| **P956** | 72,0±0,38 | 62,1±0,22 | 55,3±0,75 |
| **P933** | 73,1±0,45 | 59,4±0,32 | 51,2±0,48 |
| **P7847** | 70,1±0,46 | 63,3±0,36 | 53,4±0,60 |
| *Klebsiella pneumoniae* | | | |
| **H1878** | 74,4±0,28 | 61,2±0,26 | 56,3±0,38 |
| **H2001** | 72,4±0,25 | 60,2±0,77 | 53,9±0,67 |
| **H1893** | 71,1±0,29 | 64,4±0,34 | 52,6±0,35 |

Cette puissante synergie a été confirmée par le calcul des indices de fraction (FIC-index, cf. tableau 5) pour les CMI de l'amoxicilline et du cinéol. Pour les 6 souches bactériennes testées, ils varient entre 0,08 et 0,10. Ces résultats, inférieurs à 0,5, témoignent d'une forte synergie d'action entre l'AMX et le cinéol.

*Tableau 5 : FIC-index de l'association amoxicilline et cinéol*

| Souches | CMI 100% | | FIC-Index | Résultat |
|---|---|---|---|---|
| | Seuls | Combinés | | |
| *E. coli* **P956** | 50-100 | 1,1-6,2 | 0,08 | Synergie |
| *E. coli* **P933** | 50-100 | 1,3-6,8 | 0,09 | Synergie |
| *E. coli* **P7847** | 50-100 | 1,5-6,5 | 0,10 | Synergie |
| *K. pneumoniae* **H1878** | 50-100 | 1,6-7,1 | 0,10 | Synergie |
| *K. pneumoniae* **H2001** | 50-100 | 1,3-6,9 | 0,10 | Synergie |
| *K. pneumoniae* **H1893** | 50-100 | 1,7-6,8 | 0,10 | Synergie |

**Discussion**

Les résultats obtenus montrent clairement que les souches testées, très résistantes à l'AMX, deviennent sensibles à des concentrations minimes d'AMX lorsque l'AMX est associée à du cinéol. Ainsi, une inhibition de 100% de croissance de toutes les souches bactériennes testées a été obtenue avec l'association AMX-cinéol (CI 50% + CI 40%). Cette association permet d'obtenir un pouvoir antibactérien similaire à celui de l'AMX seule ou au cinéol seul avec des concentrations largement inférieures à la CMI de l'AMX ou du cinéol. En effet, en comparant les concentrations utilisées pour une inhibition de 100 %, on remarque que la concentration d'AMX utilisée en association avec le cinéol est 25 fois inférieure à celle de l'AMX seule et celle du cinéol utilisée en association avec l'AMX est 15 fois inférieure à celle du cinéol seul.

L'activité bactéricide de cette association d'AMX et de cinéol résulte d'une forte synergie d'action, ainsi que démontrée par le calcul de l'indice de fraction (indice inférieur ou égal à 0,1).

L'ensemble de ces essais démontre l'efficacité de l'association d'AMX et de cinéol contre des bactéries résistantes BLSE et son intérêt dans la lutte pour endiguer les résistances bactériennes.

**Exemple 2** - **Etude *in vitro* de l'effet du cinéol sur l'inhibition de l'amoxicilline par les β-lactamases**

Cette étude repose sur un test enzymatique mettant tout d'abord en contact de l'AMX associée à du cinéol avec des β-lactamases. L'activité antibactérienne de cet antibiotique a ensuite été vérifiée sur une souche d'*Escherichia coli* sensible à l'AMX.

**Matériel et Méthodes**

Matériel biologique, milieu de culture et agents antimicrobiens

Une souche bactérienne *d'Escherichia .coli* (ATCC 8739) sensible à l'amoxicilline a été fournie par l'institut national d'hygiène (INH-Rabat).

Pour chaque test effectué, des précultures de cette souche d'*E. coli* de 24h à 37°C ont été préparées à partir de cette souche bactérienne (auparavant congelée à -20°C). A partir de ces précultures, des inoculums bactériens de $3,3\times10^6$ UFC/ml ont été préparés en ajustant la densité optique à 540 nm.

Les milieux de culture Mueller Hinton gélosé et liquide ont été fournis par BIOKAR (France). La composition de ce milieu a été décrite précédemment. La préparation des deux milieux a été effectuée, selon les instructions du fournisseur.

Des Disques chargés d'antibiotiques (Diamètre Ø = 6 mm) ont été utilisés pour le test enzymatique en milieu gélosé (AMX : Amoxicilline et AMC : Amoxicilline + acide clavulanique). Ils ont été fournis par l'institut national d'hygiène (INH-Rabat). L'AMX utilisée pour le test enzymatique en milieu liquide a été fournie par Sigma Aldrich (France).

Le cinéol a été fourni par Sigma Aldrich (France). Sa préparation a été réalisée par émulsion dans 0,2% (v/v) d'agar agar selon la méthode décrite par Remmal et al (J. Essent. Oil. Res [Book], 1993, 5 : pp. 1179-1184).

La poudre de β-lactamase a été fournie par Sigma Aldrich (France). Elle a été solubilisée, selon les instructions du fournisseur, à 10 mg/ml dans 0,1 M de Tris HCl à pH 7, contenant 0,1 % de gélatine. La β-lactamase (0,03 U/ml) ainsi préparée a été conservée entre 2 et 8° C.

Protocol expérimental en milieu gélosé

En milieu gélosé, la concentration infra-inhibitrice du cinéol (concentration la plus élevée pour laquelle le cinéol n'induit pas d'inhibition) a été déterminée par la méthode de microdilution décrite précédemment. Elle est de l'ordre de 0,002 μl/ml.

A partir d'une préculture d'*E. coli* sensible de 24h, dix-huit boites de Pétri du milieu MH gélosé ont été inoculées par étalement en surface. Six boites témoins et deux boites tests ont été préparées comme suit :
Les boites n°1 et 2 correspondent aux témoins de sensibilité de la souche aux antibiotiques :

- Témoin 1 : Trois disques chargés de l'AMX seule à 20 μg/ml ont été déposés aseptiquement à la surface de la boite n°1.
- Témoin 2 : Trois disques chargés de l'AMC seule à 30 μg/ml ont été déposés aseptiquement à la surface de la boite n°2.

Les boites n°3 et 4 correspondent aux témoins de l'activité de la β-lactamase sur les antibiotiques :

- Témoin 3 : Trois disques chargés de l'AMX à 20 μg/ml ont été déposés aseptiquement à la surface de la boite n°3. A la surface de chacun de ces trois disques, 10μl de la β-lactamase à 0,03 U/ml ont été ajoutés.
- Témoin 4 : Trois disques chargés de l'AMC à 30 μg/ml ont été déposés aseptiquement à la surface de la boite n°4. A la surface de chacun de ces trois disques, 10μl de la β-lactamase à 0,03 U/ml ont été ajoutés.

Les boites n°5 et 6 correspondent aux témoins de l'activité antibactérienne des antibiotiques associés au cinéol (concentration infra-inhibitrice) :

- Témoin 5 : Trois disques chargés de l'AMX à 20 μg/ml ont été déposés aseptiquement à la surface de la boite n°5. A la surface de chacun de ces trois disques, 10 μl du cinéol à 0,002 μl/ml ont été ajoutés.
- Témoin 6 : Trois disques chargés de l'AMC à 30 μg/ml ont été déposés aseptiquement à la surface de la boite n°6. A la surface de chacun de ces trois disques, 10 μl du cinéol à 0,002 μl/ml ont été ajoutés.

Les boites n°7 et 8 correspondent aux tests de l'effet du cinéol sur l'inhibition de l'amoxicilline par les β-lactamases :

- Test 1 : Trois disques chargés de l'AMX à 20 μg/ml ont été déposés aseptiquement à la surface de la boite n°7. A la surface de chacun de ces trois disques, 10μl de la β-lactamase à 0,03 U/ml et 10 μl de cinéol à 0,002 μl/ml ont été ajoutés.
- Test 2 : Trois disques chargés de l'AMC à 30 μg/ml ont été déposés aseptiquement à la surface de la boite n°8. A la surface de chacun de ces trois disques, 10μl de la β-lactamase à 0,03 U/ml et 10 μl de cinéol à 0,002 μl/ml ont été ajoutés.

Après incubation des 8 boites de Pétri pendant 18h à 37°C, les diamètres des halos d'inhibition ont été mesurés.

Protocol expérimental en milieu liquide

La CMI de l'AMX à 100% vis-à-vis de la souche d'*Escherichia coli* sensible, déterminée par la méthode de microdilution décrite précédemment, est de l'ordre de 6 $\mu$g/ml.

A partir d'une préculture de 24h de la souche d'*E coli* sensible, des inoculums bactériens à $3,3 \times 10^6$ UFC/ml ont été préparés en ajustant la densité optique à 540 nm.

Cinq tubes témoins et un tube test ont été préparés comme décrit dans le tableau 6 (cf. ci-dessous).

Le tube n°1 correspond au témoin de stérilité du milieu. Le tube n°2 correspond au témoin positif de viabilité de la souche bactérienne. Le tube n°3 correspond au témoin de sensibilité de la souche à l'AMX. Le tube n°4 correspond au témoin de la non inhibition de la croissance bactérienne par une concentration infra-inhibitrice de cinéol. Le tube n°5 correspond au témoin de dégradation de l'AMX en présence de la $\beta$-lactamase. Le tube n°6 correspond au test de l'effet du cinéol sur l'inhibition de l'amoxicilline par les $\beta$-lactamases.

*Tableau 6: Contenu des tubes témoins et tests pour l'étude en milieu liquide*

| Tubes | Milieu Liquide MH | Inoculum bactérien (3.3 $10^6$ UFC/ml) | AMX (6 $\mu$g/ml) | Cinéol (0,002 $\mu$l/ml) | $\beta$-lactamase (0.03 U/ml) |
|---|---|---|---|---|---|
| **Tube n° 1:** | 1000 $\mu$l | - | - | - | - |
| **Tube n° 2:** | 970 $\mu$l | 30 $\mu$l | - | - | - |
| **Tube n° 3:** | 940 $\mu$l | 30 $\mu$l | 30 $\mu$l | - | - |
| **Tube n° 4:** | 940 $\mu$l | 30 $\mu$l | - | 30 $\mu$l | - |
| **Tube n° 5:** | 930 $\mu$l | 30 $\mu$l | 30 $\mu$l | - | 10 $\mu$l |
| **Tube n°6:** | 900 $\mu$l | 30 $\mu$l | 30 $\mu$l | 30 $\mu$l | 10 $\mu$l |

La DO a été mesurée au temps t=0, et après 22 heures d'incubation à 30°C. Le pourcentage d'inhibition a ensuite été calculé selon la formule décrite précédemment :

$$\% \text{ d'inhibition} = 1 - \frac{DO_{T22} - DO_{T0}}{DO_{C22} - DO_{C0}} \quad X \quad 100$$

Avec $DO_{T0}$=DO du tube test au temps t=0, $DO_{T22}$= DO du tube test après 22h d'incubation, $DO_{C0}$= DO du tube du témoin positif au temps t=0, $DO_{C22}$= DO du tube du témoin positif après 22h d'incubation.

**Résultats**

En milieu gélosé (cf. tableau 7 ci-dessous)

*Tableau 7: Diamètres des halos d'inhibition d'Escherichia. Coli sensible (en mm).*

| Traitement | Diamètres des halos (mm) * | Traitement | Diamètres des halos (mm) * |
|---|---|---|---|
| Témoin 1 : AMX seule | **15 $\pm$ 1.0** | Témoin 2 : AMC seule | **16 $\pm$ 0.4** |
| Témoin 3: AMX + $\beta$-lactamase | **6 $\pm$ 0.0** | Témoin 4 : AMC + $\beta$-lactamase | **13 $\pm$ 0.5** |
| Témoin 5 : AMX + Cinéol | **17 $\pm$ 0.3** | Témoin 6 : AMC + Cinéol | **18 $\pm$ 0.9** |
| Test 1 : AMX+ $\beta$-lactamase+ Cinéol | **12 $\pm$ 1.0** | Test 2 : AMC + $\beta$-lactamase + Cinéol | **15 $\pm$ 0.9** |
| * Moyenne de trois valeurs de halos d'inhibitions | | | |

La mesure des diamètres des halos d'inhibition montrent que :

- La souche d'*E. coli* utilisée est bien sensible à l'AMX (20 $\mu$g) et à la combinaison d'AMX et d'acide clavulanique (AMC 30 $\mu$g) avec des diamètres de halos de 15 et 16 mm, respectivement.
- L'ajout de cinéol à une concentration infra-inhibitrice (0,002 $\mu$l/ml) augmente légèrement la taille des halos d'inhibition, à 17 mm pour l'AMX (20 μg) et à 18 mm pour l'AMC (30 μg).

- L'ajout de β-lactamases (0,03 U/ml), supprime le halo d'inhibition de l'AMX (20 μg) (6 mm étant le diamètre du disque) et réduit celui de l'AMC (30 μg) à environ 13 mm.
- L'ajout de β-lactamases (0,03 U/ml) et de cinéol (0,002 μl/ml), réduit considérablement l'inhibition induite par les β-lactamases avec des diamètres de halo de l'ordre de 12 mm pour l'AMX (20 μg) et de 15 mm pour l'AMC (30 μg).

En milieu liquide (cf. tableau 8 ci-dessous)

*Tableau 8 : Pourcentages d'inhibition de la croissance de la souche d'Escherichia. Coli sensible.*

| Traitement | % d'inhibition |
|---|---|
| Témoin 4 : Cinéol seul | 0 % ± 0,0 |
| Témoin 3 : AMX seule | 100 % ± 0,0 |
| Témoin 5 : AMX + β-lactamase | 0 % ± 0,0 |
| Test 1 : AMX + β-lactamase+ Cinéol | 83,4 % ± 1,1 |

Le tableau 8 montre que :

- La concentration de cinéol utilisée (0,002 μl/ml) est bien infra-inhibitrice, il n'y a pas eu d'inhibition de croissance (Témoin 4).
- En présence de β-lactamases (0,03 U/ml), le pourcentage d'inhibition de la croissance de la souche sensible par l'AMX (6 μg/ml) est nul (Témoin 5).
- En présence de la β-lactamase (0,03 U/ml) et de cinéol (0,002 μl/ml), le pourcentage d'inhibition de la croissance de la souche sensible par l'AMX (6 μg/ml) est de l'ordre de 83,4% (Test 1).

**Discussion**

L'étude réalisée en milieu gélosé a permis de montrer que :

- En présence de cinéol, à une concentration infra-inhibitrice, l'activité antibactérienne de l'AMX et de l'AMC a été améliorée (diamètres des halos d'inhibition supérieurs à ceux obtenus avec les antibiotiques seuls). Ainsi l'activité de l'AMX et de l'AMC sont augmentées en présence de cinéol (à des concentrations infra-inhibitrices).
- En présence de β-lactamases, l'inhibition induite par l'AMX (20 μg) a été nulle et celle induite par l'AMC (30 μg) a été largement réduite. Cela est dû à l'hydrolyse du cycle β-lactame de l'amoxicilline. La β-lactamase n'est que partiellement inhibée par l'acide clavulanique.
- En présence de β-lactamases et de cinéol, l'activité antibactérienne de l'AMX et de l'AMC a été augmentée (diamètres des halos d'inhibition obtenus comparables à ceux obtenus avec les antibiotiques seuls). Dans la mesure où le cinéol a été utilisé à une concentration infra-inhibitrice, l'augmentation d'activité antibactérienne pourrait s'expliquer par une moindre efficacité des β-lactamases sur l'amoxicilline en présence de cinéol.

L'étude réalisée en milieu liquide a permis de montrer que :

- En présence de la β-lactamase, l'AMX perd totalement son activité antibactérienne, ce qui prouve la dégradation complète de cet antibiotique par hydrolyse du cycle β-lactame.
- En présence de β-lactamases et de cinéol, le pourcentage d'inhibition de l'AMX est de l'ordre de 83,4 %. Ces résultats montrent qu'en présence de cinéol à une concentration infra-inhibitrice, l'AMX acquiert une protection contre la β-lactamase qui n'arrive plus à maintenir son activité. L'activité antibactérienne de l'AMX est ainsi préservée.

L'ensemble de ces résultats montre que l'amoxicilline associée au cinéol n'est que faiblement sensible aux β-lactamases. Sans être liée par cette théorie, cela pourrait être dû à une complexation des molécules d'amoxicilline en présence de cinéol qui empêcherait les β-lactamases d'attaquer le cycle β-lactame de l'AMX.

**Exemple 3** - **Etude de l'activité antibactérienne de sérum de lapin traité par une combinaison d'amoxicilline, d'acide clavulanique et de cinéol**

**Matériel et Méthodes**

Matériel biologique, milieu de culture et agents antimicrobiens

Une souche bactérienne d'*Escherichia coli* BLSE multirésistante a été fournie par l'institut national d'hygiène (INH-Rabat). Des précultures de 24h à 37°C ont été préparées à partir de la souche bactérienne (auparavant congelée à -20°C). A partir de ces précultures, des inoculums bactériens de $2\times10^7$ UFC/ml ont été préparés en ajustant la densité optique à 540 nm.

Les milieux Mueller Hinton (MH) gélosés et liquides ont été fournis par BIOKAR (France). La composition de ces milieux a été décrite précédemment. La préparation des deux milieux a été effectuée, selon les instructions du fournisseur.

Les six lapins utilisés dans cette étude sont des lapins femelles de race néo-zélandaise, fournis par un éleveur de spécialité. Ils sont âgés de 70 à 75 jours et pèsent environ 2kg. Ils ont été répartis au hasard en 2 lots de 3 lapins et ont été nourris *ad libitum* avec un aliment industriel de type engraissement.

Protocol expérimental

Les lapins du 1er lot ont reçu une seule dose d'AMC comprenant 1,5 g d'amoxicilline et 186,5 mg d'acide clavulanique. Les lapins du 2ème lot ont reçu une seule dose d'AMC combiné à du cinéol comprenant 1,5 g d'amoxicilline, 186,5 mg d'acide clavulanique et 300 mg de cinéol.

Les solutions des deux traitements, reconstituées avec de l'eau purifiée, ont été administrées aux lapins par gavage stomacal.

Après administration du traitement, les lapins ont été immobilisés par contention à l'aide d'un dispositif approprié. Leurs oreilles ont été exposées à une lampe à infrarouge afin de dilater les veines auriculaires marginales et centrales. Des prélèvements sanguins de 0,5 ml ont été ensuite effectués au niveau de la veine auriculaire centrale au temps T=0 ($T_0$) (avant l'administration), au temps $T_1$ (après une heure), au temps $T_2$ (après deux heures), au temps $T_3$ (après trois heures) et au temps $T_6$ (après six heures).

La méthode de microdilution, décrite précédemment, a été utilisée pour déterminer le pourcentage d'inhibition des échantillons de sérum.

Des microplaques stériles à fond en U de 96 cupules et d'une capacité de 200$\mu$l ont été utilisées. Le témoin négatif consiste en 200 $\mu$l de milieu liquide MH et le témoin positif en 150 $\mu$l de milieu liquide MH et 50 $\mu$l d'inoculum bactérien à $2\times10^7$ UFC/ml. Pour chaque échantillon de sérum prélevé à un temps donné, deux cupules ont été réalisées contenant chacune 100 $\mu$l du milieu liquide MH, 50 $\mu$l de l'inoculum bactérien à $2\times10^7$ UFC/ml et 50 $\mu$l de sérum.

Pour chaque échantillon, une lecture de DO est réalisée à t=0 et après incubation des microplaques pendant 22 heures à 30°C. Le pourcentage d'inhibition des différents échantillons de sérum a été calculé selon la formule décrite précédemment :

$$\% \text{ d'inhibition} = 1 - \frac{DO_{T22}-DO_{T0}}{DO_{C22}-DO_{C0}} \quad X \quad 100$$

Avec $DO_{T0}$=DO de la cupule test au temps T0, $DO_{T22}$= DO de la cupule test après 22h d'incubation, $DO_{C0}$= DO de la cupule du témoin positif au temps t=0, $DO_{C22}$= DO de la cupule du témoin positif après 22h d'incubation.

**Résultats**

La figure 1 présente le suivi des pourcentages d'inhibition de la croissance bactérienne par les échantillons de sérums de lapins du lot traité par l'AMC seul (combinaison amoxicilline/acide clavulanique) et du lot traité par l'AMC boosté par le cinéol au cours du temps.

Avant administration du traitement (temps $T_0$), l'activité antibactérienne des échantillons de sérums de lapin des deux est quasi nulle. Une heure après administration du traitement (Ti), l'inhibition induite par le sérum des lapins traités par l'AMC seule est de l'ordre de 40 % $\pm$ 1,2 alors que celle induite par le sérum de lapins traités par l'AMC et le cinéol est de l'ordre de 47 % $\pm$ 2,1. Après deux heures de traitement, les pourcentages d'inhibition sont de 50 % $\pm$ 2,5 pour les sérums de lapins traités par l'AMC seule et de 54 % $\pm$ 2,9 pour ceux traités par l'AMC boostée par le cinéol. Après trois heures de traitement, les pourcentages d'inhibition diminuent à 41 % $\pm$ 3,2 pour les lapins traités avec l'AMC seule et à 48 % $\pm$ 1,6 pour les lapins traités avec l'AMC boostée par le cinéol. Enfin après six heures de traitement, les pourcentages d'inhibition chutent à 16 % $\pm$ 1,3 pour les sérums de lapins traités avec l'AMC seul alors qu'ils sont encore de l'ordre de 44 % $\pm$ 1,5 pour les lapins traités avec l'AMC boostée au cinéol.

Ainsi, les pourcentages d'inhibition obtenus avec les échantillons de sérum des lapins du lot traité par la combinaison d'amoxicilline, d'acide clavulanique et de cinéol sont significativement supérieurs à ceux obtenus avec les échantillons de sérums des lapins du lot de référence traité par la combinaison d'amoxicilline et d'acide clavulanique (p<0,05).

**Exemple 4** - **Développement galénique d'une formulation comprenant de l'amoxicilline, de l'acide clavulanique et du cinéol**

**Matériel et méthodes**

Toutes les matières premières, principes actifs et excipients, utilisées sont de qualité pharmaceutique.

Cette formulation se caractérise par la présence de cinéol, un principe actif volatil. Pour stabiliser ce composé volatil, plusieurs excipients ou combinaisons d'excipients présentant des propriétés adsorbantes ont été testés (cf. tableau 9 ci-dessous).

Le procédé testé pour la forme N° 8 (Huile d'arachide) consiste à introduire une étape de mouillage du granulé obtenu avec le mélange des autres principes actifs, l'amoxicilline et l'acide clavulanique, et des excipients avec une phase huileuse comprenant le cinéol. L'étape de mouillage peut être suivie d'une étape de lubrification, de mélange et de tamisage.

*Tableau 9: Composition en adsorbants des formulations testées*

| Adsorbants | Formulations testées | | | | | | | |
|---|---|---|---|---|---|---|---|---|
| | Forme 1 | Forme 2 | Forme 3 | Forme 4 | Forme 5 | Forme 6 | Forme 7 | Forme 8 |
| Levillite | √ | | √ | √ | | | | |
| Tixosil | | √ | | √ | | | | |
| Talc | | | √ | | | | | |
| Aerosil | | | √ | √ | | | | |
| Diméthicone syloide | | | | | √ | | | |
| syloide | | | | | | √ | | |
| Starcap | | | | | | | √ | |
| Huile d'arachide | | | | | | | | √ |

Toutes les matières premières ont été tamisées avant mélange sur différentes grilles du tamiseur FREWITT.

Le mélange des matières premières a été effectué dans un mélangeur de type HOBART.

Le remplissage en sachet a été effectué en utilisant l'ensacheuse MARCHESINI.

La température et l'humidité relative de la salle de fabrication ont été respectivement de l'ordre de < 20 °C et < 15 %.

La solution de mouillage, confectionnée pour le procédé de stabilisation du cinéol, a été préparée dans une enceinte fermée pour éviter toute évaporation.

Pour chaque forme testée, un contrôle complet de la qualité y compris le dosage des trois principes actifs (amoxicilline, acide clavulanique et cinéol) a été effectué :

- sur le mélange final, en fin de fabrication (dix points de prélèvement) au temps $T_0$ et après 24h, 48h et 72h d'exposition à une température < 20 °C et pour une humidité ambiante < 15 %.

- sur le sachet en fin de remplissage (dix points de prélèvement)

Les teneurs en principes actifs (%) comprises entre 95 % et 105 % sont jugées conformes. L'homogénéité du mélange est vérifiée par calcul des coefficients de variation (CV %) des teneurs individuelles obtenues pour les dix points de prélèvements. Les coefficients de variation inférieurs ou égaux à 2 % sont jugés conformes.

**Résultats**

Les teneurs moyennes en amoxicilline et acide clavulanique sont conformes pour toutes les formes testées (formes 1 à 8). Concernant le cinéol (cf. tableau 10 ci-dessous), les formes 1 à 7 présentent, dès le mélange final, une teneur en cinéol inférieure à 95 %, teneur qui décroit encore de façon importante après 24h, 48h et 72h d'exposition à une température < 20°C et pour une humidité ambiante < 15 %.

# EP 3 463 479 B1

La forme 8 (huile d'arachide) permet par contre de stabiliser le cinéol. En effet, le taux de cinéol présent dans cette forme est compris entre 95 % et 105 % dans le mélange final et se maintient dans cette gamme après 24h, 48h et 72h d'exposition à une température < 20°C et pour une humidité ambiante < 15 %.

L'homogénéité du mélange final et du remplissage en sachet est également conforme pour la formule 8, les valeurs des coefficients de variations (CV %) des teneurs ne dépassant pas 2 %.

*Tableau 10 : Teneur en cinéol (%) en fonction des différents adsorbants testés*

| Essais de faisabilité | Teneur en Cinéol (%) / Coefficient de variation (%) | | | | |
| | Mélange final | Sachet/fin de remplissage | Stabilité du mélange final | | |
| | | | Après 24h | Après 48h | Après 72h |
|---|---|---|---|---|---|
| Forme 1 | 81,9 % / 0,9 % | 69,8 % / 1,5 % | 75,2 % / 1,7 % | 62,2 % / 0,9 % | 57,8 % / 0,7 % |
| Forme 2 | 90,9 % / 1,7 % | 75,6 % / 1,1 % | 81,0 % / 1,5 % | 70,4 % / 1,3 % | 62,0 % / 1,5 % |
| Forme 3 | 89,0 % / 0,9 % | 72,3 % / 1,6 % | 74,2 % / 1,3 % | 70,1 % / 1,1 % | 59,3 % / 1,6 % |
| Forme 4 | 94,1 % / 0,6 % | 92,1 % / 0,8 % | 86,3 % / 1,3 % | 80,7 % / 1,8 % | 72,5 % / 1,3 % |
| Forme 5 | 75,9 % / 1,6 % | 72,6 % / 1,2 % | 60,8 % / 1,0 % | 55,7 % / 1,6 % | 50,8 % / 1,1 % |
| Forme 6 | 90,9 % / 1,3 % | 86,2 % / 1,3 % | 84,2 % / 1,5 % | 77,2 % / 1,2 % | 70,3 %/ 1,7 % |
| Forme 7 | 93,9 % / 0,9 % | 91,6 % / 0,7 % | 88,1 % / 1,6 % | 80,3 % / 1,4 % | 74,2 % / 1,5 % |
| Forme 8 | 102,3 % / 0,8 % | 102,1%/1,2 % | 102,1 %/1,0 % | 101,8 %/1,0 % | 101,7 %/1,3 % |

Par ailleurs, les tests de quantification d'impuretés des trois principes actifs (amoxicilline, acide clavulanique et cinéol) dans le mélange final exposé à une température < 20 °C et à une humidité ambiante < 15 % pendant 24h, 48h et 72h sont tous conformes aux critères d'acceptation (résultats non présentés). Les résultats des autres tests de qualité du mélange final et de la répartition en sachet sont également tous conformes aux critères d'acceptations (résultats non présentés).

**Exemple 5 - Exemple de formulation pharmaceutique suivant l'invention**

| Nom du composant | Quantité pour un sachet uni-dose de 3 g |
|---|---|
| Amoxicilline trihydratée | correspondant à une quantité d'amoxicilline de 500 mg |
| Mélange 1:1 de clavulanate de potassium et de silice colloïdale hydratée | correspondant à une quantité d'acide clavulanique de 62,5 mg |
| Cinéol | 100 mg |
| Syloid® A1 FP | 540 mg |
| Aspartame | 36 mg |
| Huile d'arachide | 50 mg |
| Croscarmellose sodique | 300 mg |
| Cellulose microcrystalline Avicel ® PH112 | 1241 mg* |
| Stéarate de magnésium | 18 mg |
| Aromatisant | 90 mg |
| *: La quantité de cellulose microcrystalline est ajustée pour que le poids total soit de 3 g. | |

Cette formulation a été obtenue par le procédé suivant :

**ETAPE 1 : Tamisage et pré-mélange**

L'Avicel PH 112, le Croscarmellose sodium et le Syloid Al sont mélangés après tamisage.

**ETAPE 2 : Compactage**

**2-1 Amoxcicilline**

L'Amoxicilline trihydratée est mélangée à une partie du pré-mélange de poudre obtenu à l'étape 1 puis compactée. Les granulés obtenus sont ensuite calibrés puis fractionnés.

**2-2 Acide Clavulanique**

L'Acide clavulanique est mélangé à une partie du pré-mélange de poudre obtenu à l'étape 1 puis compacté. Les granulés obtenus sont ensuite calibrés puis fractionnés.

**ETAPE 3 : Granulation**

**3-1 Préparation de la solution de Mouillage (S1)**

La solution de mouillage est obtenue en mélangeant le cinéol et l'huile d'arachide dans un récipient fermé puis fractionnée.

**3-2 Mouillage**

Les fractions du mélange compacté comprenant l'amoxicilline obtenues à l'étape 2 sont mouillées par les fractions de la solution S1 puis mélangées. Les fractions du mélange compacté comprenant l'acide clavulanique obtenues à l'étape 2, sont ajoutées.

**3.3.- Mélange**

Les différentes fractions sont réunies et mélangées.

**ETAPE 4 : Lubrification**

L'Aspartame et la composition aromatique sont mélangés après tamisage. Le stéarate de magnésium est ensuite ajouté.

**ETAPE 5 : Tamisage et mélange final**

La poudre finale est tamisée puis mélangée pendant quelques minutes.

**ETAPE 6 : Répartition**

Le mélange final est réparti dans des sachets.

**ETAPE 7 : Conditionnement secondaire en boites**

**Exemple 6** - **Etude de la stabilité d'une formulation pharmaceutique en poudre comprenant de l'amoxicilline, de l'acide clavulanique, du cinéol et de l'huile d'arachide**

**Matériel et Méthodes**

La stabilité de la formulation pharmaceutique de l'exemple 5 a été testée dans trois conditions différentes (cf. tableau 11).

*Tableau 11: Conditions expérimentales de l'étude de stabilité de la formulation*

|  | Température | Humidité relative (HR) | Durée de l'étude |
|---|---|---|---|
| **Etude de stabilité en condition 1** | 25 °C $\pm$ 2°C | 60% $\pm$ 5% | 24 mois |
| **Etude de stabilité en condition 2** | 30 °C $\pm$ 2°C | 65% $\pm$ 5% | 12 mois |
| **Etude de stabilité en condition 3** | 40 °C $\pm$ 2°C | 75% $\pm$ 5% | 6 mois |

Des enceintes climatiques à température et humidité relative contrôlées ont été utilisées afin de maintenir les formulations dans les conditions choisies.

Pour la condition 1, un contrôle de qualité a été effectué tous les 3 mois pendant la première année et tous les 6 mois pendant la seconde année (0, 3, 6, 9, 12, 18 et 24 mois).

Pour les conditions 2 et 3, un contrôle de qualité a été effectué tous les 3 mois (0, 3, 6, 9 et 12 mois, pour la condition 2 et 0, 3 et 6 mois pour la condition 3).

Ce contrôle de qualité a concerné :

- Le contrôle des qualités organoleptiques, de la teneur en eau et du pH de la suspension.
- Le dosage des trois principes actifs (amoxicilline, acide clavulanique et cinéol).
- La quantification des impuretés des trois principes actifs.
- Un contrôle microbiologique.

**Résultats**

Dans les trois conditions, tout au long de l'étude, les teneurs des trois principes actifs ont été mesurées entre 95 % et 105 % (cf. tableaux 12 à 14 ci-dessous), démontrant la stabilité de la composition.

*Tableau 12 : Résultats de l'étude de stabilité en condition 1*

|  | T 0 | T3 | T 6 | T9 | T12 | T18 | T24 |
|---|---|---|---|---|---|---|---|
| **Teneur en AMX (%)** | 100,6 % | 100,4 % | 100,3 % | 100,3 % | 100,1 % | 99,7 % | 99,4 % |
| **Teneur en Acide clavulanique (%)** | 100,2 % | 100,1 % | 100,1 % | 99,8 % | 99,7 % | 99,5 % | 99,0 % |
| **Teneur en Cinéol (%)** | 100,8 % | 100,5 % | 100,4 % | 100,2 % | 99,9 % | 99,5 % | 99,2 % |

*Tableau 13 : Résultats de l'étude de stabilité en condition 2*

|  | T 0 | T3 | T 6 | T9 | T12 |
|---|---|---|---|---|---|
| **Teneur en AMX (%)** | 100,6 % | 100,2 % | 99,9 % | 99,6 % | 99,2 % |
| **Teneur en Acide clavulanique (%)** | 100,2 % | 99,8 % | 99,5 % | 99,4 % | 99,1 % |
| **Teneur en Cinéol (%)** | 100,8 % | 100,3 % | 100,0 % | 99,6 % | 99,3 % |

*Tableau 14 : Résultats de l'étude de stabilité en condition 3*

|  | T 0 | T3 | T 6 |
|---|---|---|---|
| **Teneur en AMX (%)** | 100,6 % | 100,0 % | 99,3 % |
| **Teneur en Acide clavulanique (%)** | 100,2 % | 99,6 % | 98,9 % |
| **Teneur en Cinéol (%)** | 100,8 % | 99,7 % | 99,0 % |

La quantification des impuretés des trois principes actifs est également conforme aux critères d'acceptations pour les trois conditions (résultats non présentés).

Les autres paramètres contrôlés (teneur en eau, aspect de la poudre et de la suspension reconstituée, pH de la suspension reconstituée et contrôle microbiologique) sont tous conformes aux critères d'acceptations (résultats non présentés).

Ainsi, la formulation de l'invention conserve ses propriétés chimiques, physiques, organoleptiques et microbiologiques dans les trois conditions testées.

**Exemple 7** - Etude pharmacocinétique chez l'homme

**Protocol expérimental** :

48 sujets volontaires sains ont été recrutés pour ces expériences.

Dans une première expérience, les volontaires, répartis en deux groupes de 12, ont reçus par voie orale soit 12 g de la composition de l'exemple 5 (soit au total 2 g amoxicilline, 250 mg d'acide clavulanique et 400 mg de cinéol), soit 12 g d'une composition en tout point identique mais dépourvue de cinéol. Des prélèvements sanguins ont été réalisés toutes les demi-heures pendant les 3 premières heures, puis toutes les heures jusqu'à 6 heures, puis à 8 heures, à 10 heures et à 24 heures. La concentration plasmatique d'amoxicilline des échantillons prélevés a été dosée par chromatographie et la cinétique sérique de l'amoxicilline analysée.

Dans une deuxième expérience, les volontaires, répartis en deux groupes de 12, ont tout d'abord reçus les mêmes doses que dans l'expérience précédente avant de recevoir 3 fois par jour pendant une semaine des doses d'entretien de 3 g des mêmes compositions, c'est à dire soit 3 fois 3 g par jour de la composition de l'exemple 5 (soit pour chaque prise 500 g amoxicilline, 62,5 mg d'acide clavulanique et 100 mg de cinéol), soit 3 fois 3 g par jour d'une composition en tout point identique mais dépourvue de cinéol. Des prélèvements sanguins ont été réalisés toutes les 24 heures (concentration minimale) ainsi que deux heures après, au moment du pic de concentration (à t = 26 h, 50 h, 72 h, etc.) pendant 7 jours. La concentration plasmatique d'amoxicilline des échantillons prélevés a été dosée par chromatographie, et la cinétique sérique de l'amoxicilline analysée.

**Cinétique de l'amoxicilline pendant 24 h après administration d'une dose** unique :

Les courbes de suivi des concentrations sériques moyennes de l'amoxicilline obtenues avec des volontaires traités par une combinaison d'amoxicilline et d'acide clavulanique ou avec une combinaison d'amoxicilline, d'acide clavulanique et de cinéol se superposent presque parfaitement, en dépit d'une forte variabilité interindividuelle (cf. figure 2). Ils présentent la même allure pendant la phase d'absorption et les concentrations maximales (Cmax) sont atteintes vers deux heures et demie pour les deux formes. Les concentrations commencent à décroître à partir de deux heures et demie pour les deux formes et les deux courbes d'élimination restent presque parallèles entre le moment de l'administration et 24 h plus tard.

Pour l'ensemble des paramètres pharmacocinétiques étudiés, à savoir l'aire sous la courbe ($AUC_{0-t}$), la concentration maximale (Cmax), le temps au pic (Tmax) et le temps de demi-vie ($t_{1/2}$), les valeurs obtenues ne sont pas significativement différentes entre les deux conditions testées (cf. tableau 15 ci-dessous).

La biodisponibilité relative de l'amoxicilline dans la forme comprenant du cinéol par rapport à la forme n'en comprenant pas est : **F (AUC) = 0.888.**

L'ensemble de ces résultats permet de conclure que les deux compositions étudiées présentent une même biodisponibilité.

*Tableau 15 : Paramètres pharmacocinétiques de l'amoxicilline sérique*

| Paramètres | $AUC_{0-t}$ (ng.h/ml) | Cmax (ng/ml) | Tmax (heure) | $t_{1/2}$ (heure) |
|---|---|---|---|---|
| Composition d'AMX et d'acide clavulanique | 56161 ± 26760 | 11991 ± 1371 | 2,73 ± 0,68 | 1,66 ± 0,45 |
| Composition d'AMX, d'acide clavulanique et de cinéol | 50417 ± 12186 | 11322 ± 3171 | 2,38 ± 0,40 | 1,67 ± 0,44 |

**Cinétique de l'amoxicilline pendant l'administration de doses répétées (7 jours)** :

Les courbes de suivi des concentrations sériques moyennes de l'amoxicilline obtenues avec des volontaires traités par une combinaison d'amoxicilline et d'acide clavulanique ou avec une combinaison d'amoxicilline, d'acide clavulanique et de cinéol se superposent presque parfaitement (cf. figure 3).

Ces résultats montrent que les deux compositions étudiées ont le même comportement pharmacocinétique lors d'administrations réitérées.

**Exemple 8 - Essai clinique sur des patients à infection bactérienne sensible**

L'objectif de cet essai clinique était d'évaluer, chez des patients souffrant d'une infection urinaire à bactéries sensibles à l'amoxicilline, l'efficacité d'une formulation pharmaceutique comprenant de l'amoxicilline, de l'acide clavulanique et du cinéol par rapport à une formulation comprenant uniquement de l'amoxicilline et de l'acide clavulanique. Cet essai clinique randomisé a été réalisé sur une population de 28 patients répartis en 2 groupes (ou bras) de 14 patients chacun. Les patients de chaque groupe ont été traités en parallèle pendant 7 jours. Les patients sont des hommes et des femmes âgés de plus de 20 ans.

Un antibiogramme réalisé sur les patients avant le début du traitement a permis de valider qu'ils souffrent tous d'une infection urinaire à germes bactériens sensibles.

**Critère d'évaluation de l'efficacité :**

L'efficacité des traitements est évaluée par un examen cytobactériologique des urines (ECBU) réalisé en fin de traitement.

**Produits et posologie :**

Formulation test de l'exemple 5 (amoxicilline acide clavulanique et cinéol) comprenant 500 mg d'amoxicilline, 62,5 mg d'acide clavulanique et 100 mg de cinéol pour 3 grammes de poudre (correspondant au contenu d'un sachet).

Chaque patient éligible aux traitements de l'étude a reçu de façon aléatoire selon la table de randomisation :

- Une dose de charge de 12 grammes (4 sachets en une prise) de la formulation test le premier jour, puis 3 sachets par jour de la même formulation (1 le matin, 1 le midi et 1 le soir) pendant 6 jours (bras A).
- Une dose de charge de 12 grammes (4 sachets en une prise) de la formulation test le premier jour, puis 6 sachets par jour de la même formulation (2 le matin, 2 le midi et 2 le soir) pendant 6 jours (bras B).

Le tableau 16 ci-dessous récapitule la répartition des patients dans les différents bras ainsi que la nature de l'infection bactérienne détectée lors de l'ECBU préliminaire au traitement.

**Résultats** :

Le traitement a été efficace dans tous les cas (élimination de la bactérie initialement détectée) sauf pour un patient sur lequel le traitement a été inefficace dans le bras A (Patient N°2).

Le traitement s'est même avéré efficace chez certains patients plus complexes car réputés difficiles à stériliser en cas d'infection, à savoir des patients porteurs d'hypertrophie bénigne de la prostate, des patients diabétiques, présentant des sténoses de l'urètre, des dérivations urinaires, des calculs de la vessie ou des tumeurs de la vessie.

En conclusion, le traitement testé (amoxicilline, acide clavulanique et cinéol), tout comme le traitement de référence (amoxicilline et acide clavulanique) permet de traiter efficacement les infections urinaires à germes bactériens sensibles, y compris sur des terrains difficiles.

*Tableau 16 : Caractéristiques des patients et leur répartition*

| N° du Patient | Age (ans) | Sexe | Bactérie responsable de l'infection urinaire du patient | Bras |
|---|---|---|---|---|
| 1 | 65 | Homme | *E. coli* | B3 |
| 2 | 44 | Homme | *E. coli* | A5 |
| 3 | 80 | Homme | *E. coli* | A1 |
| 4 | 75 | Femme | *Staphylococus heamolyticus* | B12 |
| 5 | 20 | Homme | *Staphylococus epidermidis* | A14 |
| 6 | 66 | Femme | *E. coli* | A8 |
| 7 | 43 | Femme | *E. coli* | A7 |
| 8 | 46 | Homme | *Proteus mirabilis* | A4 |
| 9 | 66 | Homme | *Klebsiella pneumoniae* | B2 |
| 10 | 69 | Homme | *Staphylococus agalactiae* | A6 |
| 11 | 46 | Homme | *E. coli* | A9 |
| 12 | 58 | Homme | *Staphylococus agalactiae* | A2 |
| 13 | 52 | Homme | *Staphylococus agalactiae* | B9 |
| 14 | 78 | Femme | *E. coli* | B6 |

(suite)

| N° du Patient | Age (ans) | Sexe | Bactérie responsable de l'infection urinaire du patient | Bras |
|---|---|---|---|---|
| 15 | 77 | Femme | *E. coli* | A3 |
| 16 | 69 | Homme | *Streptococus agalpactie* | B4 |
| 17 | 76 | Homme | *E. coli* | B1 |
| 18 | 54 | Femme | *E. coli* | B5 |
| 19 | 55 | Femme | *Klebsiella oxytoca* | B7 |
| 20 | 52 | Femme | *E. coli* | A10 |
| 21 | 74 | Homme | *E. coli* | B8 |
| 22 | 70 | Homme | *Enterocoque* | B10 |
| 23 | 40 | Femme | *Streptococcus agalactiae* | A11 |
| 24 | 85 | Homme | *Klebsiella pneumoniae* | A12 |
| 25 | 26 | Homme | *E. coli* | B11 |
| 26 | 33 | Femme | *E. coli* | A13 |
| 27 | 60 | Homme | *E. coli* | B14 |
| 28 | 90 | Homme | *Streptocoque D* | B13 |

**Exemple 9** - **Essai clinique sur des patients à infection bactérienne résistante**

L'objectif de cet essai clinique était d'évaluer, chez des patients souffrant d'une infection urinaire à bactéries résistantes à une combinaison d'amoxicilline et d'acide clavulanique, l'efficacité d'une formulation pharmaceutique comprenant de l'amoxicilline, de l'acide clavulanique et du cinéol. Cet essai clinique a été réalisé sur une population de 23 patients traités pendant 7 jours.

La formulation testée (cf. exemple 2) est une poudre comprenant 500 mg d'amoxicilline, 62,5 mg d'acide clavulanique et 100 mg de cinéol pour 3 grammes de poudre (correspondant au contenu d'un sachet).

Chaque patient a d'abord reçu une dose de charge de 12 grammes (4 sachets en une prise) de la formulation test le premier jour, puis 6 sachets par jour de la même formulation (2 le matin, 2 le midi et 2 le soir) pendant 6 jours.

Le tableau 17 ci-dessous récapitule les caractéristiques des patients de cet essai dont la nature de l'infection bactérienne détectée lors de l'ECBU préliminaire au traitement.

**Résultats :**

Le traitement a été efficace dans tous les cas (élimination de la bactérie initialement détectée) sauf pour un patient (Patient N°6).

Le cas du patient N°22 est particulièrement intéressant. En effet, ce patient présentait une infection urinaire considérée comme réfractaire à tous les antibiotiques disponibles testés depuis près de 20 ans, dont l'amoxicilline et l'acide clavulanique, et a été guéri par la formulation selon l'invention.

En conclusion, la formulation selon l'invention comprenant de l'amoxicilline, de l'acide clavulanique et du cinéol s'est avérée très efficace contre les infections urinaires à germes bactériens résistants au traitement de référence, à savoir une formulation comprenant de l'amoxicilline et de l'acide clavulanique.

*Tableau 17: Caractéristiques des patients*

| N° de Patient | Age | Sexe | Bactérie responsable de l'infection urinaire du patient |
|---|---|---|---|
| 1 | 37 | Femme | *Staphylococcus aureus* |
| 2 | 49 | Homme | *Pseudomonas aeruginosa* |
| 3 | 46 | Homme | *E. coli* |
| 4 | 22 | Femme | *E. coli + Candida albicans* |
| 5 | 82 | Homme | *E. coli* |

(suite)

| N° de Patient | Age | Sexe | Bactérie responsable de l'infection urinaire du patient |
|---|---|---|---|
| 6 | 37 | Homme | *E. coli* |
| 7 | 46 | Homme | *Acinetobacter iwoffii* |
| 8 | 83 | Femme | *E. coli* |
| 9 | 71 | Homme | *E. coli* |
| 10 | 76 | Homme | *Klebsiella pneumoniae* |
| 11 | 76 | Homme | *E. coli* |
| 12 | 53 | Homme | *Klebsiella pneumoniae* |
| 13 | 66 | Femme | *E. coli* |
| 14 | 39 | Femme | *E. coli* |
| 15 | 84 | Homme | *E. coli* |
| 16 | 69 | Homme | *Klebsiella pneumoniae* |
| 17 | 77 | Homme | *Enterobacter cloacae* |
| 18 | 59 | Homme | *Klebsiella pneumoniae* |
| 19 | 21 | Homme | *E. coli* |
| 20 | 25 | Femme | *E. coli* |
| 21 | 71 | Homme | *E. coli* |
| 22 | 65 | Femme | *Klebsiella oxytoca* |
| 23 | 49 | Femme | *Proteus mirabilis* |

**Exemple 10 - Etude spectroscopique de la formation de complexes d'amoxicilline**

Les analyses de spectroscopie ont été réalisées avec des solutions comprenant 500 mg d'amoxicilline et, le cas échéant, 62,5 mg d'acide clavulanique ou 100 mg de cinéol en solution dans 100 ml d'eau.

L'analyse par spectroscopie de masse d'amoxicilline en solution dans de l'eau (cf. figure 4-A) montre la présence d'un pic principal correspondant à de l'amoxicilline moléculaire (pic à 349,06 uma) ainsi que d'un autre pic correspondant à de l'amoxicilline sous forme de dimère (pic à 731,17 uma). L'amplitude du pic des dimères d'amoxicilline est très largement inférieure à celle du pic de l'amoxicilline moléculaire. Ainsi, lorsqu'elle est seule en solution, l'amoxicilline est de façon très majoritaire sous forme moléculaire.

Lorsque de l'amoxicilline est mise en solution en présence de cinéol, en plus des pics déjà observés pour l'amoxicilline seule, de nouveaux pics apparaissent en spectroscopie de masse (cf. figure 4-B). Ces pics correspondant à des trimères d'amoxicilline (pic à 1134,24 uma) et à des tétramères d'amoxicilline (pic à 1499,34 uma).

Par contre, lorsque de l'amoxicilline est mise en solution en présence d'acide clavulanique, et en absence de cinéol, le profil spectroscopique de l'amoxicilline est inchangé (résultats non montrés).

Ainsi, l'ajout de cinéol permet le réarrangement des molécules d'amoxicillines en solution sous formes d'oligomères de 3 à 4 molécules d'amoxicilline. Ce réarrangement n'est pas observé lorsque l'amoxicilline n'est mise en présence que d'acide clavulanique.

**Revendications**

1. Formulation pharmaceutique sous forme de poudre comprenant, ou consistant essentiellement en, du cinéol, de l'amoxicilline, un inhibiteur de β-lactamases et une huile pharmaceutiquement acceptable, ladite huile pharmaceutiquement acceptable étant l'huile d'arachide.

2. Formulation selon la revendication 1, dans laquelle ledit inhibiteur de β-lactamases est de l'acide clavulanique.

3. Formulation selon l'une quelconque des revendications précédentes, dans laquelle ladite formulation comprend entre environ 5 mg et environ 100 mg de cinéol par gramme de poudre.

4. Formulation selon l'une quelconque des revendications précédentes, dans laquelle ladite formulation comprend entre environ 20 mg et environ 500 mg d'amoxicilline par gramme de poudre.

5. Formulation selon l'une quelconque des revendications précédentes, dans laquelle ladite formulation comprend entre environ 2 mg et environ 50 mg d'huile par gramme de poudre.

6. Formulation selon l'une quelconque des revendications 2 à 5, dans laquelle ladite formulation comprend entre environ 1 mg et environ 100 mg d'acide clavulanique par gramme de poudre.

7. Formulation selon l'une quelconque des revendications précédentes, dans laquelle le ratio massique amoxicilline/-cinéol est compris entre 2 et 8, de préférence entre 3 et 7, de manière plus particulièrement préférée entre 4 et 6.

8. Formulation selon l'une quelconque des revendications précédentes, dans laquelle le ratio massique amoxicilline/-huile est compris entre 5 et 15, de préférence entre 7 et 13, et de manière plus particulièrement préférée entre 8 et 12.

9. Formulation selon l'une quelconque des revendications précédentes, dans laquelle le ratio massique cinéol/huile est compris entre 0,1 et 5, de préférence entre 0.5 et 4, de manière plus particulièrement préférée entre 1 et 3.

10. Formulation selon l'une quelconque des revendications 1 à 9, dans laquelle le ratio massique amoxicilline/inhibiteur de β-lactamases est compris entre 5 et 11, de préférence entre 6 et 10, de manière plus particulièrement préférée entre 7 et 9.

11. Formulation selon l'une quelconque des revendications précédentes, dans laquelle ladite formulation est destinée à une administration par voie orale, de préférence après suspension dans un solvant aqueux.

12. Formulation selon l'une quelconque des revendications précédentes, dans laquelle ladite formulation est conditionnée dans un récipient unidose, de préférence un récipient unidose contenant entre environ 1 g et environ 150 g de poudre, de préférence entre environ 1 g et environ 10 g de poudre.

13. Formulation selon l'une quelconque des revendications précédentes, dans laquelle ladite formulation comprend en outre au moins un excipient ou support pharmaceutiquement acceptable, de préférence sélectionné dans le groupe consistant en un édulcorant, un aromatisant, un antiagglomérant, un lubrifiant, un désintégrant, et un mélange de ceux-ci.

14. Formulation selon l'une quelconque des revendications précédentes pour une utilisation dans le traitement d'une pathologie infectieuse chez un sujet, de préférence une pathologie infectieuse d'origine bactérienne, de manière préférée une pathologie infectieuse causée par une bactérie résistante aux antibiotiques de la famille des β-lactamines.

15. Formulation pour une utilisation selon la revendication 14, dans laquelle la pathologie infectieuse est sélectionnée dans le groupe constitué des cystites, en particulier les cystites aiguës récidivantes, des sinusites bactériennes, en particulier les sinusites maxillaires aiguës, des otites, en particulier les otites moyennes aiguës, des bronchites, en particulier les bronchites chroniques et/ou aiguës, des bronchopneumopathies, en particulier les bronchopneumopathies chroniques et/ou aiguës, des pyélonéphrites, des infections gynécologiques hautes, des parodontites, des infections stomatologiques sévères, en particulier les abcès, les phlegmons et les cellulites, des morsures animales, des infections des os et des articulations, en particulier l'ostéomyélite, de préférence ladite infection bactérienne est une cystite, en particulier causée par une bactérie résistante aux antibiotiques de la famille des β-lactamines.

16. Formulation pour une utilisation selon la revendication 14 ou 15, dans laquelle la formulation est administrée au sujet durant une période allant de 1 jour à 4 semaines, de préférence durant une période d'environ 7 jours, et à raison de 3 à 30 grammes par jour, en une seule ou plusieurs prises journalières.

17. Procédé de fabrication de la formulation pharmaceutique selon l'une quelconque des revendications 1 à 13 comprenant :

  - l'obtention d'une solution de mouillage par mélange de cinéol et d'une huile pharmaceutiquement acceptable, ladite huile pharmaceutiquement acceptable étant l'huile d'arachide ;
  - le mouillage d'une poudre comprenant de l'amoxicilline par la solution de mouillage afin d'obtenir une préparation poudreuse comprenant l'amoxicilline, le cinéol et l'huile ;

- le mélange de la préparation poudreuse comprenant l'amoxicilline, le cinéol et l'huile avec une poudre comprenant un inhibiteur de β-lactamase, de préférence de l'acide clavulanique ; et/ou

- optionnellement, la poudre comprenant de l'amoxicilline et/ou la poudre comprenant un inhibiteur de β-lactamase, de préférence de l'acide clavulanique, comprennent en outre un désintégrant et/ou un antiagglomérant ; et/ou

- optionnellement, l'ajout d'édulcorant, d'aromatisant et/ou de lubrifiant, et le mélange de ceux-ci afin d'obtenir une poudre homogène ; et/ou

- optionnellement, le tamisage de la poudre ainsi obtenue; et/ou

- optionnellement, le conditionnement de la poudre tamisée dans des récipients unidoses.


**Patentansprüche**

1. Pharmazeutische Formulierung in Pulverform, umfassend oder im Wesentlichen bestehend aus Cineol, Amoxicillin, einem β-Lactamase-Inhibitor und einem pharmazeutisch akzeptablen Öl, wobei es sich bei dem pharmazeutisch akzeptablen Öl um Erdnussöl handelt.

2. Formulierung nach Anspruch 1, wobei es sich bei dem β-Lactamase-Inhibitor um Clavulansäure handelt.

3. Formulierung nach einem der vorhergehenden Ansprüche, wobei diese Formulierung zwischen etwa 5 mg und etwa 100 mg Cineol pro Gramm Pulver enthält.

4. Formulierung nach einem der vorhergehenden Ansprüche, wobei diese Formulierung zwischen etwa 20 mg und etwa 500 mg Amoxicillin pro Gramm Pulver enthält.

5. Formulierung nach einem der vorhergehenden Ansprüche, wobei diese Formulierung etwa 2 mg bis etwa 50 mg Öl pro Gramm Pulver enthält.

6. Formulierung nach einem der Ansprüche 2 bis 5, wobei diese Formulierung etwa 1 mg bis etwa 100 mg Clavulansäure pro Gramm Pulver enthält.

7. Formulierung nach einem der vorhergehenden Ansprüche, wobei das Massenverhältnis von Amoxicillin zu Cineol 2 bis 8, vorzugsweise 3 bis 7 und besonders bevorzugt 4 bis 6 beträgt.

8. Formulierung nach einem der vorhergehenden Ansprüche, wobei das Massenverhältnis von Amoxicillin zu Öl zwischen 5 und 15, vorzugsweise zwischen 7 und 13 und besonders bevorzugt zwischen 8 und 12 liegt.

9. Formulierung nach einem der vorhergehenden Ansprüche, wobei das Massenverhältnis von Cineol zu Öl zwischen 0,1 und 5, vorzugsweise zwischen 0,5 und 4, besonders bevorzugt zwischen 1 und 3 liegt.

10. Formulierung nach einem der Ansprüche 1 bis 9, wobei das Massenverhältnis von Amoxicillin zu β-Lactamase-Inhibitor zwischen 5 und 11, vorzugsweise zwischen 6 und 10, besonders bevorzugt zwischen 7 und 9 liegt.

11. Formulierung nach einem der vorhergehenden Ansprüche, wobei diese Formulierung zur oralen Anwendung bestimmt ist, vorzugsweise nach Suspension in einem wässrigen Lösungsmittel.

12. Formulierung nach einem der vorhergehenden Ansprüche, wobei diese Formulierung in einem Einzeldosisbehältnis verpackt ist, vorzugsweise in einem Einzeldosisbehältnis mit einem Pulvergehalt zwischen ca. 1 g und ca. 150 g, besonders bevorzugt zwischen ca. 1 g und ca. 10 g.

13. Formulierung nach einem der vorhergehenden Ansprüche, wobei diese Formulierung ferner mindestens einen pharmazeutisch verträglichen Hilfsstoff oder Trägerstoff enthält, vorzugsweise ausgewählt aus der Gruppe bestehend aus einem Süßungsmittel, einem Aromastoff, einem Trennmittel, einem Gleitmittel, einem Sprengmittel oder einer Mischung davon.

14. Formulierung nach einem der vorhergehenden Ansprüche zur Verwendung bei der Behandlung einer Infektionskrankheit bei einem Patienten, vorzugsweise einer Infektionskrankheit bakteriellen Ursprungs, noch bevorzugter einer Infektionskrankheit, die durch ein gegen β-Lactam-Antibiotika resistentes Bakterium verursacht wird.

15. Formulierung zur Verwendung nach Anspruch 14, wobei die infektiöse Pathologie ausgewählt ist aus der Gruppe bestehend aus Zystitis, insbesondere rezidivierender akuter Zystitis, bakterieller Sinusitis, insbesondere akuter Kieferhöhlenentzündung, Otitis, insbesondere akuter Mittelohrentzündung, Bronchitis, insbesondere chronischer und/oder akuter Bronchitis, Bronchopneumopathien, insbesondere chronischer und/oder akuter Bronchopneumopathien, Pyelonephritis, Infektionen des oberen gynäkologischen Bereichs, Parodontitis, schweren stomatologischen Infektionen, insbesondere Abszessen, Phlegmonen und Zellulitis, Tierbissen, Knochen- und Gelenkinfektionen, insbesondere Osteomyelitis, wobei es sich bei dieser bakteriellen Infektion vorzugsweise um eine Zystitis handelt, die insbesondere durch ein gegen β-Lactam-Antibiotika resistentes Bakterium verursacht wird.

16. Formulierung zur Verwendung nach Anspruch 14 oder 15, wobei die Formulierung dem Probanden über einen Zeitraum von 1 Tag bis 4 Wochen, vorzugsweise über einen Zeitraum von etwa 7 Tagen, in einer Dosis von 3 bis 30 Gramm pro Tag in einer oder mehreren Tagesdosen verabreicht wird.

17. Verfahren zur Herstellung der pharmazeutischen Formulierung nach einem der Ansprüche 1 bis 13, umfassend:

- Herstellen einer Benetzungslösung durch Mischen von Cineol und einem pharmazeutisch verträglichen Öl, wobei es sich bei diesem pharmazeutisch verträglichen Öl um Erdnussöl handelt;
- Benetzen eines Amoxicillin-haltigen Pulvers mit der Benetzungslösung, um ein Pulverpräparat zu erhalten, das Amoxicillin, Cineol und das Öl enthält;
- Mischen des Pulverpräparats, das Amoxicillin, Cineol und das Öl enthält, mit einem Pulver, das einen β-Lactamase-Inhibitor, vorzugsweise Clavulansäure, enthält; und/oder
- gegebenenfalls das Amoxicillin-haltige Pulver und/oder das Pulver, das einen β-Lactamase-Inhibitor, vorzugsweise Clavulansäure, enthält, zusätzlich ein Sprengmittel und/oder ein Trennmittel enthält; und/oder
- gegebenenfalls die Zugabe von Süßstoff, Aromastoff und/oder Gleitmittel und das Mischen dieser Stoffe, um ein homogenes Pulver zu erhalten; und/oder
- gegebenenfalls das Sieben des so erhaltenen Pulvers; und/oder
- gegebenenfalls die Verpackung des gesiebten Pulvers in Einzeldosisbehältern.

**Claims**

1. A pharmaceutical formulation in powder form comprising, or consisting substantially of, cineole, amoxicillin, a β-lactamase inhibitor and a pharmaceutically acceptable oil, said pharmaceutically acceptable oil being peanut oil.

2. The formulation according to claim 1, wherein said β-lactamase inhibitor is clavulanic acid.

3. The formulation according to any one of the preceding claims, wherein said formulation comprises between about 5 mg and about 100 mg of cineole per gram of powder.

4. The formulation according to any one of the preceding claims, wherein said formulation comprises between about 20 mg and about 500 mg of amoxicillin per gram of powder.

5. The formulation according to any one of the preceding claims, wherein said formulation comprises between about 2 mg and about 50 mg of oil per gram of powder.

6. The formulation according to any one of claims 2 to 5, wherein said formulation comprises between about 1 mg and about 100 mg of clavulanic acid per gram of powder.

7. The formulation according to any one of the preceding claims, wherein the amoxicillin/cineole mass ratio is comprised between 2 and 8, preferably between 3 and 7, more particularly preferably between 4 and 6.

8. The formulation according to any one of the preceding claims, wherein the amoxicillin/oil mass ratio is comprised between 5 and 15, preferably between 7 and 13, and more particularly preferably between 8 and 12.

9. The formulation according to any one of the preceding claims, wherein the cineole/oil mass ratio is comprised between 0.1 and 5, preferably between 0.5 and 4, more particularly preferably between 1 and 3.

10. The formulation according to any one of claims 1 to 9, wherein the amoxicillin/β-lactamase inhibitor mass ratio is comprised between 5 and 11, preferably between 6 and 10, more particularly preferably between 7 and 9.

11. The formulation according to any one of the preceding claims, wherein said formulation is intended for oral administration, preferably after suspension in an aqueous solvent.

12. The formulation according to any one of the preceding claims, wherein said formulation is packaged in a single-dose container, preferably a single-dose container containing between about 1 g and about 150 g of powder, preferably between about 1 g and about 10 g of powder.

13. The formulation according to any one of the preceding claims, wherein said formulation further comprises at least one pharmaceutically acceptable excipient or carrier, preferably selected from the group consisting of a sweetener, a flavoring agent, an anti-caking agent, a lubricant, a disintegrant, and a mixture thereof.

14. The formulation according to any one of the preceding claims for use in the treatment of an infectious disease in a subject, preferably an infectious disease of bacterial origin, more preferably an infectious disease caused by a bacterium resistant to β-lactam antibiotics.

15. The formulation for use according to claim 14, wherein the infectious pathology is selected from the group consisting of cystitis, in particular recurrent acute cystitis, bacterial sinusitis, in particular acute maxillary sinusitis, otitis, in particular acute otitis media, bronchitis, in particular chronic and/or acute bronchitis, bronchopneumonia, in particular chronic and/or acute bronchopneumonia, pyelonephritis, upper gynecological infections, periodontitis, severe stomatological infections, in particular abscesses, phlegmons and cellulitis, animal bites, bone and joint infections, in particular osteomyelitis, preferably said bacterial infection is cystitis, in particular caused by a bacterium resistant to β-lactam antibiotics.

16. The formulation for use according to claim 14 or 15, wherein the formulation is administered to the subject for a period of 1 day to 4 weeks, preferably for a period of about 7 days, at a dose of 3 to 30 grams per day, in one or more daily doses.

17. A method for manufacturing the pharmaceutical formulation according to any one of claims 1 to 13 comprising:

- obtaining a wetting solution by mixing cineole and a pharmaceutically acceptable oil, said pharmaceutically acceptable oil being peanut oil;
- wetting a powder comprising amoxicillin with the wetting solution in order to obtain a powder preparation comprising amoxicillin, cineole and oil;
- mixing the powdered preparation comprising amoxicillin, cineole and oil with a powder comprising a β-lactamase inhibitor, preferably clavulanic acid; and/or
- optionally, the powder comprising amoxicillin and/or the powder comprising a β-lactamase inhibitor, preferably clavulanic acid, further comprise a disintegrant and/or an anti-caking agent; and/or
- optionally, adding a sweetener, flavoring and/or lubricant agent, and mixing the latter to obtain a homogeneous powder; and/or
- optionally, sifting the resulting powder; and/or
- optionally, packaging the sifted powder in single-dose containers.

Figure 1

Figure 2

Figure 3

**Figure 4**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2016041958 A

**Littérature non-brevet citée dans la description**

- *CHEMICAL ABSTRACTS*, 470-82-6
- *CHEMICAL ABSTRACTS*, 26787-78-0
- *CHEMICAL ABSTRACTS*, 58001-44-8
- **REMMAL A et al.** *J. Essent. Oil. Res [Book*, 1993, vol. 5, 1179-1184
- **CASEY JT et al.** *J Microbiol Meth [Book*, 2004, vol. 58, 327-334
- **PATTON T et al.** *J Microbiol Meth [Book*, 2006, vol. 64, 84-95
- **ODDS FC et al.** *J Antimicrob Chemoth [Book*, 2003, vol. 52, 1
- **REMMAL et al.** *J. Essent. Oil. Res [Book*, 1993, vol. 5, 1179-1184